# EUROPEAN PATENT APPLICATION

(11) **EP 2 366 800 A1**
(43) Date of publication of application: **21.09.2011**
(21) Application number: 10461504.2
(22) Date of filing: 01.03.2010
(51) Int. Cl.: C12Q 1/68

(54) **Kit, method and use for the diagnosis of papillary thyroid cancer using a gene expression profile**

(71) Applicant: Centrum Onkologii-Instytut im M. Sklodowskiej-Curie Oddzial w Gliwicach, 40-100 Gliwice (PL)
(72) Inventor: Jarzab, Michal, 41-807, Zabrze (PL); Oczko-Wojciechowska, Malgorzata, 41-808, Zabrze (PL); Wiench, Malgorzata, 44-105, Gliwice (PL); Fujarewicz, Krzysztof, 43-100, Tychy (PL); Pfeifer, Aleksandra, 41-705, Ruda Slaska (PL); Swierniak, Michal, 44-122, Gliwice (PL); Jarzab, Barbara, 44-101, Gliwice (PL)
(74) Representative: Witek, Rafal

(57) **Abstract**

The subject of the present invention is a set of genes and its use and the connected method of molecular diagnostics of papillary thyroid cancer, preferably used to study expression at the level of RNA.

SEQUENCE LISTING

<110>  Centrum Onkologii - Instytut im. M. Skłodowskiej-Curie,
       Oddział w Gliwicach

<120>  Zestaw, spoób i zastosowanie do wykrywania profilu ekspresji
       genów charakterystycznego dla raka brodawkowatego tarczycy

<130>  PK/0923/AG

<160>  148

<170>  PatentIn version 3.3

<210>  1
<211>  25
<212>  DNA
<213>  artificial

<220>
<223>  sonda 1 z zestawu 201012_at

<400>  1
agaaatgcct cacagctatc gtgaa                                          25


<210>  2
<211>  25
<212>  DNA
<213>  artificial

<220>
<223>  sonda 2 z zestawu 201012_at

<400>  2
gctatcgtga agtgcgccac aagca                                          25


<210>  3
<211>  25
<212>  DNA
<213>  artificial

<220>
<223>  sonda 3 z zestawu 201012_at

<400>  3
gcgccacaag caaaccagct ttctt                                          25


<210>  4
<211>  25
<212>  DNA
<213>  artificial

<220>
<223>  sonda 4 z zestawu 201012_at

<400>  4
aaaccagctt tctttgcaga gaagc                                          25


<210>  5
<211>  25
<212>  DNA
<213>  artificial

<220>

<223>  sonda 5 z zestawu 201012_at

<400>  5
gcagagaagc ttcatcaagc catga                                           25


<210>  6
<211>  25
<212>  DNA
<213>  artificial

<220>
<223>  sonda 6 z zestawu 201012_at

<400>  6
ggtgttggaa ctcgccataa ggcat                                           25


<210>  7
<211>  25
<212>  DNA
<213>  artificial

<220>
<223>  sonda 7 z zestawu 201012_at

<400>  7
tatggtttcc cgttctgaaa ttgac                                           25


<210>  8
<211>  25
<212>  DNA
<213>  artificial

<220>
<223>  sonda 8 z zestawu 201012_at

<400>  8
agaagatgta tggtatctcc ctttg                                           25


<210>  9
<211>  25
<212>  DNA
<213>  artificial

<220>
<223>  sonda 9 z zestawu 201012_at

<400>  9
cctttgccaa gccatcctgg atgaa                                           25


<210>  10
<211>  25
<212>  DNA
<213>  artificial

<220>
<223>  sonda 10 z zestawu 201012_at

<400>  10
gaaactaaac attcccttga tggtc                                           25


<210>  11
<211>  25

<212>  DNA
<213>  artificial

<220>
<223>  sonda 11 z zestawu 201012_at

<400>  11
gatggtctca agctatgatc agaag                                          25


<210>  12
<211>  25
<212>  DNA
<213>  artificial

<220>
<223>  sonda 1 z zestawu 207808_s_at

<400>  12
gtggcttcac atttaaacca gtatc                                          25


<210>  13
<211>  25
<212>  DNA
<213>  artificial

<220>
<223>  sonda 2 z zestawu 207808_s_at

<400>  13
gtgggacata ttttcctgag agcag                                          25


<210>  14
<211>  25
<212>  DNA
<213>  artificial

<220>
<223>  sonda 3 z zestawu 207808_s_at

<400>  14
cagctgcagt ctgtcaggat gagat                                          25


<210>  15
<211>  25
<212>  DNA
<213>  artificial

<220>
<223>  sonda 4 z zestawu 207808_s_at

<400>  15
ggtggggaaa tctgaagtgg gtaca                                          25


<210>  16
<211>  25
<212>  DNA
<213>  artificial

<220>
<223>  sonda 5 z zestawu 207808_s_at

<400>  16
gtgggtcaca caaggtctac attac                                          25

<210>  17
<211>  25
<212>  DNA
<213>  artificial

<220>
<223>  sonda 6 z zestawu 207808_s_at

<400>  17
catagttttc cttgaatcca acttt                                          25


<210>  18
<211>  25
<212>  DNA
<213>  artificial

<220>
<223>  sonda 7 z zestawu 207808_s_at

<400>  18
taaggaggta agattgccac gtgcc                                          25


<210>  19
<211>  25
<212>  DNA
<213>  artificial

<220>
<223>  sonda 8 z zestawu 207808_s_at

<400>  19
cgtgcctgct ggtactgtga tgcat                                          25


<210>  20
<211>  25
<212>  DNA
<213>  artificial

<220>
<223>  sonda 9 z zestawu 207808_s_at

<400>  20
ggcagtttta tcacgtttga atcta                                          25


<210>  21
<211>  25
<212>  DNA
<213>  artificial

<220>
<223>  sonda 10 z zestawu 207808_s_at

<400>  21
gaatctacca ttcatagcca gatgt                                          25


<210>  22
<211>  25
<212>  DNA
<213>  artificial

<220>

<223>  sonda 11 z zestawu 207808_s_at

<400>  22
gtatcagatg tttcactgac agttt                                          25


<210>  23
<211>  25
<212>  DNA
<213>  artificial

<220>
<223>  sonda 1 z zastawu 209395_at

<400>  23
gctgtgggga tagtgaggca tcgca                                          25


<210>  24
<211>  25
<212>  DNA
<213>  artificial

<220>
<223>  sonda 2 z zastawu 209395_at

<400>  24
ggggatagtg aggcatcgca atgta                                          25


<210>  25
<211>  25
<212>  DNA
<213>  artificial

<220>
<223>  sonda 3 z zastawu 209395_at

<400>  25
gaggcatcgc aatgtaagac tcggg                                          25


<210>  26
<211>  25
<212>  DNA
<213>  artificial

<220>
<223>  sonda 4 z zastawu 209395_at

<400>  26
gcatcgcaat gtaagactcg ggatt                                          25


<210>  27
<211>  25
<212>  DNA
<213>  artificial

<220>
<223>  sonda 5 z zastawu 209395_at

<400>  27
gtaagactcg ggattagtac acact                                          25


<210>  28
<211>  25

<212>  DNA
<213>  artificial

<220>
<223>  sonda 6 z zastawu 209395_at

<400>  28
gactcgggat tagtacacac ttgtt                                          25


<210>  29
<211>  25
<212>  DNA
<213>  artificial

<220>
<223>  sonda 7 z zastawu 209395_at

<400>  29
ggattagtac acacttgttg atgat                                          25


<210>  30
<211>  25
<212>  DNA
<213>  artificial

<220>
<223>  sonda 8 z zastawu 209395_at

<400>  30
ggaaatgttt acagatcccc aagcc                                          25


<210>  31
<211>  25
<212>  DNA
<213>  artificial

<220>
<223>  sonda 9 z zastawu 209395_at

<400>  31
aaatgtttac agatccccaa gcctg                                          25


<210>  32
<211>  25
<212>  DNA
<213>  artificial

<220>
<223>  sonda 10 z zastawu 209395_at

<400>  32
accttcactt aggaacgtaa tcgtg                                          25


<210>  33
<211>  25
<212>  DNA
<213>  artificial

<220>
<223>  sonda 11 z zastawu 209395_at

<400>  33
taggaacgta atcgtgtccc ctatc                                          25

<210>  34
<211>  25
<212>  DNA
<213>  artificial

<220>
<223>  sonda 1 z zastawu 209396_s_at

<400>  34
tcaccaatgc catcaaggat gcact                                                25


<210>  35
<211>  25
<212>  DNA
<213>  artificial

<220>
<223>  sonda 2 z zastawu 209396_s_at

<400>  35
caaggatgca ctcgctgcaa cgtag                                                25


<210>  36
<211>  25
<212>  DNA
<213>  artificial

<220>
<223>  sonda 3 z zastawu 209396_s_at

<400>  36
cacacagcac gggggccaag gatgc                                                25


<210>  37
<211>  25
<212>  DNA
<213>  artificial

<220>
<223>  sonda 4 z zastawu 209396_s_at

<400>  37
tgcagaggtc cacaacacac agatt                                                25


<210>  38
<211>  25
<212>  DNA
<213>  artificial

<220>
<223>  sonda 5 z zastawu 209396_s_at

<400>  38
cacagatttg agctcagccc tggtg                                                25


<210>  39
<211>  25
<212>  DNA
<213>  artificial

<220>

<223>  sonda 6 z zastawu 209396_s_at

<400>  39
ccctagccct ccttatcaaa ggaca                                              25


<210>  40
<211>  25
<212>  DNA
<213>  artificial

<220>
<223>  sonda 7 z zastawu 209396_s_at

<400>  40
aaggacacca ttttggcaag ctcta                                              25


<210>  41
<211>  25
<212>  DNA
<213>  artificial

<220>
<223>  sonda 8 z zastawu 209396_s_at

<400>  41
ggcaagctct atcaccaagg agcca                                              25


<210>  42
<211>  25
<212>  DNA
<213>  artificial

<220>
<223>  sonda 9 z zastawu 209396_s_at

<400>  42
atcctacaag acacagtgac catac                                              25


<210>  43
<211>  25
<212>  DNA
<213>  artificial

<220>
<223>  sonda 10 z zastawu 209396_s_at

<400>  43
agtgaccata ctaattatac cccct                                              25


<210>  44
<211>  25
<212>  DNA
<213>  artificial

<220>
<223>  sonda 11 z zastawu 209396_s_at

<400>  44
gcaaagccag cttgaaacct tcact                                              25


<210>  45
<211>  25

<212>  DNA
<213>  artificial

<220>
<223>  sonda 1 z zestawu 209810_at

<400>  45
gaaaaaccag ccactgcttt acagg                                          25


<210>  46
<211>  25
<212>  DNA
<213>  artificial

<220>
<223>  sonda 2 z zestawu 209810_at

<400>  46
   acaggggttt gaagctgagc cccgc                                           25


<210>  47
<211>  25
<212>  DNA
<213>  artificial

<220>
<223>  sonda 3 z zestawu 209810_at

<400>  47
ggattttaca gctacttgca attca                                          25


<210>  48
<211>  25
<212>  DNA
<213>  artificial

<220>
<223>  sonda 4 z zestawu 209810_at

<400>  48
gcaattcccc agcgtagtca agggt                                          25


<210>  49
<211>  25
<212>  DNA
<213>  artificial

<220>
<223>  sonda 5 z zestawu 209810_at

<400>  49
agtcaagggt ggacactgca cgctc                                          25


<210>  50
<211>  25
<212>  DNA
<213>  artificial

<220>
<223>  sonda 6 z zestawu 209810_at

<400>  50
actgcacgct ctggcatgat gggat                                          25

<210>  51
<211>  25
<212>  DNA
<213>  artificial

<220>
<223>  sonda 7 z zestawu 209810_at

<400>  51
catgatggga tggcgaccgg gcaag                                          25


<210>  52
<211>  25
<212>  DNA
<213>  artificial

<220>
<223>  sonda 8 z zestawu 209810_at

<400>  52
gggtgattct atttctgctg tgatt                                          25


<210>  53
<211>  25
<212>  DNA
<213>  artificial

<220>
<223>  sonda 9 z zestawu 209810_at

<400>  53
agctcagctg gggttgtgca agcta                                          25


<210>  54
<211>  25
<212>  DNA
<213>  artificial

<220>
<223>  sonda 10 z zestawu 209810_at

<400>  54
gcaagctagg gacccattcc tgtgt                                          25


<210>  55
<211>  25
<212>  DNA
<213>  artificial

<220>
<223>  sonda 11 z zestawu 209810_at

<400>  55
gtaatacaat gtctgcacca atgct                                          25


<210>  56
<211>  25
<212>  DNA
<213>  artificial

<220>

<223>  sonda 1 z zestawu 210342_s_at

<400>  56
tgaacgagtg tgcagacggt gccca                                          25


<210>  57
<211>  25
<212>  DNA
<213>  artificial

<220>
<223>  sonda 2 z zestawu 210342_s_at

<400>  57
gaaacaccaa aggcggcttc cagtg                                          25


<210>  58
<211>  25
<212>  DNA
<213>  artificial

<220>
<223>  sonda 3 z zestawu 210342_s_at

<400>  58
gcggacccct acgagttagg agacg                                          25


<210>  59
<211>  25
<212>  DNA
<213>  artificial

<220>
<223>  sonda 4 z zestawu 210342_s_at

<400>  59
gctctgctga tcggaggctt cgcag                                          25


<210>  60
<211>  25
<212>  DNA
<213>  artificial

<220>
<223>  sonda 5 z zestawu 210342_s_at

<400>  60
aggtctcacc tcgacggtga tttgc                                          25


<210>  61
<211>  25
<212>  DNA
<213>  artificial

<220>
<223>  sonda 6 z zestawu 210342_s_at

<400>  61
cacgcactgg cactaaatcc acact                                          25


<210>  62
<211>  25

<212>  DNA
<213>  artificial

<220>
<223>  sonda 7 z zestawu 210342_s_at

<400>  62
tgcccatctc ggagacaggc ggagg                                          25


<210>  63
<211>  25
<212>  DNA
<213>  artificial

<220>
<223>  sonda 8 z zestawu 210342_s_at

<400>  63
ggaactcccg agctgagatg cggaa                                          25


<210>  64
<211>  25
<212>  DNA
<213>  artificial

<220>
<223>  sonda 9 z zestawu 210342_s_at

<400>  64
gatggaaggc cgggatactc acagg                                          25


<210>  65
<211>  25
<212>  DNA
<213>  artificial

<220>
<223>  sonda 10 z zestawu 210342_s_at

<400>  65
acagcttcat gttcccaaaa tcacc                                          25


<210>  66
<211>  25
<212>  DNA
<213>  artificial

<220>
<223>  sonda 11 z zestawu 210342_s_at

<400>  66
tcagcaggac gactgttttc ccaac                                          25


<210>  67
<211>  25
<212>  DNA
<213>  artificial

<220>
<223>  sonda 1 z zestawu 210495_x_at

<400>  67
agccctttgc tctggaggaa gttct                                          25

<210> 68
<211> 25
<212> DNA
<213> artificial

<220>
<223> sonda 2 z zestawu 210495_x_at

<400> 68
caagcatcac cctgggagtt tcctg 25


<210> 69
<211> 25
<212> DNA
<213> artificial

<220>
<223> sonda 3 z zestawu 210495_x_at

<400> 69
gggagtttcc tgagggtttt ctcat 25


<210> 70
<211> 25
<212> DNA
<213> artificial

<220>
<223> sonda 4 z zestawu 210495_x_at

<400> 70
aaatgagggc tgcacattgc ctgtt 25


<210> 71
<211> 25
<212> DNA
<213> artificial

<220>
<223> sonda 5 z zestawu 210495_x_at

<400> 71
attgcctgtt ctgcttcgaa gtatt 25


<210> 72
<211> 25
<212> DNA
<213> artificial

<220>
<223> sonda 6 z zestawu 210495_x_at

<400> 72
aaagtcaccc aaacacttct gcttt 25


<210> 73
<211> 25
<212> DNA
<213> artificial

<220>

<223>  sonda 7 z zestawu 210495_x_at

<400>  73
tgctttcact taagtgtctg gcccg                                          25


<210>  74
<211>  25
<212>  DNA
<213>  artificial

<220>
<223>  sonda 8 z zestawu 210495_x_at

<400>  74
gtctggcccg caatactgta ggaac                                          25


<210>  75
<211>  25
<212>  DNA
<213>  artificial

<220>
<223>  sonda 9 z zestawu 210495_x_at

<400>  75
tatccacagt actcactttt tccaa                                          25


<210>  76
<211>  25
<212>  DNA
<213>  artificial

<220>
<223>  sonda 10 z zestawu 210495_x_at

<400>  76
gaaatatctt tctcttacct gttat                                          25


<210>  77
<211>  25
<212>  DNA
<213>  artificial

<220>
<223>  sonda 11 z zestawu 210495_x_at

<400>  77
tatgtgccaa agctttacta ctgtg                                          25


<210>  78
<211>  25
<212>  DNA
<213>  artificial

<220>
<223>  sonda 1 z zestawu 212850_s_at

<400>  78
ttcctgagtt gaaacttctc ctgtg                                          25


<210>  79
<211>  25

<212>  DNA
<213>  artificial

<220>
<223>  sonda 2 z zestawu 212850_s_at

<400>  79
gatcaattct tttctagtca gttct                                          25


<210>  80
<211>  25
<212>  DNA
<213>  artificial

<220>
<223>  sonda 3 z zestawu 212850_s_at

<400>  80
agagagagat accaggccct tagcc                                          25


<210>  81
<211>  25
<212>  DNA
<213>  artificial

<220>
<223>  sonda 4 z zestawu 212850_s_at

<400>  81
ctccccacat tacacttaac aagca                                          25


<210>  82
<211>  25
<212>  DNA
<213>  artificial

<220>
<223>  sonda 5 z zestawu 212850_s_at

<400>  82
gagtgagcac tttgggtcct tagac                                          25


<210>  83
<211>  25
<212>  DNA
<213>  artificial

<220>
<223>  sonda 6 z zestawu 212850_s_at

<400>  83
gggtccttag actcatgtct gggga                                          25


<210>  84
<211>  25
<212>  DNA
<213>  artificial

<220>
<223>  sonda 7 z zestawu 212850_s_at

<400>  84
ggatcttatt gcacttgggc tgttc                                          25

<210>  85
<211>  25
<212>  DNA
<213>  artificial

<220>
<223>  sonda 8 z zestawu 212850_s_at

<400>  85
atttgagcac tgatttactc tgtaa                                             25


<210>  86
<211>  25
<212>  DNA
<213>  artificial

<220>
<223>  sonda 9 z zestawu 212850_s_at

<400>  86
aatctctctg tcctaaacta atgga                                             25


<210>  87
<211>  25
<212>  DNA
<213>  artificial

<220>
<223>  sonda 10 z zestawu 212850_s_at

<400>  87
cgattctccc atgctcatgt gtaat                                             25


<210>  88
<211>  25
<212>  DNA
<213>  artificial

<220>
<223>  sonda 11 z zestawu 212850_s_at

<400>  88
atggttttaa cgttactcac tggag                                             25


<210>  89
<211>  25
<212>  DNA
<213>  artificial

<220>
<223>  sonda 1 z zestawu 216442_x_at

<400>  89
tggaggaagt tctccagctt cagct                                             25


<210>  90
<211>  25
<212>  DNA
<213>  artificial

<220>

<223>  sonda 2 z zestawu 216442_x_at

<400>  90
caagcatcac cctgggagtt tcctg                                          25


<210>  91
<211>  25
<212>  DNA
<213>  artificial

<220>
<223>  sonda 3 z zestawu 216442_x_at

<400>  91
gggagtttcc tgagggtttt ctcat                                          25


<210>  92
<211>  25
<212>  DNA
<213>  artificial

<220>
<223>  sonda 4 z zestawu 216442_x_at

<400>  92
aaatgagggc tgcacattgc ctgtt                                          25


<210>  93
<211>  25
<212>  DNA
<213>  artificial

<220>
<223>  sonda 1 z zestawu 216442_x_at

<400>  93
attgcctgtt ctgcttcgaa gtatt                                          25


<210>  94
<211>  25
<212>  DNA
<213>  artificial

<220>
<223>  sonda 6 z zestawu 216442_x_at

<400>  94
aaagtcaccc aaacacttct gcttt                                          25


<210>  95
<211>  25
<212>  DNA
<213>  artificial

<220>
<223>  sonda 7 z zestawu 216442_x_at

<400>  95
tgctttcact taagtgtctg gcccg                                          25


<210>  96
<211>  25

<212>  DNA
<213>  artificial

<220>
<223>  sonda 8 z zestawu 216442_x_at

<400>  96
gtctggcccg caatactgta ggaac                                          25


<210>  97
<211>  25
<212>  DNA
<213>  artificial

<220>
<223>  sonda 9 z zestawu 216442_x_at

<400>  97
tatccacagt actcactttt tccaa                                          25


<210>  98
<211>  25
<212>  DNA
<213>  artificial

<220>
<223>  sonda 10 z zestawu 216442_x_at

<400>  98
gaaatatctt tctcttacct gttat                                          25


<210>  99
<211>  25
<212>  DNA
<213>  artificial

<220>
<223>  sonda 11 z zestawu 216442_x_at

<400>  99
tatgtgccaa agctttacta ctgtg                                          25


<210>  100
<211>  25
<212>  DNA
<213>  artificial

<220>
<223>  sonda 1 z zestawu 221577_x_at

<400>  100
gacggcgtca aggtcgtggg acgtg                                          25


<210>  101
<211>  25
<212>  DNA
<213>  artificial

<220>
<223>  sonda 2 z zestawu 221577_x_at

<400>  101
caactgctgg cagaatcttc gtccg                                          25

<210>  102
<211>  25
<212>  DNA
<213>  artificial

<220>
<223>  sonda 3 z zestawu 221577_x_at

<400>  102
gtcgccacgg gaggtgcaag tgacc                                              25


<210>  103
<211>  25
<212>  DNA
<213>  artificial

<220>
<223>  sonda 4 z zestawu 221577_x_at

<400>  103
tgcaagtgac catgtgcatc ggcgc                                              25


<210>  104
<211>  25
<212>  DNA
<213>  artificial

<220>
<223>  sonda 5 z zestawu 221577_x_at

<400>  104
gccagttccg ggcggcaaac atgca                                              25


<210>  105
<211>  25
<212>  DNA
<213>  artificial

<220>
<223>  sonda 6 z zestawu 221577_x_at

<400>  105
tgcacgcgca gatcaagacg agcct                                              25


<210>  106
<211>  25
<212>  DNA
<213>  artificial

<220>
<223>  sonda 7 z zestawu 221577_x_at

<400>  106
cagctacaat cccatggtgc tcatt                                              25


<210>  107
<211>  25
<212>  DNA
<213>  artificial

<220>

<223>  sonda 8 z zestawu 221577_x_at

<400>  107
tgctcattca aaagaccgac accgg                                          25


<210>  108
<211>  25
<212>  DNA
<213>  artificial

<220>
<223>  sonda 9 z zestawu 221577_x_at

<400>  108
ccgacaccgg ggtgtcgctc cagac                                          25


<210>  109
<211>  25
<212>  DNA
<213>  artificial

<220>
<223>  sonda 10 z zestawu 221577_x_at

<400>  109
cgctccagac ctatgatgac ttgtt                                          25


<210>  110
<211>  25
<212>  DNA
<213>  artificial

<220>
<223>  sonda 11 z zestawu 221577_x_at

<400>  110
ttgttagcca aagactgcca ctgca                                          25


<210>  111
<211>  25
<212>  DNA
<213>  artificial

<220>
<223>  sonda 1 z zestawu 203510_at

<400>  111
gaagccaagg gttaacccag caagc                                          25


<210>  112
<211>  25
<212>  DNA
<213>  artificial

<220>
<223>  sonda 2 z zestawu 203510_at

<400>  112
gggtgtgtca cactgaaact caata                                          25


<210>  113
<211>  25

<212>  DNA
<213>  artificial

<220>
<223>  sonda 3 z zestawu 203510_at

<400>  113
gttgagtttg gctgttgttg cagga                                          25


<210>  114
<211>  25
<212>  DNA
<213>  artificial

<220>
<223>  sonda 4 z zestawu 203510_at

<400>  114
gatagtgcag agacttacca gaaga                                          25


<210>  115
<211>  25
<212>  DNA
<213>  artificial

<220>
<223>  sonda 5 z zestawu 203510_at

<400>  115
aatccaaata ttgccgtttc ataaa                                          25


<210>  116
<211>  25
<212>  DNA
<213>  artificial

<220>
<223>  sonda 6 z zestawu 203510_at

<400>  116
agaactgtct ctaccagggt caaga                                          25


<210>  117
<211>  25
<212>  DNA
<213>  artificial

<220>
<223>  sonda 7 z zestawu 203510_at

<400>  117
gaactcgggg aaacatccca tcaac                                          25


<210>  118
<211>  25
<212>  DNA
<213>  artificial

<220>
<223>  sonda 8 z zestawu 203510_at

<400>  118
atcccatcaa caggactaca cactt                                          25

<210>  119
<211>  25
<212>  DNA
<213>  artificial

<220>
<223>  sonda 9 z zestawu 203510_at

<400>  119
aaacttgtcc ttagattaat gtgtc                                             25


<210>  120
<211>  25
<212>  DNA
<213>  artificial

<220>
<223>  sonda 10 z zestawu 203510_at

<400>  120
ttagatactt gtcactgcct atacc                                             25


<210>  121
<211>  25
<212>  DNA
<213>  artificial

<220>
<223>  sonda 11 z zestawu 203510_at

<400>  121
gctgaactga atggtacttc gtatg                                             25


<210>  122
<211>  25
<212>  DNA
<213>  artificial

<220>
<223>  sonda 1 z zestawu 207144_s_at

<400>  122
ccaagagatg agctccgtgg cctac                                             25


<210>  123
<211>  25
<212>  DNA
<213>  artificial

<220>
<223>  sonda 2 z zestawu 207144_s_at

<400>  123
taccctgggg tagcctcaaa tggaa                                             25


<210>  124
<211>  25
<212>  DNA
<213>  artificial

<220>

<223>  sonda 3 z zestawu 207144_s_at

<400>  124
tagttcctcg ggatctccaa taggc                                          25


<210>  125
<211>  25
<212>  DNA
<213>  artificial

<220>
<223>  sonda 4 z zestawu 207144_s_at

<400>  125
ccctcacttg ctggctagta tgcag                                          25


<210>  126
<211>  25
<212>  DNA
<213>  artificial

<220>
<223>  sonda 5 z zestawu 207144_s_at

<400>  126
gcaggacccc tgcaaaactg ggact                                          25


<210>  127
<211>  25
<212>  DNA
<213>  artificial

<220>
<223>  sonda 6 z zestawu 207144_s_at

<400>  127
ctgctatcat cgattcggac ccagt                                          25


<210>  128
<211>  25
<212>  DNA
<213>  artificial

<220>
<223>  sonda 7 z zestawu 207144_s_at

<400>  128
aagtgctgat gtcgctggtg gtgga                                          25


<210>  129
<211>  25
<212>  DNA
<213>  artificial

<220>
<223>  sonda 8 z zestawu 207144_s_at

<400>  129
gtggaactgg ggttggaccg agcca                                          25


<210>  130
<211>  25

<212>  DNA
<213>  artificial

<220>
<223>  sonda 9 z zestawu 207144_s_at

<400>  130
gaccgagcca atgagcttcc ggagc                                          25


<210>  131
<211>  25
<212>  DNA
<213>  artificial

<220>
<223>  sonda 10 z zestawu 207144_s_at

<400>  131
atgagtttga cttcactgcg gactt                                          25


<210>  132
<211>  25
<212>  DNA
<213>  artificial

<220>
<223>  sonda 11 z zestawu 207144_s_at

<400>  132
gactttccat ctagctgcta atgcc                                          25


<210>  133
<211>  25
<212>  DNA
<213>  artificial

<220>
<223>  sonda 1 z zestawu 37004_at

<400>  133
ccactgcttt acaggacagg gggtt                                          25


<210>  134
<211>  25
<212>  DNA
<213>  artificial

<220>
<223>  sonda 2 z zestawu 37004_at

<400>  134
acccccatgc actcaaagat tggat                                          25


<210>  135
<211>  25
<212>  DNA
<213>  artificial

<220>
<223>  sonda 3 z zestawu 37004_at

<400>  135
ggattttaca gctacttgca attca                                          25

<210>  136
<211>  25
<212>  DNA
<213>  artificial

<220>
<223>  sonda 4 z zestawu 37004_at

<400>  136
gggaacatac agaactctaa aagat                                          25


<210>  137
<211>  25
<212>  DNA
<213>  artificial

<220>
<223>  sonda 5 z zestawu 37004_at

<400>  137
cagcaattcc ccagcgtagt caagg                                          25


<210>  138
<211>  25
<212>  DNA
<213>  artificial

<220>
<223>  sonda 6 z zestawu 37004_at

<400>  138
tagtcaaggg tggacactgc acgct                                          25


<210>  139
<211>  25
<212>  DNA
<213>  artificial

<220>
<223>  sonda 7 z zestawu 37004_at

<400>  139
agggtggaca ctgcacgctc tggca                                          25


<210>  140
<211>  25
<212>  DNA
<213>  artificial

<220>
<223>  sonda 8 z zestawu 37004_at

<400>  140
cgaccgggca agctttcttc ctcga                                          25


<210>  141
<211>  25
<212>  DNA
<213>  artificial

<220>

<223>  sonda 9 z zestawu 37004_at

<400>  141
tcctcgagat gctctgctgc ttgag                                          25


<210>  142
<211>  25
<212>  DNA
<213>  artificial

<220>
<223>  sonda 10 z zestawu 37004_at

<400>  142
agatgctctg ctgcttgaga gctat                                          25


<210>  143
<211>  25
<212>  DNA
<213>  artificial

<220>
<223>  sonda 11 z zestawu 37004_at

<400>  143
tctgctgctt gagagctatt gcttt                                          25


<210>  144
<211>  25
<212>  DNA
<213>  artificial

<220>
<223>  sonda 12 z zestawu 37004_at

<400>  144
ctgcttgaga gctattgctt tgtta                                          25


<210>  145
<211>  25
<212>  DNA
<213>  artificial

<220>
<223>  sonda 13 z zestawu 37004_at

<400>  145
tttgtctttc tgtaaggtgg acttc                                          25


<210>  146
<211>  25
<212>  DNA
<213>  artificial

<220>
<223>  sonda 14 z zestawu 37004_at

<400>  146
tctttctgta aggtggactt ccagc                                          25


<210>  147
<211>  25

<212>  DNA
<213>  artificial

<220>
<223>  sonda 15 z zestawu 37004_at

<400>  147
tgtaaggtgg acttccagct tttga                                          25


<210>  148
<211>  25
<212>  DNA
<213>  artificial

<220>
<223>  sonda 16 z zestawu 37004_at

<400>  148
agtcctaggg tgattctatt tctgc                                          25

## Description

The subject of the present invention is a set of genes, its use and relates to a model of molecular diagnostics applied to papillary thyroid cancer. The present invention is related to the science of molecular diagnostics of diseases and comprises the analysis of gene expression designed to measure the expression of a group of genes, to diagnose (discriminate) a specific malignancy. Specifically, the present invention relates to the analysis of multiple genes by microarray DNA analysis or real-time polymerase chain reaction to diagnose papillary thyroid cancer.

The majority of thyroid cancers are well differentiated ones and originate from well defined epithelial lineages (follicular versus C cells). Two main types of differentiated cancers (papillary, PTC and follicular, FTC) arise from one type of cell, enabling investigation of alternative pathways of tumourigenesis (Giordano, Detours , Jain, Eszlinger).

Thyroid cancer has been evaluated using DNA microarray-based analysis of cancer gene expression profile since 2001 (Huang). The relatively easy accessibility of benign thyroid lesions supplies comparative material for investigation of cancer-specific molecular events. Especially in the papillary histotype, the analysis of gene expression profile has been performed in many aspects. The gene signatures of various initiating events have been described and molecular multigene signatures have been proposed for different PTC morphological variants and/or other subgroups (Finley, Giordano, Detours, Jain, Eszlinger). More and more reports discuss the diagnostic aspects of gene expression profiling in thyroid tumours (Inohara 99, Brenet 2002, Lubitz and Fahey).

The amount of data is huge and enables one to compare and verify the results obtained by different authors (Eszlinger). Extensive data mining in published microarray-derived datasets helps one to understand the knowledge extractable from the previous reports, evaluate one's own data and to optimize further studies. Until now, only one formal meta-analysis of the published thyroid cancer genes has been performed (Griffith 2006).

Simultaneously, the proposed gene signatures which make it possible to differentiate PTC from benign tumours are relatively scarce and in most cases are based on single gene analyses. The resulting single genes have been combined in diagnostic kits comprising several genes. Again, most combinations were performed at the level of immunohistochemistry. There are only a few proposals based on RNA analysis. The first RNA-based multigene classifier, based on a microarray study, included TFF3, SERPINA1, TIMP1 and CRABP1 (Hawthorn 2004), a few other studies appeared simultaneously to or after this analysis. Kebebew et al. proposed a set of 6 markers for differentiating malignant and benign thyroid tumours. In the international paper WO 2009/026605 molecular markers to detected cancer cells of the thyroid were proposed. Additionally, WO 2009/111881 proposed other useful biomarkers, e.g. cytokeratin-19 and epidermal growth factor receptor (EGFR).

However, the use of genes previously known from the literature in our own dataset comprising more than 100 samples analyzed by the microarray technique pointed out insufficient sensitivity and specificity of these methods. It may explain why these methods are still not applicable for routine use in the diagnostic clinical setting (see e.g. the Recommendations of Polish Endocrine Tumours Working Group: Diagnosis and treatment of thyroid cancer).

It is often claimed that immunohistochemical methods applied to fine-needle histological specimens show superiority to the RNA-based methods, related to the ability to distinguish in microscope image the tumour cells form other cells present in fine-needle aspirate. The solution, which may be applied to select optimal molecular RNA markers is to use laser microdissection of tumour cells. By this approach one is able to separate tumour cells from the stroma and select these markers, which are specific for cancer cell, and are not expressed, neither in stroma nor in lymphocytic infiltrate. Our analysis of the literature points out that none of the solutions proposed for thyroid cancer was based on this approach and this may explain the lack of success in the practical use of these gene expression signatures. By the proposed approach, one is able to select gene classifiers consisting of multiple genes, often more than 20, and measure them routinely by real-time quantitative PCR reaction.

To summarize: the existing technical solutions (multigene signatures supporting the diagnosis of thyroid cancer) were selected in relatively small groups of patients, without the use of micro-dissection and until now were not routinely applied in clinical practice.

The question we had to solve is whether the primary studies which lead to the selection of the thyroid cancer gene signature, should be based on immunohistochemical studies or on molecular studies. It shall be stressed that immunohistochemical studies were carried out in last 10-15 years very intensively, and despite this they did not result in any effective diagnostic algorithm. Quite the opposite, all international consensus diagnostic guidelines for thyroid nodules published between 2008-2010 stress the absence of a useful algorithm and do not recommended their use in practice.

As the needed improvement of papillary thyroid cancer diagnostics in material from fine-needle aspiration biopsy, important for the clinical management of patients with thyroid nodule, shall not be fully achieved by classic cytological or immunohistochemical methods, it is crucial to work out a sensitive and specific molecular test.

Papillary thyroid cancer comprises at least 85-90% of all thyroid carcinomas and thus the potential molecular test is of large practical importance, especially in the context of the large prevalence of nodular thyroid disease - in Poland as many as about 1 million females may require the fine needle aspiration biopsy of the thyroid.

Despite the fact that many cases of papillary thyroid carcinoma may be diagnosed in classic cytological analysis, the risk of a false negative result is significant, ca. 10%. Taking into account the risk of false negatives, many clinicians decide to carry on the surgical treatment, when the biopsy resulted in sub-optimal amount of material for cytological diagnosis. This occurs in about 15% of biopsies. The use of a sensitive molecular test may be a good solution for that problem. Moreover, in future the analysis of RNA markers might be carried out semiautomatic in fine-needle aspiration biopsy material, which is much more difficult for cytological diagnostics. The proposed invention is this related to the high demand for rapid and automated diagnostics of this subtype of thyroid cancer.

According to the present invention, a large collection of previously identified (from previous studies) samples analysed using DNA microarrays were evaluated for diagnostic potential. It turns out that evaluation of the quality of classification indicates that each of the techniques results in at most 88-92% correctly identified samples. This is unequivocal evidence that the classification criteria used thus far are suboptimal, and genes selected using earlier studies lack optimal classification potential. Extant techniques, not using laser microdissection at the stage of compiling the classification criteria did not facilitate a 95% prediction accuracy which is the threshold for potential clinical use. The present invention thus fulfils the need of a molecular tool facilitating the identification of papillary thyroid cancer with a defined level of statistical significance (minimum 95% correctly identified samples) and is an original solution, because it is the first to use laser microdissection of tumour cells for the initial collection of the genes used in the molecular analysis and detection of papillary thyroid cancer.

In light of the above, it is the goal of the present invention is to identify RNA markers for thyroid cancer, focused on the detection of the most prevalent form of tumour in this cancer, this being papillary cancer, through a microarray analysis of sections and isolated cells of the papillary cancer obtained via laser microdissection, validated using microarray analysis on larger tumour fragments collected during surgery, as well as real-time PCR performed on this material.

This goal encompasses the indication of a group of genes whose expression changes in papillary thyroid cancer, due to which the evaluation of their expression can be used in diagnosing this type of cancer.

The aim of the present invention is to propose a set of genes relevant to the diagnosis of papillary thyroid cancer.

The subject of the present invention is a set of RNA sequences for detecting gene expression characteristic for papillary thyroid cancer, characterized in that it comprises elements used for the detection of the following genes: ANXA1, CHI3L1, CITED1, FN1, GDF15, LRP4, MET, PROS 1, SFTPB, TPO, MPPED2, TFF3, and DPP4.

Preferably, the kit is constructed to measure expression at the level of RNA and it preferably contains nucleotide sequences encompassing: 201012_at (SEQ ID NO 1-11), 207808_s_at (SEQ ID NO 12-22), 209395_at (SEQ ID NO 23-33), 209396_s_at (SEQ ID NO 34-44), 209810_at (SEQ ID NO 45-55), 210342_s_at (SEQ ID NO 56-66), 210495_x_at (SEQ ID NO 67-77), 212850_s_at (SEQ ID NO 78-88), 216442_x_at (SEQ ID NO 89-99), 221577_x_at (SEQ ID NO 100-110), 203510_at (SEQ ID NO 111-121), 207144_s_at (SEQ ID NO 122-132), and 37004_at (SEQ ID NO 132-148).

The next subject of the present invention is a method of detecting papillary thyroid cancer, characterised in that a sample of tumour tissue is collected from a patient and then the gene expression profile is examined in the sample, wherein the expression profile characteristic of papillary thyroid cancer used is the profile encompassing the increased expression of the following genes: ANXA1, CHI3L1, CITED1, FN1, GDF15, LRP4, MET, PROS1, SFTPB, MPPED2, TFF3, and DPP4 as well as the depressed expression of the TPO gene.

Preferably, the expression is examined at the level of RNA in the method according the present invention.

The method according to the invention is characterised in that the expression profile is examined using sets of nucleotide probes possessing sequences encompassed by the following sets: 201012_at (SEQ ID NO 1-11), 207808_s_at (SEQ ID NO 12-22), 209395_at (SEQ ID NO 23-33), 209396_s_at (SEQ ID NO 34-44), 209810_at (SEQ ID NO 45-55), 210342_s_at (SEQ ID NO 56-66), 210495_x_at (SEQ ID NO 67-77), 212850_s_at (SEQ ID NO 78-88), 216442_x_at (SEQ ID NO 89-99), 221577_x_at (SEQ ID NO 100-110), 203510_at (SEQ ID NO 111-121), 207144_s_at (SEQ ID NO 122-132), and 37004_at (SEQ ID NO 132-148). The next subject of the present invention is the use of a set of genes consisting of: ANXA1, CHI3L1, CITED1, FN1, GDF15, LRP4, MET, PROS1, SFTPB, TPO, MPPED2, 25 TFF3, and DPP4 for detecting papillary thyroid cancer.

In a preferable embodiment, the use relates to probes belonging to a group encompassing sets of nucleotide probes possessing sequences encompassed by the following sets: 201012_at (SEQ ID NO 1-11), 207808_s_at (SEQ ID NO 12-22), 209395_at (SEQ ID NO 23-33), 209396_s_at (SEQ ID NO 34-44), 209810_at (SEQ ID NO 45-55), 30 210342_s_at (SEQ ID NO 56-66), 210495_x_at (SEQ ID NO 67-77), 212850_s_at (SEQ ID NO 78- 88), 216442_x_at (SEQ ID NO 89-99), 221577_x_at (SEQ ID NO 100-110), 203510_at (SEQ ID NO 111-121), 207144_s_at (SEQ ID NO 122-132), and 37004_at (SEQ ID NO 132-148) for detecting papillary thyroid cancer.

### Examples

### Reference PTC dataset

The genes published by other authors were validated in our PTC dataset, obtained using high density oligonucleotide microarray (U133A) analysis of 16 PTCs and 16 respective normal tumours (Jarzab), which is publicly available.

### Example 1

### Results of the meta-analysis on gene lists

Via a systematic analysis of all reports included in the study we found out 1337 unique Gene IDs (LocusLink IDs). 1161 could be correctly mapped to our data set. There were 1099 genes mentioned only in a single group. From the remaining 238 genes (209 correctly mapped), 166 (148) appeared twice, 45 (37) were listed in three gene lists, 16 (15) were found in 4 gene lists while 8 (6) genes were given in 5 gene lists and 3 appeared more than 5 times. Two genes (FN1, up-regulated and TFF3, down-regulated) were found in 7 gene lists (and additionally, on our gene list). In total, 72 (61) genes were listed in more than two groups. For validation, we reanalyzed the significance of the genes published by other authors in our PTC dataset (Table 1). A gene was considered significant if its FDR was <5%. In total, 509 genes from the initial list (43.7%) were confirmed in our PTC dataset (Table 2). From 656 genes which appeared not significant, there were only 67 (10.2%) genes which were indicated more than once in other gene lists.

The highest degree of confirmation was observed in the Huang-Prasad gene list, as 90.2% of genes listed by them proved to be significant in our independent dataset. The three other PTC-related lists by Mazzanti, Barden-Finley and Wasenius showed 67.5-57.9% level of confirmation in our PTC dataset. The degree of similarity was even higher for the lists by Eszlinger, Takano-Hasegawa and Aldred despite the fact that the authors considered either benign thyroid tumours (Eszlinger) and follicular tumours (Takano, Aldred). The lowest degree of overlap was observed for Onda et al. who analyzed anaplastic cancers (Table 1). Subsequently we focused on the genes, listed by at least 4 other groups (Table 4). Among the 24 genes selected, 20 (83.3%) were significant, 14 with the FDR in the 10-6 or even lower range. Four genes did not meet the FDR<5% criterion: DUSP1, LDHA, DDIT3 and thyroglobulin (the last two genes appeared to be completely insignificant in our analysis).

Contrary to thyroglobulin, the changes in expression of other thyroid-specific genes were confirmed by our approach: TPO, indicated by 6 studies, and both DIO1 and DI02, listed by 5 and 4 studies respectively, exhibited a highly significant increase of their expression in PTC. There were 125 further genes, selected by 3 or 2 groups. From among these we listed in Table 3 those fulfilling the very strict criterion of FDR <0.01% in our PTC samples. Combining 18 significant genes from Table 3 and the ones listed in Table 4 we obtained 85 genes which are listed by at least two other groups and exhibit the significant change in expression with FDR<0.01% in our PTC dataset.

The gene lists obtained by different microarray groups were further evaluated by hierarchical clustering, based on the percentage of genes similar between the lists. The analysis was performed on data from 22 groups (one analysis was excluded, as it provided only one gene). We found out that the gene lists clustered into three distinct groups (Fig. 1). The first group, with much larger intra-group correlation than two remaining ones, included two arms. In one there were four tightly correlated studies performed by oligonucleotide microarrays: (Huang-Prasad, Barden-Finley, Jarzab and Giordano). All of them were performed on papillary thyroid cancer, thus, the cluster was dubbed the PTC cluster. The other group contained two different gene sets, obtained either from follicular thyroid cancer (Aldred) or benign thyroid tumours (Eszlinger) by the same oligonucleotide microarray platform. This subgroup was called the FTC/benign cluster. Even if these two clusters ( PTC and FTC/benign) are correlated to each other more than with other studies, the distance between them is as large as the distance between two other main groups.

The two other clusters showed a larger similarity between themselves than with the first group. In one of them (the middle one) there were three PTC studies (Wasenius, Hamada, Yano), one study analyzing the difference between benign and malignant follicular thyroid tumours by SAGE (Cerutti) and one study in anaplastic thyroid cancer (Onda). In the third cluster the subgroup of strongest correlation included two studies identifying metastasis-related genes in follicular cancer (Zou and Chen), together with genes shown by Baris-Jacques, obtained mainly from oncocytic tumours. Of note is also the similarity between the gene sets of Wreesman (PTC) and Weber-Sarquis (FTC/FAs). An interesting pattern emerges through the analysis of gene clusters. They visibly divide into two groups, with the smaller cluster of genes frequently occurring in the four analyses of papillary thyroid cancer performed using a high density oligonucleotide microarray and rarely occurring in any of the other studies. However, it is clearly visible that many genes reported in one histotype may be found also in other thyroid cancer types. More importantly, a substantial number of them are changed also in benign thyroid tumours.

Table 1 presents the evaluation of genes from various gene lists in our PTC dataset. Genes were considered significant if FDR was <5% for the comparison of PTC and respective normal/benign tissue. Only genes correctly mapped to the PTC dataset were included and gene lists with less than 15 correctly mapped transcripts were excluded.

**Table 1.**

| | significant | not significant | total | % significant |
|---|---|---|---|---|
| Huang-Prasad | 55 | 6 | 61 | 90.16% |
| Eszlinger | 23 | 8 | 31 | 74.19% |
| Takano-Hasegawa | 14 | 5 | 19 | 73.68% |
| Aldred | 53 | 24 | 77 | 68.83% |
| Mazzanti | 27 | 13 | 40 | 67.50% |
| Barden-Finley | 153 | 81 | 234 | 65.38% |
| Wasenius | 11 | 8 | 19 | 57.89% |
| Weber-Sarquis | 47 | 39 | 86 | 54.65% |
| Wreesmann | 29 | 26 | 55 | 52.73% |
| Giordano | 58 | 53 | 111 | 52.25% |
| Jain | 38 | 41 | 79 | 48.10% |
| Chen | 19 | 34 | 53 | 35.85% |
| Cerutti | 6 | 11 | 17 | 35.29% |
| Baris-Jacques | 94 | 179 | 273 | 34.43% |
| Chevillard | 45 | 89 | 134 | 33.58% |
| Yano | 10 | 20 | 30 | 33.33% |
| Zou | 19 | 41 | 60 | 31.67% |
| Onda | 20 | 54 | 74 | 27.03% |

**Table 2.**

| Number of gene lists, in which the gene occurred | Number of genes significant in our data | Number of genes not shown to be differentially expressed | Total number of genes |
|---|---|---|---|
| 1 | 364 | 589 | 953 |
| | 38.2% | 61.8% | |
| 2 | 96 | 58 | 154 |
| | 62.3% | 37.7% | |
| 3 | 29 | 5 | 34 |
| | 85.3% | 14.7% | |
| 4 | 12 | 3 | 15 |
| | 80.0% | 20.0% | |
| 5 | 5 | 1 | 6 |
| | 83.3% | 16.7% | |
| 6-7 | 3 | 0 | 3 |
| | 100.0% | 0% | |
| All | 509 | 656 | 1165 |
| | 43.7% | 56.3% | |

Table 3 presents genes from at least 4 gene lists, ranked according to the difference in our PTC data set

**Table 3.**

| Gene Symbol | Gene_ID | Affy_ID | Gene name | Signal Log Ratio | FDR | B | Number of occurrences in other gene lists |
|---|---|---|---|---|---|---|---|
| FN1 | 2335 | 212464_s_at | fibronectin 1 | 3.21 | 2.9E-14 | 30.17 | 7+1 * |
| MET | 4233 | 203510_at | met proto-oncogene (hepatocyte growth factor receptor) | 2.48 | 2.1E-13 | 27.50 | 5+1* |
| LGALS3 | 3958 | 208949_s_at | lectin, galactoside-binding, soluble, 3 (galectin 3) /// galectin-3 internal gene | 1.91 | 6.3E-12 | 23.79 | 4+1* |
| TIMP1 | 7076 | 201666_at | tissue inhibitor of metalloproteinase 1 (erythroid potentiating activity, collagenase inhibitor) | 2.31 | 1.3E-10 | 20.31 | 5+1* |
| TFF3 | 7033 | 204623_at | trefoil factor 3 (intestinal) | -4.71 | 1.5E-09 | 17.56 | 7 |
| DUSP6 | 1848 | 208892_s_at | dual specificity phosphatase 6 | 1.85 | 3.9E-09 | 16.47 | 4+1* |
| CCND1 | 595 | 208712_at | cyclin D1 (PRAD1: parathyroid adenomatosis 1) | 1.70 | 1.0E-08 | 15.30 | 4+1* |
| ANXA 1 | 301 | 201012_at | annexin A1 | 1.28 | 1.8E-07 | 11.94 | 4 |
| CRABP 1 | 1381 | 205350_at | cellular retinoic acid binding protein 1 | -3.45 | 3.2E-07 | 11.10 | 4 |
| BCL2 | 596 | 203685_at | B-cell CLL/lymphoma 2 | -1.50 | 3.3E-07 | 11.04 | 5 |
| CITED 1 | 4435 | 207t44_s_at | Cbp/p300-interacting transactivator, with Glu/Asp-rich carboxy-terminal domain, 1 | 4.58 | 3.6E-07 | 10.96 | 4+1* |
| HGD | 3081 | 214308_s_at | homogentisate 1,2-dioxygenase (homogentisate oxidase) | -2.07 | 6.1E-07 | 10.33 | 5 |
| DI02 | 1734 | 203700_s_at | deiodinase, iodothyronine, | -1.25 | 1.9E-06 | 8.95 | 4 |
| | | | type II | | | | |
| ITPR 1 | 3708 | 203710_at | inositol 1,4,5-triphosphate receptor, type 1 | -1.81 | 2.1 E-06 | 8.84 | 4 |
| IGFBP6 | 3489 | 203851_at | insulin-like. growth factor binding protein 6 | 1.42 | 0.0001 | 3.88 | 4 |
| DI01 | 1733 | 206457_s_at | deiodinase, iodothyronine, type I | -3.09 | 0.0001 | 3.86 | 5 |
| TPO | 7173 | 210342_s_at | thyroid peroxidase | -4.10 | 0.0001 | 3.78 | 6 |
| ID4 | 3400 | 209292_at | Inhibitor of DNA binding 4, dominant negative helix-loop-helix protein | -1.31 | 0.0001 | 3.74 | 4 |
| APOD | 347 | 201525_at | apolipoprotein D | -2.01 | 0.0003 | 2.69 | 4 |
| TNC | 3371 | 201645_at | tenascin C (hexabrachion) | 1.99 | 0.0012 | 1.08 | 4 |
| DUSP1 | 1843 | 201044_x_at | dual specificity phosphatase 1 | -1.55 | 0.0508 | -3.53 | 4 |
| LDHA | 3939 | 200650_s_at | lactate dehydrogenase A | 0.23 | 0.1381 | -4.76 | 4 |
| DDIT3 | 1649 | 209383_at | DNA-damage-inducible transcript 3 | -0.10 | 0.7406 | -6.70 | 5 |
| TG | 7038 | 203673_at | Thyroglobulin | 0.05 | 0.8355 | -6.81 | 4 |

Table 4 presents top-ranked genes selected from gene lists containing the genes listed in 2 or 3 other reports (FDR<0.0001 in our PTC dataset), ranked according to the difference in our PTC dataset.

**Table 4.**

| Gene Symbol | Gene_ID | Affy_ID | Gene Name | Signal Log Ratio | FDR | B | Number of occurrences in other gene lists |
|---|---|---|---|---|---|---|---|
| DPP4 | 1803 | 203716_s_at | dipeptidylpeptidase 4 (CD26, adenosine deaminase complexing protein 2) | 7.08 | 2.7E-21 | 45.66 | 2+1* |
| SERPINA1 | 5265 | 202833_s_at | Serine (or cysteine) proteinase inhibitor, clade A (alpha-1 antiproteinase, antitrypsin), member 1 | 4.24 | 2.2E-15 | 33.40 | 3+1* |
| CDH3 | 1001 | 203256_at | cadherin 3, type 1, P-cadherin (placental) | 5.58 | 6.9E-15 | 31.96 | 2+1* |
| LAMB3 | 3914 | 209270_at | laminin, beta 3 | 4.22 | 7.7E-14 | 28.66 | 2+1* |
| PROS1 | 5627 | 207808_s_at | protein S (alpha) | 2.54 | 5.9E-12 | 23.92 | 2 |
| SDC4 | 6385 | 202071_at | syndecan 4 (amphiglycan, | 2.40 | 8.4E-12 | 23.47 | 3 |
| | | | ryudocan) | | | | |
| PDLIM4 | 8572 | 214175_x_at | PDZ and LIM domain 4 | 3.28 | 7.1E-11 | 21.02 | 3 |
| GDF15 | 9518 | 221577_x_at | growth differentiation factor 15 | 3.48 | 1.4E-10 | 20.24 | 2 |
| PRSS23 | 11098 | 202458_at | protease, serine, 23 | 2.16 | 2.1E-10 | 19.78 | 2+1* |
| C11orf8 | 744 | 205413_at | chromosome 11 open reading frame 8 | -3.30 | 3.1E-10 | 19.34 | 3 |
| SCEL | 8796 | 206884_s_at | Sciellin | 4.12 | 8.9E-10 | 18.22 | 2+1* |
| RAB27A | 5873 | 209515_s_at | RAB27A, member RAS oncogene family | 2.12 | 1.2E-09 | 17.89 | 2 |
| ETV5 | 2119 | 203349_s_at | ets variant gene 5 (ets-related molecule) | 1.36 | 1.3E-09 | 17.80 | 2+1* |
| CTSC | 1075 | 201487_at | cathepsin C | 2.36 | 1.5E-09 | 17.59 | 2+1* |
| FABP4 | 2167 | 203980_at | fatty acid binding protein 4, adipocyte | -3.63 | 2.1E-09 | 17.23 | 3 |
| QPCT | 25797 | 205174_s_at | glutaminyl-peptide cyclotransferase (glutaminyl cyclase) | 3.30 | 3.7E-09 | 16.57 | 2+1* |
| PLAU | 5328 | 211668_s_at | plasminogen activator, urokinase /// plasminogen activator, urokinase | 2.75 | 5.2E-09 | 16.16 | 2+1* |
| EPS8 | 2059 | 202609_at | epidermal growth factor receptor pathway substrate 8 | 1.56 | 1.3E-08 | 14.98 | 3+1* |
| CD44 | 960 | 209835_x_at | CD44 antigen (homing function and Indian blood group system) | 1.09 | 1.9E-08 | 14.58 | 3+1* |
| ARGBP2 | 8470 | 204288_s_at | Arg/Abl-interacting protein ArgBP2 | -2.06 | 3.0E-08 | 14.05 | 3 |
| CHI3L 1 | 1116 | 209396_s_at | chitinase 3-like 1 (cartilage glycoprotein-39) | 3.99 | 6.0E-08 | 13.28 | 2+1* |
| KRT19 | 3880 | 201650_at | Keratin 19 | 3.51 | 6.9E-08 | 13.09 | 3+1* |
| CCL21 | 6366 | 204606_at | chemokine (C-C motif) ligand 21 | -4.01 | 1.2E-07 | 12.49 | 3 |
| CCND2 | 894 | 200951_s_at | cyclin D2 | 1.43 | 1.3E-07 | 12.38 | 3+1* |
| KCNN4 | 3783 | 204401_at | potassium intermediate/small conductance calcium-activated channel, subfamily N, member 4 | 3.02 | 1.5E-07 | 12.13 | 2 |
| GPM6A | 2823 | 209470_s_at | glycoprotein M6A | -2.94 | 1.5E-07 | 12.13 | 2 |
| DPP6 | 1804 | 207789_s_at | dipeptidylpeptidase 6 | -2.42 | 2.0E-07 | 11.78 | 2 |
| GPR51 | 9568 | 211679 _x_at | G protein-coupled | 2.45 | 2.2E-07 | 11.61 | 2+1* |
| | | | receptor 51 /// G protein-coupled receptor 51 | | | | |
| ADORA1 | 134 | 205481_at | adenosine A1 receptor | 2.05 | 2.4E-07 | 11.51 | 2 |
| MATN2 | 4147 | 202350_s_at | matrilin 2 | -2.46 | 2.7E-07 | 11.33 | 3 |
| GPC3 | 2719 | 209220_at | glypican 3 | -1.76 | 2.8E-07 | 11.24 | 2 |
| CITED2 | 10370 | 209357_at | Cbp/p300-interacting transactivator, with Glu/Asp-rich carboxy-terminal domain, 2⁻ | -1.61 | 6.5E-07 | 10.25 | 2 |
| SLC39A14 | 23516 | 212110_at | solute carrier family 39 (zinc transporter), member 14 | -1.06 | 6.6E-07 | 10.23 | 2 |
| CA4 | 762 | 206209-s-at | carbonic anhydrase N | -2.66 | 7.3E-07 | 10.11 | 2 |
| LCN2 | 3934 | 212531_at | lipocalin 2 (oncogene 24p3) | 3.12 | 9.2E-07 | 9.82 | 2+1* |
| FGFR2 | 2263 | 203638_s_at | fibroblast growth factor receptor 2 (bacteria-expressed kinase, keratinocyte growth factor receptor, craniofacial dysostosis 1, Crouzon syndrome, Pfeiffer syndrome, Jackson-Weiss syndrome) | -1.61 | 9.9E-07 | 9.73 | 2 |
| HBB | 3043 | 211696_x_at | hemoglobin, beta /// hemoglobin, beta | -2.06 | 1.1E-06 | 9.64 | 2+1* |
| PLXNC1 | 10154 | 213241_at | plexin C 1 | 2.00 | 1.2E-06 | 9.50 | 2 |
| ADH1B | 125 | 209613_s_at | alcohol dehydrogenase IB (class I), beta polypeptide | -3.59 | 1.3E-06 | 9.45 | 2 |
| DPT | 1805 | 207977_s_at | dermatopontin | -3.20 | 1.5E-06 | 9.25 | 2 |
| AKR1C1 | 1645 | 204151_x_at | aldo-keto reductase family 1, member C 1 (dihydrodiol dehydrogenase 1; 20-alpha (3-alpha)-hydroxysteroid dehydrogenase) | -1.74 | 1.5E-06 | 9.19 | 2 |
| HBA1 | 3039 | 211699_x_at | hemoglobin, alpha 1 /// hemoglobin, alpha 1 /// hemoglobin, alpha 2 /// hemoglobin, alpha 2 | -2.44 | 1.8E-06 | 9.00 | 2 |
| SFTPB | 6439 | 213936_x_at | surfactant, pulmonary-associated protein | 2.66 | 2.5E-06 | 8.62 | 2 |
| | | | B | | | | |
| FHL1 | 2273 | 210299_s_at | four and a half LIM domains 1 | -1.88 | 2.7E-06 | 8.52 | 3 |
| TNFRSF11B | 4982 | 204932_at | tumour necrosis factor receptor superfamily, member 11 b (osteoprotegerin) | -2.08 | 3.2E-06 | 8.28 | 2 |
| RGS16 | 6004 | 209325_s_at | regulator of G-protein signalling 16 | -1.78 | 5.3E-06 | 7.65 | 2 |
| COL9A3 | 1299 | 204724_s_at | collagen, type IX, alpha 3 | -4.39 | 5.8E-06 | 7.53 | 2 |
| CHRDL 1 | 91851 | 209763_at | chordin-like 1 | -2.44 | 6.0E-06 | 7.49 | 2 |
| COL1A2 | 1278 | 202404_s_at | collagen, type I, alpha 2 | 1.78 | 6.2E-06 | 7.45 | 2 |
| CD36 | 948 | 209555_s_at | CD36 antigen (collagen type I receptor, thrombospondin receptor) | -1.44 | 6.6E-06 | 7.37 | 2 |
| RAP1GA1 | 5909 | 203911_at | RAP1, GTPase activating protein 1 | -1.61 | 7.4E-06 | 7.24 | 2 |
| IL1RAP | 3556 | 205227_at | interleukin 1 receptor accessory protein | 2.16 | 7.9E-06 | 7.15 | 2 |
| PGCP | 10404 | 208454_s_at | plasma glutamate carboxypeptidase | -1.10 | 8.6E-06 | 7.04 | 3 |
| SDC2 | 6383 | 212157_at | syndecan 2 (heparan sulfate proteoglycan 1, cell surface-associated, fibroglycan) | -1.20 | 1.1E-05 | 6.75 | 3 |
| IRS1 | 3667 | 204686_at | insulin receptor substrate 1 | -1.63 | 1.2E-05 | 6.66 | 2 |
| MFGE8 | 4240 | 210605 _s_at | milk fat globule-EGF factor 8 protein | 1.60 | 1.5E-05 | 6.39 | 2 |
| LGALS1 | 3956 | 201105_at | lectin, galactoside-binding, soluble, 1 (galectin 1) | 1.17 | 1.8E-05 | 6.18 | 2 |
| DUSP5 | 1847 | 209457_at | dual specificity phosphatase 5 | 2.05 | 1.9E-05 | 6.06 | 3 |
| PLA2R1 | 22925 | 207415_at | phospholipase A2 receptor 1, 180kDa | -1.40 | 3.2E-05 | 5.45 | 2 |
| MT1F | 4494 | 213629 _x_at | metallothionein 1F (functional) | -2.22 | 4.2E-05 | 5.12 | 3 |
| PHLDA2 | 7262 | 209803_s_at | pleckstrin homology-like domain, family A, member 2 | 1.92 | 4.2E-05 | 5.10 | 3 |
| COL8A1 | 1295 | 214587_at | collagen, type VIII, alpha 1 | 1.67 | 5.3E-05 | 4.82 | 3 |
| CTSB | 1508 | 213275_x_at | cathepsin B | 0.63 | 6.9E-05 | 4.52 | 2 |
| MUC1 | 4582 | 207847_s_at | mucin 1, transmembrane | 1.87 | 8.1E-05 | 4.32 | 3 |
| MST1R | 4486 | 205455_at | macrophage stimulating 1 | 0.98 | 8.1E-05 | 4.32 | 2 |
| | | | receptor (c-met-related tyrosine kinase) | | | | |
| P8 | 26471 | 209230_s_at | p8 protein (candidate of metastasis 1) | -1.78 | 8.4E-05 | 4.27 | 2 |
| SLC26A4 | 5172 | 206529_x_at | Solute carrier family 26, member 4 | -1.80 | 9.3E-05 | 4.16 | 2 |
| MT1G | 4495 | 204745_x_at | metallothionein 1G | -1.69 | 9.3E-05 | 4.15 | 3 |

| | | | | | | | |
|---|---|---|---|---|---|---|---|
| * The +1 indicator means that the gene was also included in our own PTC gene list published previously (RFR-100 gene set in (Jarzab et al., 2005) | | | | | | | |

### Example 2

### Data analysis of gross PTC tumour specimens

During the initial stage we used the dataset obtained via gene expression analysis of 363 thyroid samples: 165 samples obtained by Affymetrix HG-U133A microarray and 198 samples obtained by Affymetrix HG-U133 Plus 2.0 microarray. Samples from both platforms were pre-processed together after matching the paired probesets by GC-RMA algorithm. Gene filtering was performed: we excluded probesets with less than 10 % of expression data that have at least a 1.5-fold change in either direction from gene's median value OR with the p-value of the log-ratio variation of more than 0.05. 9812 probe sets passed these filtering criteria and were included in the final dataset.

From all 165 U133A samples, 53 samples corresponding to PTC and 67 corresponding normal/benign thyroid samples were selected. We used the boostrap-based Support Vector Machines class prediction method to assess class membership for each sample. 8 outlier samples were excluded, finally the analysis was carried out in 112 samples, 62 samples of a normal/benign thyroid and 50 PTC samples. The Support Vector Machines method with Recursive Feature Elimination was used to select 200 genes. The 0.632+ bootstrap cross-validation method was used to compute misclassification rate. When applied to HG-U133A data, this method has a sensitivity of 0.987, specificity 1, PPV 1 and NPV 0.989.

**Table 5. List of genes differentiating bulk PTC and benign/normal thyroid tissue, as obtained from data on HG-U133A microarray.**

| | Paramet ric p-value | t-value | Normal/benign thyroid (geom. mean) | PTC (geom. mean) | Fold-change | Probe set | Gene symbol | Description |
|---|---|---|---|---|---|---|---|---|
| 1 | < 1e-07 | -26.172 | 740.4415066 | 8872.3463153 | 0.083455 | 216442 x a t | FN1 | fibronectin 1 |
| 2 | < 1e-07 | -25.932 | 763.8327088 | 8355.4395141 | 0.0914174 | 210495 x a t | FN1 | fibronectin 1 |
| 3 | < 1e-07 | -25.239 | 90.7741553 | 2236.0551636 | 0.0405957 | 202833 s at | SERPI NA1 | serpin peptidase inhibitor, clade A |
| | | | | | | | | (alpha-1 antiproteinase, antitrypsin), member 1 |
| 4 | < 1e-07 | -24.917 | 486.0957738 | 6660.1549495 | 0.0729857 | 212464 s at | FN1 | fibronectin 1 |
| 5 | < 1e-07 | -24.323 | 689.0282742 | 7996.5393383 | 0.0861658 | 211719 x a t | FN1 | fibronectin |
| 6 | 1e-07 | -24.213 | 17.4336948 | 137.1755936 | 0.1270904 | (211478 s at | DPP4 | dipeptidyl-peptidase 4 |
| 7 | < 1e-07 | -23.709 | 30.2139978 | 195.6755926 | 0.1544086 | 203716 s at | DPP4 | dipeptidyl-peptidase 4 |
| 8 | < 1e-07 | -23.439 | 290.2323555 | 4137.7192292 | 0.0701431 | 211429 s at | SERPI NA1 | serpin peptidase inhibitor, clade A (alpha-1 antiproteinase, antitrypsin), member 1 |
| 9 | < 1e-07 | -22.37 | 60.1126534 | 441.1727934 | 0.1362565 | 203256 at | CDH3 | cadherin 3, type 1,P-cadherin (placental) |
| 10 | < 1e-07 | -21.514 | 38.0427172 | 167.8676008 | 0.2266233 | 202528 at | GALE | UDP-galactose-4-epimerase |
| 11 | < 1e-07 | -20.905 | 55.3860833 | 1430.9722406 | 0.0387052 | 202286 s at | TACS TD2 | tumour-associated calcium signal transducer 2 |
| 12 | < 1e-07 | -20.873 | 35.8560623 | 229.3901983 | 0.1563104 | 209270 at | LAMB 3 | laminin beta 3 |
| 13 | < 1e-07 | -19.917 | 26.9159071 | 1177.0115899 | 0.1520573 | 203717 at | IDPP4 | dipeptidyl-peptidase 4 |
| 14 | 1e-07 | -19.405 | 23.5406237 | 219.9024666 | 0.1070503 | 208025 s at | HMGA 2 | high mobility group AT-hook 2 |
| 15 | < 1e-07 | -18.953 | 38.8820466 | 216.5235256 | 0.1795742 | 203780 at | MPZL 2 | myelin protein zero-like 2 |
| 16 | < 1e-07 | -18.878 | 144.4412232 | 1006.3524115 | 0.1435295 | 203510 at | MET | met proto-oncogene (hepatocyte growth factor receptor) |
| 17 | < 1e-07 | -18.821 | 34.5248799 | 97.6115531 | 0.3536967 | 222173 s at | TBC1 D2 | TBC 1 domain family, member 2 |
| 18 | < 1e-07 | -18.785 | 45.286213 | 479.6106773 | 0.0944229 | 212850 s at | LRP4 4 | low density lipoprotein receptor-related protein 4 |
| 19 | < 1e-07 | -17.935 | 24.6773046 | 166.550981 | 0.1481667 | 205174 s at | QPCT | glutaminyl-peptide cyclotransferase |
| 20 | < 1e-07 | -17.729 | 40.1031912 | 118.7315875 | 0.3377635 | 205481 at | ADOR A1 | adenosine A1 receptor |
| 21 | < 1e-07 | -17.535 | 45.8911852 | 487.3856498 | 0.0941579 | 201650 at | KRT19 | keratin 19 |
| 22 | < 1e-07 | -17.465 | 44.7487106 | 235.4211323 | 0.1900794 | 205016 at | TGFA | transforming growth factor, alpha |
| 23 | < 1e-07 | -17.143 | 70.9465835 | 359.9617949 | 0.1970948 | 209228 x a | TUSC3 | tumour suppressor candidate 3 |
| 24 | < 1e-07 | -16.866 | 107.0562959 | 348.4481088 | 0.3072374 | 202733 at | P4HA2 | prolyl 4-hydroxylase, alpha polypeptide II |
| 25 | < 1e-07 | -16.649 | 276.9875639 | 1401.0181511 | 0.1977045 | 21261 at | DTX4 | deltex homolog 4 (Drosophila) |
| 26 | < 1e-07 | -16.537 | 77.1855073 | 797.9308868 | 0.0967321 | 204124 at | SLC34 A2 | solute carrier family 34 (sodium phosphate), member 2 |
| 27 | < 1e-07 | -16.074 | 50.920028 | 303.6319947 | 0.1677031 | 206765 at | KCNJ2 | potassium inwardly- |
| | | | | | | | | rectifying channel, subfamily J, member 2 |
| 28 | < 1e-07 | -16.012 | 61.9292201 | 408.1851782 | 0.1517184 | 213423 x a t | TUSC3 | tumour suppressor candidate 3 |
| 29 | < 1e-07 | -15.924 | 218.8096017 | 551.8606066 | 0.3964943 | 202826 at | SPINT 1 | serine peptidase inhibitor, Kunitz type 1 |
| 30 | < 1e-07 | -15.741 | 22.400886 | 103.2315393 | 0.2169965 | 205485 at | RYR1 | ryanodine receptor 1 (skeletal) |
| 31 | < 1e-07 | -15.607 | 126.6392388 | 508.3788054 | 0.2491041 | 203854 at | CFI | complement factor I |
| 32 | < 1e-07 | -15.592 | 87.8832396 | 410.0966115 | 0.2142989 | 218313 s at | GALN T7 | UDP-N-acetyl-alpha-D-galactosamine:polypepti de N-acetylgalactosaminyltran sferase 7 (GalNAc-T7) |
| 33 | < 1e-07 | -15.294 | 107.8973873 | 771.2100355 | 0.1399066 | 207808 s at | PROS 1 | protein S (alpha) |
| 34 | < 1e-07 | -15.038 | 43.5995677 | 339.1970953 | 0.1285376 | 211564 s at | PDLI M4 | PDZ and LIM domain 4 |
| 35 | < 1e-07 | -14.726 | 33.5237794 | 581.3084257 | 0.0576695 | 209395 at | CHI3L 1 | chitinase 3-like 1 (cartilage glycoprotein-39) |
| 36 | < 1e-07 | -14.703 | 60.1960441 | 232.3023615 | 0.259128 | 1222108 at | AMIG O2 | adhesion molecule with Ig-like domain 2 |
| 37 | < 1e-07 | -14.646 | 22.1974582 | 132.6830295 | 0.1672969 | 205954 at | RXRG | retinoid X receptor, gamma |
| 38 | < 1e-07 | -14.621 | 96.485612 | 363.789484 | 0.2652238 | 209035 at | MDK | midkine (neurite growth-promoting factor 2) |
| 39 | < 1e-07 | -14.526 | 42.3689506 | 151.4565321 | 0.2797433 | 204105 s at | NRCA M | neuronal cell adhesion molecule |
| 40 | < 1e-07 | -14.192 | 205.2367414 | 763.6988023 | 0.2687404 | 202458 at | PRSS2 3 | protease, serine, 23 |
| 41 | < 1e-07 | -14.032 | 41.0017305 | 656.0854583 | 0.0624945 | 209396 s at | CHI3L | chitinase 3-like 1 (cartilage glycoprotein-39) |
| 42 | < 1e-07 | -14.001 | 71.1833397 | 239.2342156 | 0.2975467 | 202599 s at | NRIPI | nuclear receptor interacting protein 1 |
| 43 | < 1e-07 | -13.885 | 25.4355462 | 260.4675782 | 0.0976534 | 221577 x a t | DF15 | growth differentiation factor 15 |
| 44 | < 1e-07 | -13.708 | 53.3900873 | 312.2115254 | 0.1710061 | 211668 s at | PLAU | plasminogen activator, urokinase |
| 45 | < 1e-07 | -13.443 | 75.8442425 | 974.7983502 | 0.07785051 | 209810 at | SFTPB | surfactant protein B |
| 46 | < 1e-07 | -13.411 | 163.061707 | 566.7623162 | 0.2877074 i | 218368 s at | TNFR SF12A | tumour necrosis factor receptor superfamily, member 12A |
| 47 | < 1e-07 | -13.32 | 41.9630727 | 296.3955635 | 0.1415779 | 219630 at | PDZK 1IP1 | PDZK1 interacting protein 1 |
| 48 | < 1e-07 | -13.107 | 35.8865404 | 127.6986991 | 0.2810251 | 209803 s at | PHLD A2 | homology-like domain, family A, member 2 |
| 49 | < 1e-07 | -13.098 | 1174.3487411 | 3735.174612 | 0.3144026 | 201012 at | ANXA 1 | annexin A1 |
| 50 | < 1e-07 | -13.037 | 48.9151583 | 652.177119 | 0.0750029 | 37004 at | SFTPB | surfactant protein B |
| 51 | < 1e-07 | -12.769 | 88.6618728 | 841.6519994 | 0.1053427 | 207144 s at | CITED 1 | Cbp/p300-interacting transactivator, with Glu/Asp-rich carboxy-terminal domain, 1 |
| 52 | < 1e-07 | -12.697 | 148.7346284 | 549.0447911 | 0.2708971 | 202609 at | EPS8 | epidermal growth factor receptor pathway substrate 8 |
| 53 | < 1e-07 | -12.686 | 119.0097119 | 786.9782889 | 0.1512236 | 206595 at | CST6 | cystatin E/M |
| 54 | < 1e-07 | -12.675 | 137.7710544 | 733.1679965 | 0.187912 | 218309 at | CAMK 2N1 | calcium/calmodulin-dependent protein kinase II inhibitor 1 |
| 55 | < 1e-07 | -12.398 | 38.3206042 | 162.5229832 | 0.2357858 | 209474 s at I | ENTP D1 | ectonucleoside triphosphate diphosphohydrolase 1 |
| 56 | < 1e-07 | -12.151 | 131.4103827 | 364.1073522 | 0.3609111 | 218856 at | TNFR SF21 | tumour necrosis factor receptor superfamily, member 21 |
| 57 | < 1e-07 | -12.112 | 35.4245448 | 106.5406362 | 0.332498 | 220332 at | CLDN 16 | claudin 16 |
| 58 | < 1e-07 | -11.806 | 179.4757608 | 963.1121493 | 0.1863498 | 203413 at | NELL2 | NEL-like 2 (chicken) |
| 59 | < 1e-07 | -11.68 | 298.8592846 | 786.7601434 | 0.3798607 | 201417 at | SOX4 | SRY (sex determining region Y)-box 4 |
| 60 | < 1e-07 | -11.392 | 43.0464555 | 94.5732307 | 0.4551653 | 206574 s at | PTP4A 3 | protein tyrosine phosphatase type IVA, member 3 |
| 61 | < 1e-07 | -10.637 | 288.0020872 | 1193.8782386 | 0.2412324 | 202404 s at | COL1 A2 | collagen, type I, alpha 2 |
| 62 | < 1e-07 | -10.606 | 35.3711207 | 68.7772724 | 0.514285 | 203986 at | STBD1 | starch binding domain 1 |
| 63 | < 1e-07 | -10.477 | 53.9209563 | 377.559435 | 0.1428145 | 207695 s at | IGSFI | immunoglobulin superfamily, member 1 |
| 64 | < 1e-07 | 10.351 | 186.2958357 | 432.4831288 | 0.4307586 | 202145 at | LY6E | lymphocyte antigen 6 complex, locus E |
| 65 | < 1e-07 | -10.324 | 62.2172164 | 250.4085414 | 0.2484628 | 213936 x a t | SFTPB | surfactant protein B |
| 66 | < 1e-07 | -10.126 | 49.3938002 | 164.5406246 | 0.3001921 | 217077 s at | GABB R2 R2 | gamma-aminobutyric acid (GABA) B receptor, 2 |
| 67 | < 1e-07 | -10.023 | 14.3587161 | 64.9690194 | 0.2210087 | 219932 at | SLC27 A6 | solute carrier family 27 (fatty acid transporter), member 6 |
| 68 | < 1e-07 | -9.695 | 80.8374329 | 256.874519 | 0.3146962 | 211679 x a t | GABB R2 | gamma-aminobutyric acid (GABA) B receptor, 2 |
| 69 | < 1e-07 | -9.688 | 24.9460924 | 50.0389635 | 0.4985334 | 218720 x a t | SEZ6L 2 | seizure related 6 homolog (mouse)-like 2 |
| 70 | < 1e-07 | -9.088 | 87.0520558 | 244.443968 | 0.3561227 | 202912 at | ADM | adrenomedullin |
| 71 | < 1e-07 | -8.866 | 90.6230699 | 329.0128241 | 0.2754393 | 218872 at | TESC | tescalcin |
| 72 | < 1e-07 | -8.866 | 255.9256144 | 1188.9727815 | 0.2152493 | 202310 s at | COL1 A1 | collagen, type I, alpha 1 |
| 73 | < 1e-07 | -8.863 | 111.389368 | 440.5005533 | 0.25287 | 209365 s at | ECM1 | extracellular matrix protein 1 |
| 74 | < 1e-07 | -8.782 | 143.6376638 | 664.6449246 | 0.2161119 | 210809 s at | POST N | periostin, osteoblast specific factor |
| 75 | < 1e-07 | -8.42 | 1352.9900515 | 3098.6410578 | 0.4366398 | 215111 s at | TSC22 D1 | TSC22 domain family, member 1 |
| 76 | < 1e-07 | -8.309 | 62.453463 | 153.8477989 | 0.4059432 | 202363 at | SPOC K1 | sparc/osteonectin, cwcv and kazal-like domains proteoglycan (testican) 1 |
| 77 | < 1e-07 | -8.004 | 39.9523842 | 90.3880414 | 0.4420096 | 209529 at | PPAP2 C | phosphatidic acid phosphatase type 2C |
| 78 | < 1e-07 | -7.946 | 93.0043195 | 296.8437589 | 0.3133107 | 204268 at | S100A 2 | S100 calcium binding protein A2 -1 |
| 79 | < 1e-07 | -7.87 | 551.6081523 | 1568.9242656 | 0.3515837 | 201852 x a t | COL3 A1 | collagen, type III, alpha 1 |
| 80 | < 1e-07 | -7.409 | 58.8051647 | 325.8897132 | 0.180445 | 218002 s at | CXCL 14 | chemokine (C-X-C motif) ligand 14 |
| 81 | < 1e-07 | -7.361 | 21.1842381 | 50.1660694 | 0.4222822 | 203757 s at | CEAC AM6 | carcinoembryonic antigen-related cell adhesion molecule 6 (non-specific cross reacting antigen) |
| 82 | < 1e-07 | -7.152 | 48.5343904 | 127.5872172 | 0.3804017 | 206315 at | CRLF1 | cytokine receptor-like factor 1 |
| 83 | < 1e-07 | -7.016 | 46.5947908 | 77.2369884 | 0.6032704 | 210904 s at | IL13R A1 | interleukin 13 receptor, alpha 1 |
| 84 | < 1e-07 | -7.005 | 16.3084571 | 40.1247366 | 0.406444 | 220595 at | PDZR N4 | PDZ domain containing ring finger 4 |
| 85 | < 1e-07 | -6.993 | 52.8608974 | 130.9252911 | 0.4037486 | 219250 s at | FLRT3 | fibronectin leucine rich transmembrane protein 3 |
| 86 | < 1e-07 | -6.63 | 1398.1216866 | 2898.1167543 | 0.4824242 | 202403 s at | COL1 A2 | collagen, type I, alpha 2 |
| 87 | < 1e-07 | -6.531 | 106.8996187 | 181.0594937 | 0.5904116 | 212012 at | PXDN | peroxidasin homolog (Drosophila) |
| 88 | < 1e-07 | -6.514 | 354.3383607 | 899.7373003 | 0.3938242 | 203851 at | IGFBP 6 | insulin-like growth factor binding protein 6 |
| 89 | < 1e-07 | -6.479 | 44.0394885 | 89.9455484 | 0.4896239 | 211657 at | CEAC AM6 | carcinoembryonic antigen-related cell adhesion molecule 6 (non-specific cross reacting antigen) |
| 90 | < 1e-07 | -6.374 | 120.2118656 | 197.8522978 | 0.6075839 | 200637 s at | PTPRF | protein tyrosine phosphatase, receptor type, F |
| 91 | < 1e-07 | -6.336 | 81.5682316 | 134.5967737 | 0.6060192 | 221009 s at | ANGP TL4 | angiopoietin-like 4 |
| 92 | <1e-07 | -5.96 | 25.0349373 | 48.9351762 | 0.5115939 | 204714 s at | F5 | coagulation factor V (proaccelerin, labile factor) |
| 93 | 6e-07 | -5.311 | 609.3166647 | 1448.6566594 | 0.4206081 | 217767 at | C3 | complement component 3 |
| 94 | 1.1e-06 | -5.166 | 59.0858976 | J99.210437 | 0.5955613 | 210735 s at | CA12 | carbonic anhydrase XII |
| 95 | 4.3e-06 | -4.837 | 30.4129302 | 67.5713604 | 0.4500861 | 218835 at | SFTPA 2B | surfactant protein A2B |
| 96 | 7.2e-06 | -4.714 | 40.1435633 | 53.6088762 | 0.7488231 | 213611 at | AQP5 | aquaporin 5 |
| 97 | 1.26e-05 | -4.575 | 78.921958 | 160.1544425 | 0.4927866 | 203963 at | CA12 CA12 | carbonic anhydrase XII |
| 98 | 2.28e-05 | -4.425 | 29.2412988 | 47.8975518 | 0.6104967 | 204508 s at CA12 | CA12 | carbonic anhydrase XII |
| 99 | 0.00017 35 | -3.888 | 65.4661585 | 104.1890626 | 0.62834 | 206626 x a t | SSX1 | synovial sarcoma, X breakpoint 1 |
| 100 | 0.00025 96 | -3.775 | 113.0238857 | 210.9971989 | 0.5356653 | 214164 × a t | CA12 | carbonic anhydrase XII |
| 101 | 0.00039 44 | -3.656 | 123.5873351 | 230.1897864 | 0.5368932 | 215867 x a t | CA12 | carbonic anhydrase XII |
| 102 | 0.00159 36 | -3.238 | 88.4144134 | 122.7313505 | 0.7203898 | 214070 s at | ATP10 B | ATPase, class V, type 10B |
| 103 | 0.00324 35 | -3.01 | 71.0585074 | 95.3895969 | 0.7449293 | 210394 × a t | SSX4 | synovial sarcoma, X breakpoint 4 |
| 104 | 0.00406 54 | -2.935 | 24.5175333 | 33.0486624 | 0.7418616 | 204540 at | EEF1A 2 | translation elongation factor 1 alpha 2 |
| 105 | 0.00411 64 | -2.93 | 52.2078042 | 68.0432433 | 0.7672739 | 211425 x a t | SSX4B | synovial sarcoma, X breakpoint 4B |
| 106 | 0.03233 28 | -2.168 | 34.4358337 | 45.7918842 | 0.7520074 | 204483 at | ENO3 | enolase 3 (beta, muscle) |
| 107 | 0.03586 79 | -2.125 | 33.8499233 | 41.7747704 | 0.8102959 | 220630s at | CHIA | chitinase, acidic |
| 108 | 0.03836 11 | -2.096 | 11.1044384 | 13.1772735 | 0.8426962 | 213436 at | CNR1 | cannabinoid receptor I (brain) |
| 109 | 0.07574 62 | -1.793 | 108.284083 | 145.3815104 | 0.7448271 | 204533 at | CXCL 10 | chemokine (C-X-C motif) ligand 10 |
| 110 | 0.19131 51 | -1.315 | 112.3046774 | 129.2893824 | 0.8686303 | 206227 at 206227 at | CILP | cartilage intermediate layer protein, nucleotide pyrophosphohydrolase |
| 111 | 0.41821 67 | -0.813 | 188.150322 | 228.2925593 | 0.8241632 | 203915 at | CXCL 9 | chemokine CXCL motif) ligand 9 |
| 112 | 0.42344 94 | -0.803 | 25.4173537 | 26.9284631 | 0.9438843 | 205597 at | SLC44 A4 | solute carrier family 44, member 4 |
| 113 | 0.52125 01 | -0.643 | 44.6063567 | 53.4448853 | 0.8346235 | 205242 at | CXCL 13 | chemokine (C-X-C motif) ligand 13 |
| 114 | 0.66278 6 | -0.437 | 112.4390995 | 124.6328006 | 0.902163 | 205890 s at | UBD | ubiquitin D |
| 115 | 0.92132 64 | -0.099 | 109.2837793 | 112.4718382 | 0.9716546 | 201909 at | RPS4Y 1 | ribosomal protein S4, Y-linked 1 |
| 116 | 0.97085 67 | -0.037 | 720.5725927 | 731.3407587 | 0.9852761 | AFFX-r2-Hs18SrRNA -3 s at | NA | NA |
| 117 | 0.87934 16 | 0.152 | 453.5890335 | 433.7740742 | 1.0456804 t | 221728 x a | XIST | X (inactive)-specific transcript (non-protein coding) |
| 118 | 0.70313 73 | 0.382 | 237.4267503 | 213.9129435 | 1.1099223 | 214218 s at | XIST | X (inactive)-specific transcript (non-protein coding) |
| 119 | 110.70127 | 10.385 | 87.0355115 | 83.9978198 | 1.0361639 | 218807 at | VAV3 | vav 3 guanine nucleotide exchange factor |
| 120 | 0.53072 86 | 0.629 | 90.1320161 | 80.1994241 | 1.1238487 | 205960 at | PDK4 | pyruvate dehydrogenase kinase, isozyme 4 |
| 121 | 0.36417 67 | 0.911 | 17.3524146 | 15.9623369 | 1.0870848 | 205442 at | MFAP 3L | microfibrillar-associated protein 3-like |
| 12 2 | 0.32546 87 | 0.988 | 88.0799616 | 77.0338169 | 1.1433934 | 213939 s at | at RUFY 3 | RUN and FYVE domain containing 3 |
| 123 | 0.22493 46 3 46 | 1.22 | 101.0586075 | 84.3338551 | 1.198316 | 206115 at | EGR3 EGF3 | early growth response 3 |
| 124 | 0.12161 43 | 1.56 | 307.7701803 | 259.1673184 | 1.1875347 | 218706 s at | D3 | GRAM domain containing 3 |
| 125 | 0.07192 43 | 1.817 | 71.0521509 | 58.6362122 | 1.2117452 | 205518 s | at CMAH | cytidine monophosphate-N-acetylneuraminic acid hydroxylase (CMP-N-acetylneuraminate monooxygenase) pseudogene |
| 126 | 0.07162 61 | 1.819 | 58.9427298 | 29.0555122 | 2.0286247 | AFFX-r2-Hs18SrRNA -5 at | NA | NA |
| 127 | 0.04612 62 | 2.017 | 92.2999562 | 48.7523554 | 1.893241 | AFFX-HUMRGE/ M10098 5 NA at | NA | NA |
| 128 | 0.03544 73 | 2.129 | 38.3960791 | 27.6402742 | 1.3891352 | 206211 at | SELE | selectin E |
| 129 | 0.01949 79 | 2.371 | 70 9672092 | 52.6156479 | 1.3487852 | 213998 s | at DDX1 7 | DEAD (Asp-Glu-Ala-Asp) box polypeptide 17 |
| 130 | 0.0127834 | 2.531 0 | 53.3250655 | 36.6428544 | 1.4552651 | 213593 s at | at TRA2 | transformer 2 alpha homolog (Drosophila) |
| 131 | 13 0.01226 1 86 | 2.546 | 373.8977509 | 250.9680635 | 1.489822 | 202269 x at | GBP1 | guanylate binding otein 1, interferon-inducible, 67kDa |
| 132 | 0.0004617 | 3.351 | 99.4113503 | 59.6338658 | 1.6670284 | 222073 at | COL4 A3 | collagen, type IV, alpha 3 (Goodpasture antigen) |
| 133 | 0.00046 17 | 3.611 | 129.4679886 | 89.2178191 | 1.451145 | 203708 at | PDE4B | phosphodiesterase 4B, cAMP-specific (phosphodiesterase E4 dunce homolog, Drosophila) |
| 134 | 9.19e-05 | 4.061 | 60.8782439 | 144.8302308 | 1.357973 | 205464 at | SCNN 1B | sodium channel, nonvoltage-gated 1, beta |
| 13 5 | 3.9e-06 | 4.863 | 2082.3545956 | 957.0298041 | 2.1758514 | 1201893 x t | a DCN | decorin |
| 136 | 3.7e-06 | 4.872 | 802.9202611 | 310.6917297 | 2.5842988 | 211896 s at | DCN | decorin |
| 137 | 1.7e-06 | 5.057 | 475.8526167 | 234.3693257 | 2.0303537 | 205249 at | EGR2 | early growth response 2 (Krox-20 homolog, Drosophila) |
| 138 | 1e-06 | 5.183 | 6670.2691839 | 320.397693 | 2.0919913 | 205549 at | PCP4 | Purkinje cell protein 4 |
| 139 | 6e-07 | 5.29 | 289.4158236 | 176.489672 | 1.6398457 | 213106 at | ATP8 A1 | ATPase, aminophospholipid transporter (APLT), class I, type 8A, member 1 |
| 140 | 2e-07 | 5.592 | 738.0366247 | 405.5736072 | .8197351 | 218723 s at | C13orf 15 | chromosome 13 open reading frame 15 |
| 141 | < 1e-07 | 5.934 | 576.2618069 | 236.0518526 | 2.4412509 | 213917 at | PAX8 | paired box 8 |
| 142 | < 1e-07 | 5.979 | 179.8091434 | 85.2885404 | 2.1082451 | 202018 s at | LTF | lactotransferrin |
| 143 | < 1e-07 | 6.452 | 512.9453458 | 209.5194034 | 2.4481997 | 201693 s at | EGR1 | early growth response 1 |
| 144 | < 1e-07 | 6.462 | 197.4824557 | 144.2592461 | 1.3689414 | 213295 at | CYLD | cylindromatosis (turban tumour syndrome) |
| 145 | < 1e-07 | 6.618 | 246.548183 | 140.4312356 | 1.7556506 | 210260 s at | TNFAI P8 | tumour necrosis factor, alpha-induced protein 8 |
| 146 | < 1e-07 | 6.772 | 285.9104125 | 78.5358773 | 3.640507 | 214977 at | NA | NA |
| 147 | < 1e-07 | 7.073 | 442.3363271 | 255.1319965 | 1.7337548 | 213016 at | BBX | bobby sox homolog (Drosophila) |
| 14 8 | <1e-07 | 7.114 | 1029.659478 | 531.6673366 | 1.9366612 | 203554 x a t | PTTG1 | pituitary tumour-transforming 1 |
| 149 | < 1e-07 | 7.462 | 476.4115277 | 284.1955259 | 1.6763513 | 204079 at | TPST2 | tyrosylprotein sulfotransferase 2 |
| 150 | < 1e-07 | 7.713 | 459.4429613 | 215.708618 | 2.129924 | 213229 at | DICER 1 | ribonuclease type III |
| 151 | < 1e-07 | 8.459 | 232.9178471 | 100.4922197 | 2.31777 | 214696 at | C17orf 91 | chromosome 17 open reading frame 91 |
| 152 | < 1e-07 | 8.534 | 301.4026655 | 134.9367944 | 2.2336581 | 204793 at | GPRA SP1 | G protein-coupled receptor associated sorting protein 1 |
| 153 | < 1e-07 | 8.919 | 379.0281593 | 150.6545048 | 2.5158767 | 202746 at | ITM2A | integral membrane protein 2A |
| 154 | < 1e-07 | 9.061 | 1225.450726 | 520.9279387 | 2.3524381 | 212158 at | SDC2 | syndecan 2 |
| 155 | < 1e-07 | 9.261 | 97.1827947 | 31.518784 | 3.0833294 | 210078 s at | KCNA B1 | potassium voltage-gated channel, shaker-related subfamily, beta member 1 |
| 156 | < 1e-07 | 9.316 | 186.1646663 | 78.6808135 | 2.3660745 | 206061 s at | DICER 1 | dicer 1, ribonuclease type III |
| 157 | < 1e-07 | 10.167 | 356.2847982 | 140.396192 | 2.5377098 | 204612 at | PKIA | protein kinase (cAMP-dependent, catalytic) inhibitor alpha |
| 158 | < 1e-07 | 10.196 | 1266.9935628 | 533.4835495 | 2.374944 | 213737 x a t | GOLG A9P | golgi autoantigen, golgin subfamily a, 9 pseudogene |
| 159 | < 1e-07 | 10.225 | 1168.2073553 | 327.3431582 | 3.5687545 | 209230 s at | NUPR 1 | nuclear protein 1 |
| 16 0 | < 1e-07 | 10.584 | 708.5341498 | 246.6087207 | 2.8731107 | 212157 at | SDC2 | syndecan 2 |
| 161 | < 1e-07 | 11.234 | 4117.901303 | 605.5238546 | 6.80056 | 209116 × a t | HBB | hemoglobin, beta |
| 162 | < 1e-07 | 11.245 | 6263.1703561 | 1155.3546555 | 5.4209937 | 211745 × a t | HBA1 | hemoglobin, alpha 1 |
| 163 | < 1e-07 | 11.429 | 5422.9833204 | 1000.6071666 | 5.4196927 | 211696 x a t | HBB | hemoglobin, beta |
| 164 | < 1e-07 | 11.518 | 71.0640697 | 23.3679617 | 3.0410898 | 204932 at | TNFR SF11B | tumour necrosis factor receptor superfamily, member 11b |
| 165 | < 1e-07 | 11.532 | 722.2663249 | 327.2523947 | 2.207062 | 215691 x a t | HSPB1 1 | heat shock protein family B (small), member 11 |
| 166 | < 1e-07 | 11.551 | 74.9566138 | 32.0097607 | 2.3416799 | 219029 at | C5orf2 8 | chromosome 5 open reading frame 28 |
| 167 | < 1e-07 | 11.57 | 5646.7049093 | 1010.140344 | 5.5900202 | 211699 x a t | HBA1 | hemoglobin, alpha 1 |
| 168 | < 1e-07 | 11.607 | 670.1717793 | 143.2051924 | 4.6798008 | 209292 at | ID4 | inhibitor of DNA binding 4, dominant negative helix-loop-helix protein |
| 169 | < 1e-07 | 11.617 | 5612.4671569 | 1009.0765873 | 5.5619833 | 209458 x a t | HBA1 | hemoglobin, alpha 1 hemoglobin, 1 |
| 170 | < 1e-07 | 11.734 | 285.1442933 | 138.1718589 | 2.063693 | 220987 s at | NUAK 2 | NUAK family, SNF1-like kinase, 2 |
| 171 | < 1e-07 | 11.752 | 4758.1247654 | 845.780775 | 5.6257188 | 214414 x a t | HBA1 | hemoglobin, alpha 1 |
| 172 | < 1e-07 | 11.755 | 5341.6306796 | 965.9200008 | 5.5300964 | 217414 x a t | HBA1 | hemoglobin, alpha 1 |
| 173 | < 1e-07 | 11.793 | 654.7088121 | 218.1656268 | 3.0009714 | 209325 s at | RGS16 | regulator of G-protein signaling 16 |
| 174 | < 1e-07 | 11.837 | 299.2638457 | 85.5265239 | 3.4990765 | 205066 s at | ENPPI' | ectonucleotide pyrophosphatase/phosph odiesterase 1 |
| 175 | < 1e-07 | 11.91 | 5829.5196186 | 1072.8108539 | 5.4338746 | 204018 x a t | HBA1 | hemoglobin, alpha 1 |
| 176 | < 1e-07 | 12.016 | 5858.0042653 | 1034.2290438 | 5.6641266 | 217232 x a t | HBB | hemoglobin, beta |
| 177 | < 1e-07 | 12.291 | 303.4278409 | 81.3956638 | 3.7278133 | 213664 at | SLC1A 1 | solute carrier family 1 (neuronal/epithelial high affinity glutamate transporter, system Xag), member 1 |
| 178 | < 1e-07 | 12.293 | 82.4566835 | 25.5260781 | 3.2302919 | 205357 s at | AGTR 1 | angiotensin II receptor, type |
| 179 | < 1e-07 | 12.693 | 130.0540686 | 34.0522917 | 3.8192457 | 219778 at | ZFPM 2 | zinc finger protein, multitype 2 |
| 180 | < 1e-07 | 13.147 | 593.9084299 | 120.2305719 | 4.9397455 | 203699 s at | DIO2 | deiodinase, iodothyronine, type II |
| 181 | < 1e-07 | 13.275 | 147.1106724 | 44.2872646 | 3.3217376 | 209242 at | PEG3 | paternally expressed 3 |
| 182 | < 1e-07 | 13.399 | 357.7696352 | 107.6138534 | 3.3245686 | 206167 s at | ARHG AP6 | ho GTPase activating protein 6 |
| 183 | < 1e-07 | 13.712 | 3402.1375882 | 753.6338797 | 4.5143108 | 203240 at | FCGB Fc P | fragment of IgG binding protein |
| 184 | < 1e-07 | 13.846 | 330.6315645 | 70.7236111 | 4.6749814 | 206209 s at | CA4 | carbonic anhydrase IV |
| 185 | < 1e-07 | 13.96 | 264.7405774 | 47.0264339 | 5.629612 | 204933 s at | TNFR SFIIB | tumour necrosis factor receptor superfamily, member 11b |
| 186 | < 1e-07 | 13.97 | 68.2775199 | 14.4476206 | 4.7258661 | 209469 at | A | GPM6 glycoprotein M6A |
| 187 | < 1e-07 | 14.057 | 256.2577098 | 39.5520345 | 6.479002 | 211276 at | TCEA L2 | transcription elongation factor A (SII)-like 2 |
| 188 | < 1e-07 | 14.077 | 664.2120327 | 72.6645605 | 9.1407975 | 203980 at | FABP4 | fatty acid binding protein 4, adipocyte |
| 189 | < 1e-07 | 14.222 | 1074.069004 | 49.8437255 | 21.5487304 | 206457 s at | DIO1 | deiodinase, iodothyronine, type 1 |
| 190 | < 1e-07 | 14.823 | 126.5557347 | 38.4315705 | 3.2930149 | 210400 at | C | glutamate receptor, ionotropic,N-methyl D-aspartate 2C |
| 191 | < 1e-07 | 15.422 | 434.9220085 | 56.2031854 | 7.7383872 | 206529 x a t | SLC26 A4 | solute carrier family 26, member 4 |
| 192 | < 1e-07 | 16.239 | 4406.7519936 | 180.2130832 | 24.4530082 | 210342 s at | TPO | thyroid peroxidase |
| 193 | < 1e-07 | 17.429 | 1345.1171917 | 128.1065158 | 10.4999904 | 202350 s at | MATN, 2 | MATN matrilin 2 |
| 194 | < 1e-07 | 18.718 | 439.7182194 | 58.6240206 | 7.5006493 | 214735 at | IPCEF 1 factors | interaction protein for 1 cytohesin exchange 1 |
| 195 | < 1e-07 | 19.131 | 1261.3403813 | 177.5325808 | 7.1048389 | 219049 at | CSGA T1 | chondroitin sulfate N-LNAC acetylgalactosaminyltran sferase 1 |
| 196 | < 1e-07 | 19.412 | 590.2414111 | 69.5384989 | 8.4879803 | 205051 s at | KIT | v-kit Hardy-Zuckerman 4 feline sarcoma viral oncogene homolog |
| 197 | < 1e-07 | 19.539 | 1398.3873774 | 241.3729173 | 5.7934726 | 204288 s | S2 | at SORB sorbin and SH3 domain containing 2 |
| 198 | < 1e-07 | 21.353 | 296.2744319 | 43.386672 | 6.8286969 | 205221 at | HGD | homogentisate 1,2- dioxygenase (homogentisate oxidase) |
| 199 | < 9 1e-07 | 25.818 | 3269.354958 | 103.0213817 | 31.7347225 | 204623 at | TFF3 | trefoil factor 3 (intestinal) |
| 200 | < 1e-07 | 26.104 | 253.1369352 | 26.5717246 | 9.5265527 | 205413 at | D2 | MPPE metallophosphoesterase domain containing 2 |

To obtain a sufficient class prediction method we used an SVM technique based on the expression of 200 selected genes. To build a prediction rule, as defined by the inner sum of the weights (w*ᵢ*) and log2- we transformed the expression (x*ᵢ*) of significant genes. The expression values are log intensities of the microarray signal. A sample is classified to the class normal/benign if the sum is greater than the threshold; that is the Σ*ᵢ*w*ᵢ* x*ᵢ* > threshold. The threshold for the Support Vector Machine predictor is -1.087.

**Table 6.**

| Gene Weights | Genes | Support Vector Machines |
|---|---|---|
| 1 | 202286_s_at | -0.0163 |
| 2 | 212850_s_at | -0.0145 |
| 3 | 210342_s_at | 0.0126 |
| 4 | 204623_at | 0.0124 |
| 5 | 202833_s_at | -0.012 |
| 6 | 203413_at | -0.0115 |
| 7 | 209810_at | -0.0116 |
| 8 | 201650_at | -0.011 |
| 9 | 220595_at | -0.011 |
| 10 | 211719_x_at | -0.0109 |
| 11 | 37004_at | -0.011 |
| 12 | 216442_x_at | -0.0107 |
| 13 | 212464_s_at | -0.0105 |
| 14 | 209395_at | -0.0103 |
| 15 | 203980_at | 0.0105 |
| 16 | 21045_x_at | -0.0102 |
| 17 | 206457_s_at | 0.0102 |
| 18 | 209396_s_at | -0.0099 |
| 19 | 211478_s_at | -0.0099 |
| 20 | 219049_at | 0.0099 |
| 21 | 203716_s_at | -0.0099 |
| 22 | 205954_at | -0.0099 |
| 23 | 209116_x_at | 0.0098 |
| 24 | 206529_x_at | 0.0095 |
| 25 | 218835_at | -0.0091 |
| 26 | 214414_x_at | 0.0093 |
| 27 | 205174_s_at | -0.0087 |
| 28 | 206315_at | -0.0087 |
| 29 | 208025_s_at | -0.0087 |
| 30 | 219630_at | -0.0088 |
| 31 | 205549_at | 0.0086 |
| 32 | 206765_at | -0.0087 |
| 33 | 209365_s_at | -0.0089 |
| 34 | 211696_x_at | 0.0087 |
| 35 | 211429_s_at | -0.0084 |
| 36 | 204714_s_at | -0.0083 |
| 37 | 214977_at | 0.0084 |
| 38 | 204018_x_at | 0.0083 |
| 39 | 214218_s_at | 0.0082 |
| 40 | 209458_x at | 0.0081 |
| 41 | 205413_at | 0.0081 |
| 42 | 211745_x_at | 0.0081 |
| 43 | 211699_x_at | 0.0081 |
| 44 | 203717_at | -0.008 |
| 45 | 217414_x_at | 0.0079 |
| 46 | 217232_x_at | 0.0079 |
| 47 | 203256_at | -0.0078 |
| 48 | 204288_s_at | 0.0077 |
| 49 | 206595_at | -0.0075 |
| 50 | 209035_at | -0.0077 |
| 51 | 202746_at | 0.0077 |
| 52 | 201909_at | -0.0075 |
| 53 | 218872_at | -0.0074 |
| 54 | 221728_x_at | 0.0075 |
| 55 | 203854_at | -0.0075 |
| 56 | 207808_s_at | -0.0073 |
| 57 | 209292_at | 0.0073 |
| 58 | 222073_at | 0.0072 |
| 59 | 221577_x_at | -0.0073 |
| 60 | AFFX-r2-Hs18SrRNA-5_at | 0.0072 |
| 61 | 203757_s_at | -0.0071 |
| 62 | 206626_x_at | -0.0072 |
| 63 | 209529_at | -0.0072 |
| 64 | 205066_s_at | 0.007 |
| 65 | 213229_at | 0.0071 |
| 66 | 205485_at | -0.0071 |
| 67 | 215867_x_at | -0.0069 |
| 68 | 205890_s_at | 0.0071 |
| 69 | AFFX-HUMRGE/M10098_5_at | 0.007 |
| 70 | 205242_at | 0.007 |
| 71 | 202912_at | -0.0069 |
| 72 | 203851_at | -0.0067 |
| 73 | 222108_at | -0.0068 |
| 74 | 218807_at | 0.0067 |
| 75 | 205016_at | -0.0068 |
| 76 | 213423_x_at | -0.0067 |
| 77 | 206115_at | 0.0068 |
| 78 | 204932_at | 0.0068 |
| 79 | 215691_x_at | 0.0068 |
| 80 | 209230_s_at | 0.0067 |
| 81 | 214070_s_at | -0.0068 |
| 82 | 204105_s_at | -0.0068 |
| 83 | 202363_at | -0.0066 |
| 84 | 209270_at | -0.0066 |
| 85 | 213737_x_at | 0.0066 |
| 86 | 206209_s_at | 0.0066 |
| 87 | 213106_at | 0.0063 |
| 88 | 203915_at | 0.0065 |
| 89 | 209325_s_at | 0.0064 |
| 90 | 220630_s_at | -0.0064 |
| 91 | 209803_s_at | -0.0062 |
| 92 | 204268_at | -0.0062 |
| 93 | 211896_s_at | 0.0067 |
| 94 | 205481_at | -0.0063 |
| 95 | 210735_s_at | -0.0063 |
| 96 | 217767_at | -0.0062 |
| 97 | 204540_at | -0.0064 |
| 98 | 202269_x_at | 0.0063 |
| 99 | AFFX-r2-Hs18SrRNA-3_s_at | 0.0062 |
| 100 | 203510_at | -0.0062 |
| 101 | 202310_s_at | -0.0061 |
| 102 | 211668_s_at | -0.0061 |
| 103 | 203554_x_at | 0.0061 |
| 104 | 205960_at | -0.006 |
| 105 | 205597_at | -0.0062 |
| 106 | 211657_at | -0.006 |
| 107 | 202458_at | -0.0062 |
| 108 | 202145_at | -0.0062 |
| 109 | 204124_at at | -0.0062 |
| 110 | 212611_at | -0.0062 |
| 111 | 205249_at | 0.0061 |
| 112 | 202404_s_at | -0.0059 |
| 113 | 203963_at | -0.006 |
| 114 | 214735_at | 0.0061 |
| 115 | 206061_s_at | 0.0061 |
| 116 | 210078_s_at | 0.0059 |
| 117 | 211425_x_at | -0.0059 |
| 118 | 203699_s_at | 0.006 |
| 119 | 209228__ x_at | -0.0059 |
| 120 | 204933_s_at | 0.006 |
| 121 | 207144_s_at | -0.0058 |
| 122 | 205051_s_at | 0.0059 |
| 123 | 205357_s_at | 0.006 |
| 124 | 206211_at | 0.0059 |
| 125 | 218856_at | -0.0058 |
| 126 | 201417_at | -0.0059 |
| 127 | 220332_at | -0.0058 |
| 128 | 204508_s_at | -0.0058 |
| 129 | 210260_s_at | 0.0058 |
| 130 | 210394_x_at | -0.0057 |
| 131 | 201693_s_at | 0.0058 |
| 132 | 206167_s_at | 0.0057 |
| 133 | 218309_at | -0.0057 |
| 134 | 221009_s_at | -0.0057 |
| 135 | 219250_s_at | -0.0057 |
| 136 | 213917_at | 0.0057 |
| 137 | 211276_at | 0.0057 |
| 138 | 210809_s_at | -0.0055 |
| 139 | 203780_at | -0.0055 |
| 140 | 212012_at | -0.0055 |
| 141 | 201012_at | -0.0055 |
| 142 | 202733_at | -0.0054 |
| 143 | 213998_s_at | 0.0056 |
| 144 | 217077_s_at | -0.0056 |
| 145 | 209242_at | 0.0056 |
| 146 | 213939_s_at | 0.0054 |
| 147 | 213664_at | 0.0054 |
| 148 | 200637_s_at | -0.0055 |
| 149 | 204533_at | 0.0055 |
| 150 | 202350_s_at | 0.0055 |
| 151 | 207695_s_at | -0.0055 |
| 152 | 209469_at | 0.0056 |
| 153 | 205442_at | -0.0054 |
| 154 | 218002_s-_at | -0.0053 |
| 155 | 214164_x_at | -0.0054 |
| 156 | 204793_at | -0.0055 |
| 157 | 206574_s_at | -0.0054 |
| 158 | 210904_s_at | -0.0054 |
| 159 | 201893_x_at | 0.0057 |
| 160 | 211679_x_at | -0.0054 |
| 161 | 205518_s_at | 0.0054 |
| 162 | 213611_at | -0.0053 |
| 163 | 213611_at | -0.0054 |
| 164 | 203240_at | 0.0053 |
| 165 | 202826_at | -0.0054 |
| 166 | 215111_s_ at | -0.0053 |
| 167 | 202599_s_at | -0.0053 |
| 168 | 202609_at | -0.0052 |
| 169 | 205464_at | -0.0053 |
| 170 | 213016_at | 0.0054 |
| 171 | 210400_at | 0.0053 |
| 172 | 204612_at | 0.0053 |
| 173 | 202018_s_at | 0.0052 |
| 174 | 204079_at | 0.0053 |
| 175 | 201852_x_at | -0.0051 |
| 176 | 218313_s_at | -0.0052 |
| 177 | 212158_at | 0.0053 |
| 178 | 211564_s_at | -0.0052 |
| 179 | 206227_at | -0.0052 |
| 180 | 205221_at | 0.0052 |
| 181 | 219029_at | 0.0052 |
| 182 | 209474_s_at | -0.0052 |
| 183 | 218368_s_at | -0.0052 |
| 184 | 204483_at | -0.0053 |
| 185 | 219778_at | 0.0054 |
| 186 | 218720_x at | -0.0051 |
| 187 | 202528_at | -0.0052 |
| 188 | 212157_at | 0.0052 |
| 189 | 213593_s_at | 0.0051 |
| 190 | 222173_s_at | -0.0052 |
| 191 | 203708_at | 0.0053 |
| 192 | 202403_s_at | 0.005 |
| 193 | 218706_s_at | 0.0051 |
| 194 | 203986_at | -0.0051 |
| 195 | 214696_at | 0.0052 |
| 196 | 213295_at | 0.0051 |
| 197 | 219932_at | -0.0052 |
| 198 | 218723_s_at | 0.0051 |
| 199 | 213936_x_at | -0.005 |
| 200 | 220987_s_at | 0.005 |

### Example 3

### The analysis of a subset obtained by gene expression analysis of 198 thyroid samples by HG-U133 Plus 2.0 microarrays

We analyzed also the subset obtained by gene expression analysis of 198 thyroid samples by HG-U133 Plus 2.0 microarrays. 37 samples were excluded, and the dataset contained 88 PTC and 73 normal/benign thyroid specimens. The dataset was transformed via SVM, and outlier samples (26 samples) were excluded, finally the analysis was carried out in 135 samples, 65 samples of normal/benign thyroid and 80 PTC samples. The Recursive Feature Elimination method was used to select 200 genes. The 0.632+ bootstrap cross-validation method was used to compute misclassification rate. When applied to HG-U133 Plus 2.0 data, this method has a sensitivity of 0.996, specificity 0.993, PPV 0.994 and NPV 0.995

**Table 7. List of genes differentiating bulk PTC tissue and benign/normal thyroid, as obtained from data on HG-U133 Plus 2.0 microarray**

| | Parame tric p-value | t-valu e | % CV supp ort | Geom mean of intensities in class 1 | Geom mean of intensities in class 2 | Fold-change | Probe set | Gene symbol | Description |
|---|---|---|---|---|---|---|---|---|---|
| 1 | < 1e-07 | 29.6 74 | 101 | 700.8386 421 | 8195.478 8617 | 0.08551 53 | 216442 x at | FN1 | fibronectin 1 |
| 2 | < 1e-07 | 29.6 65 | 101 | 737.1898 05 | 8234.769 1197 | 0.08952 16 | 210495 x at | FN1 | fibronectin 1 |
| 3 | < 1e-07 | 28.6 68 | 101 | 529.2648 896 | 6974.806 517 | 0.07588 24 | 212464 s at | FN1 | fibronectin 1 |
| 4 | < 1e-07 | 28.1 56 | 101 | 16.17325 84 | 231.6214 524 | 0.06982 63 | 211478 s at | DPP4 DPP4 | dipeptidyl-peptidase 4 |
| 5 | < 1e-07 | 28.0 8 | 101 | 12.65725 2 | 170.1882 391 | 0.07437 21 | 206884 s at | SCEL | sciellin |
| 6 | < 1e-07 | 27.1 82 | 101 | 29.56746 09 | 395.5763 866 | 0.07474 53 | 203717 at | DPP4 | dipeptidyl-peptidase 4 |
| 7 | < 1e-07 | 26.2 01 | 101 | 743.5611 779 | 8038.510 5392 | 0.09249 99 | 211719 x at | FN1 | fibronectin 1 |
| 8 | < 1e-07 | 25.7 5 | 101 | 29.63248 47 | 245.0211 55 | 0.12093 85 | 209270 at | LAMB3 | laminin, beta 3 |
| 9 | < 1e-07 | 24.9 42 | 101 | 24.79486 12 | 221.9821 68 | 0.11169 75 | 203716 s at | DPP4 | dipeptidyl-peptidase 4 |
| 10 | < 1e-07 | 24.9 15 | 101 | 30.29386 7 | 99.41902 32 | 0.30470 9 | 204669 s at | RNF24 | ring finger protein 24 |
| 11 | < 1e-07 | 24.6 04 | 101 | 331.9909 142 | 2057.337 1114 | 0.16136 92 | 203510 at | MET | met proto-oncogene (hepatocyte growth factor receptor) |
| 12 | < 1e-07 | 24.2 | 101 | 194.8202 725 | 1289.682 2946 | 0.15106 07 | 207808 s at | PROS 1 | protein S (alpha) |
| 13 | < 1e-07 | 24.2 35 | 101 | 58.18351 03 | 1259.665 5725 | 0.04618 96 | 207144 s at | CITED1 | Cbp/p300-interacting transactivator, with Glu/Asp-rich carboxy-terminal domain, 1 |
| 14 | < 1e-07 | 23.6 88 | 101 | 58.69138 84 | 405.4696 186 | 0.14474 92 | 203354 s at | PSD3 PSD3 | pleckstrin and Sec7 domain containing 3 |
| 15 | < 1e-07 | 23.4 54 | 101 | 33.14826 71 | 1121.323 9922 | 0.02956 17 | 209396 s at | CHI3L1 | chitinase 3-like 1 (cartilage glycoprotein-39) glycoprotein-39) |
| 16 | < 1e-07 | 23.3 73 | 101 | 17.74686 5 | 400.8726 038 | 0.04427 06 | 208025 s at | HMGA2 | high mobility group AT-hook 2 |
| 17 | < 1e-07 | 23.2 24 | 101 | 59.53290 4 | 749.2352 326 | 0.07945 82 | 202436 s at | CYP1B1 | cytochrome P450, family 1, subfamily B, polypeptide 1 |
| 18 | < 1e-07 | 23.0 87 | 101 | 32.64626 74 | 1448.907 7511 | 0.02253 16 | 209395 at | CHI3L1 | chitinase 3-like 1 (cartilage glycoprotein-39) |
| 19 | < 1e-07 | 23.0 62 | 101 | 28.96576 62 | 264.5781 303 | 0.10947 91 | 211564 s at | PDLIM4 | PDZ and LIM domain |
| 20 | < 1e-07 | 22.1 92 | 101 | 46.49736 05 | 710.4429 698 | 006544 84 | 212850 s at | LRP4 | low density lipoprotein receptor-related protein 4 |
| 21 | < 1e-07 | 22.1 11 | 101 | 50.45868 83 | 543.3451 067 | 0.09286 67 | 203355 s at | PSD3 | pleckstrin and Sec7 domain containing 3 |
| 22 | < 1e-07 | 21.7 97 | 101 | 56.27773 2 | 314.3847 735 | 0.17900 91 | 205016 at | TGFA | transforming growth factor, alpha |
| 23 | < 1e-07 | 21.6 77 | 101 | 52.54314 92 | 226.6523 894 | 0.23182 26 | x at | PDLIM4 | PDZ and LIM domain 4 |
| 24 | < 1e-07 | 21.6 59 | 101 | 22.77581 5 | 231.7800 36 | 0.09826 48 | 220332 at | CLDN1 6 | claudin 16 |
| 25 | < 1e-07 | 21.5 08 | 101 | 624.3185 014 | 2528.208 9141 | 0.24694 1 | 208891 at | DUSP6 | dual specificity phosphatase 6 |
| 26 | < 1e-07 | 21.4 14 | 101 | 76.92193 16 | 1675.702 1425 | 0.04590 43 | 202286 s at | TACST D2 | tumour-associated calcium signal transducer 2 |
| 27 | < 1e-07 | 21.3 26 | 101 | 37.00810 2 | 339.1283 259 | 0.10912 71 | 202435 s at | CYP1B1 | cytochrome P450, family 1, subfamily B, polypeptide 1 |
| 28 | < 1e-07 | 21.2 03 | 101 | 99.95026 16 | 578.7263 621 | 0.17270 73 | 218613 at | PSD3 | pleckstrin and Sec7 domain containing 3 |
| 29 | < 1e-07 | 20.9 59 | 101 | 18.40545 56 | 158.5039 526 | 0.11611 99 | 204014 at | DUSP4 | dual specificity phosphatase 4 |
| 30 | < 1e-07 | 20.2 7 | 101 | 20.74699 93 | 118.4092 1 | 0.17521 44 | 205485 at | RYR1 | ryanodine receptor (skeletal) |
| 31 | < 1e-07 | 19.7 33 | 101 | 243.6829 609 | 1425.448 2962 | 0.17095 18 | 212611 at | DTX4 | deltex homolog 4 (Drosophila) |
| 32 | < 1e-07 | 19.177 | 101 | 35.47897 19 | 444.5648 337 | 0.07980 61 | 206765 at | KCNJ2 | potassium inwardly-rectifying channel, subfamily J, member 2 |
| 33 | < 1e-07 | 18.9 24 | 101 | 58.32639 18 | 368.9579 324 | 0.15808 41 | 205174 s at | QPCT | glutaminyl-peptide cyclotransferase |
| 34 | < 1e-07 | 18.8 07 | 101 | 76.22296 69 | 305.7508 071 | 0.24929 77 | 221840 at | PTPRE | protein tyrosine phosphatase, receptor type, E |
| 35 | < 1e-07 | 18.6 56 | 101 | 15.76189 67 | 189.8149 757 | 0.08303 82 | 205728 at | NA | DNA |
| 36 | < 1e-07 | 18.6 22 | 101 | 61.49577 48 | 539.9894 751 | 0.11388 33 | 205479 s at | PLAU | lasminogen activator, urokinase |
| 37 | < 1e-07 | 18.5 28 | 101 | 222.9702 887 | 1241.645 4007 | 0.17957 65 | 208892 s at | DUSP6 | dual specificity phosphatase 6 |
| 38 | < 1e-07 | 18.4 79 | 101 | 260.3498 472 | 1634.660 1081 | 0.15926 85 | 208893 s at | DUSP6 | dual specificity phosphatase 6 |
| 39 | < 1e-07 | 18.4 17 | 101 | 36.57652 66 | 386.8485 273 | 0.09455 | 202437 s at | CYP1B1 | cytochrome P450, family 1, subfamily B, polypeptide 1 |
| 40 | < 1e-07 | 18.1 26 | 101 | 9.759971 4 | 26.98666 89 | 0.36165 9 | 218691 s at | PDLIM4 | PDZ and LIM domain 4 |
| 41 | < 1e-07 | 17.9 61 | 101 | 174.0420 711 | 796.1105 733 | 0.21861 54 | 206114 at | EPHA4 | EPH receptor A4 |
| 42 | < 1e-07 | 16.7 12 | 101 | 48.45020 117 | 398.1380 609 | 0.12169 2 | 214701 s at | FN1 | fibronectin 1 |
| 43 | < 1e-07 | 16.6 87 | 101 | 130.6533 226 | 513.9734 225 | 0.25420 25 | 211599 x at | MET | met proto-oncogene (hepatocyte growth factor receptor) |
| 44 | < 1e-07 | 16.6 51 | 101 | 43.70536 41 | 856.0270 201 | 0.05105 61 | 209810 at | SFTPB | surfactant protein B |
| 45 | < 1e-07 | 16.6 26 | 101 | 30.27352 26 | 659.4125 346 | 0.04590 98 | 37004 at | SFTPB | surfactant protein B |
| 46 | < 1e-07 | 16.4 82 | 101 | 39.41511 06 | 296.2635 734 | 0.13304 07 | 211668 s at | PLAU | plasminogen activator, urokinase |
| 47 | < 1e-07 | 16.3 | 101 | 45.64596 3 | 173.0041 102 | 0.26384 32 | 219743 at | HEY2 | hairy/enhancer-of-split related with YRPW motif 2 |
| 48 | < 1e-07 | 15.8 47 | 101 | 27.35831 89 | 271.5219 515 | 0.10075 91 | 219630 at | PDZK1I P1 | PDZK1 interacting protein 1 |
| 49 | < 1e-07 | 15.7 69 | 101 | 26.08259 28 | 145.2165 984 | 0.17961 16 | 212992 at | AHNAK 2 | HNAK nucleoprotein 2 |
| 50 | < 1e-07 | -15.7 14 | 101 | 1176.8944592 | 4846.7433882 | 0.2428217 | 201012 at | ANXA1 | annexin A1 |
| 51 | < 1e-07 | 15.6 55 | 101 | 98.240511 | 293.5830 722 | 0.33462 59 | 210605 s at | MFGE8 | milk fat globule-EGF factor 8 protein |
| 52 | < 1e-07 | 15.5 79 | 101 | 50.2016032 | 276.5722 365 | 0.18151 35 | 21335 at | TIAM1 | T-cell lymphoma invasion and metastasis 1 |
| 53 | < 1e-07 | 15.1 93 | 101 | 114.6878 901 | 381.0535 58 | 0.30097 58 | 33322 i at | SFN | stratifin |
| 54 | < 1e-07 | 15.1 59 | 101 | 14.18422 44 | 128.1764583 | 0.11066 17 | 219932 at | SLC27A 6 | solute carrier family 27 (fatty acid transporter), member 6 |
| 55 | < 1e-07 | 15.1 48 | 101 | 50.88611 13 | 253.2248 156 | 0.20095 23 | 33323 r at | SFN | stratifin |
| 56 | < 1e-07 | 14.9 48 | 101 | 599.6852 003 | 1663.810 0575 | 0.36042 89 | 212372 at | MYH10 | myosin, heavy chain 10, non-muscle |
| 57 | < 1e-07 | 14.8 63 | 101 | 32.44047 55 | 172.7300 0.18781 137 | 0.18781 03 | 204285 s at | PMAIP1 | phorbol-12-myristate 13-acetate-induced protein 1 |
| 58 | < 1e-07 | 14.7 7 | 101 | 65.83999 4 | 237.4471 324 | 0.27728 28 | 205425 at | HIP1 | huntingtin interacting protein 1 |
| 59 | < 1e-07 | 14.7 24 | 101 | 96.83739 05 | 357.5032 401 | 0.27087 14 | 213067 at | MYH10 | myosin, heavy chain 10, non-muscle |
| 60 | < 1e-07 | 14.6 11 | 101 | 212.9111 475 | 837.7639 24 | 0.25414 22 | 202458 at | PRSS23 | PRSS23 protease, serine, 23 protease, serine, 23 |
| 61 | < 1e-07 | 14.5 37 | 14.5 101 | 206.3462 018 | 716.2279 816 | 0.28810 13 | 201926 s at | CD55 | CD55 molecule, decay accelerating factor for complement (Cromer blood group) |
| 62 | < 1e-07 | 14.4 98 | 101 | 71.36620 93 | 391.9905 237 | 0.18206 11 | 207691 x at | ENTPD 1 | ectonucleoside triphosphate diphosphohydrolase 1 |
| 63 | < 1e-07 | 14.1 4 | 101 | 63.34410 44 | 309.5974 874 | 0.20460 15 | 211679 x at | GABBR 2 | gamma-aminobutyric acid (GABA) B receptor, 2 |
| 64 | < 1e-07 | 14.0 25 | 101 | 55.81752 21 | 292.3215 787 | 0.19094 56 | 209474 s at | ENTPD 1 | ectonucleoside triphosphate diphosphohydrolase 1 |
| 65 | < 1e-07 | 14.0 07 | 101 | 152.0773 555 | 1006.085 7206 | 0.15115 75 | 206595 at | CST6 | cystatin E/M |
| 66 | < 1e-07 | 14.0 04 | 101 | 65.9622745 | 321.3969 792 | 0.20523 61 | 203548 s at | LPL | lipoprotein lipase |
| 67 | < 1e-07 | 13.782 | 101 | 116.6241 763 | 716.3520 578 | 0.16280 29 | 203021 at | SLPI | secretory leukocyte peptidase inhibitor |
| 68 | < 1e-07 | 13.7 39 | 101 | 40.77680 82 | 215.5737 134 | 0.18915 48 | 217077 s at | GABBR 2 | gamma-aminobutyric acid (GABA) B receptor, 2 |
| 69 | < 1e-07 | 13.6 85 | 101 | 49.16165 41 | 174.8046 777 | 0.28123 76 | 202068 s at | I LDLR | low density lipoprotein receptor |
| 70 | < 1e-07 | 13.3 21 | 101 | 115.8136 532 | 255.7295 315 | 0.45287 56 | 203518 at | LYST | lysosomal trafficking regulator |
| 71 | < 1e-07 | 12.6 63 | 101 | 17.53485 79 | 128.3342 571 | 0.13663 43 | 205328 at | CLDN1 0 | claudin 10 |
| 72 | < 1e-07 | 12.4 77 | 101 | 31.94851 41 | 190.8936 054 | 0.16736 29 | 212531 at | LCN2 | lipocalin 2 |
| 73 | < 1e-07 | 12.4 68 | 101 | 35.89683 12 | 307.4829 307 | 0.11674 41 | 204446 s at | ALOX5 | arachidonate 5-lipoxygenase |
| 74 | < 1e-07 | 12.4 52 | 101 | 34.68107 99 | 112.9487 894 | 0.30705 14 | 206714 at | ALOX1 5B | arachidonate 15-lipoxygenase, type B |
| 75 | < 1e-07 | -12.2 77 | 101 | 56.07405 66 | 207.3878 358 | 0.27038 26 | 215506 s at | DIRAS3 | DIRAS family, GTP-binding RAS-like 3 |
| 76 | < 1e-07 | 11.9 82 | 101 | 12.49071 9 | 43.63344 57 | 0.28626 48 | 204286 s at | PMAIP1 | phorbol-12-myristate-13-acetate-induced protein 1 |
| 77 | < 1e-07 | -11.9 38 | 101 | 236.3052 255 | 1285.855 2347 | 0.18377 28 | 203413 at | NELL2 | NEL-like 2 (chicken) |
| 78 | < 1e-07 | 11.8 95 | 101 | 18.20478 97 | 37.14381 27 | 0.49011 63 | 204760 s at | NR1D1 | nuclear receptor subfamily 1, group D, member 1 |
| 79 | < 1e-07 | 11.8 18 | 101 | 15.86222 31 | 56.81549 41 | 0.27918 83 | 211343 s at | COL13 A1 | collagen, type XIII, alpha 1 |
| 80 | < 1e-07 | 11.8 13 | 101 | 96.63211 02 | 331.6200 556 | 0.29139 4 | 203549 s at | LPL | lipoprotein lipase |
| 81 | < 1e-07 | 11.7 95 | 101 | 10.30052 63 | 75.02892 65 | 0.13728 74 | 214702 at | FN1 | fibronectin 1 |
| 82 | < 1e-07 | 11.7 39 | 101 | 89.62413 55 | 409.3915 451 | 0.21892 03 | 213524 s at | G0S2 | G0/G1 switch 2 |
| 83 | < 1e-07 | 11.3 72 | 101 | 71.33577 44 | 164.3525 489 | 0.43404 12 | 221088 s at | PPP1R9 A | protein phosphatase 1, regulatory (inhibitor) subunit 9A |
| 84 | < 1e-07 | 11.3 46 | 101 | 60.61164 44 | 324.3370 43 | 0.18687 86 | 201645 at | TNC I | tenascin C |
| 85 | < 1e-07 | -11.2 56 | 101 | 62.0285944 | 274.5000306 | 0.2259694 | 219476 at | Clorf11 6 | chromosome 1 open reading frame 116 |
| 86 | < 1e-07 | 11.256 | 101 | 17.9476504 | 124.7535874 | 0.1438648 | 205402 x at | PRSS2 | protease, serine, 2 (trypsin 2) |
| 87 | < 1e-07 | 11.15 | 101 | 33.4101694 | 88.5149956 | 0.3774521 | 218883 s at | MLF1IP | MLF1 interacting protein |
| 88 | < 1e-07 | 11.039 | 101 | 261.2119262 | 513.2213961 | 0.5089654 | 218885 s at | GALNT 12 | UDP-N-acetyl-alpha-D-galactosamine:polypep tide N-acetylgalactosaminyltr ansferase 12 (Ga1NAc-T12) |
| 89 | < 1e-07 | -11.005 | 101 | 97.64726 17 | 492.1272192 | 0.1984187 | 219250 s at | FLRT3 | fibronectin leucine rich transmembrane protein 3 |
| 90 | < le-07 | 10.724 | 101 | 23.3226784 | 227.9166533 | 0.1023299 | 221266 s at | TM7SF4 | transmembrane 7 superfamily member 4 |
| 91 | < 1e-07 | 10.613 | 101 | 30.8151787 | 194.5419544 | 0.1583986 | 221577 x at | GDF15 | growth differentiation factor 15 |
| 92 | < 1e-07 | 10.281 | 101 | 15.6433426 | 72.5020552 | 0.2157641 | 216470 x at | PRSS1 | protease, serine, 1 (trypsin 1) |
| 93 | < 1e-07 | 10.158 | 101 | 67.4827158 | 475.2400251 | 0.1419971 | 209365 s at | ECM1 | extracellular matrix protein 1 |
| 94 | < 1e-07 | 9.779 | 101 | 60.3665284 | 138.1280462 | 0.4370331 | 219654 at | PTPLA | protein tyrosine phosphatase-like (proline instead of catalytic arginine), member A |
| 95 | < 1e-07 | 9.72 2 | 101 | 43.81736 36 | 205.8857248 | 0.2128237 | 209774 x at | CXCL2 | chemokine (C-X-C motif) ligand 2 |
| 96 | < 1e-07 | 9.564 | 101 | 11.505946 | 36.8559336 | 0.3121871 | 203757 s at | CEACA M6 | carcinoembryonic antigen-related cell adhesion molecule 6 (non-specific cross reacting antigen) |
| 97 | < 1e-07 | 9.188 | 101 | 15.0320438 | 34.8270767 | 0.4316195 | 213582 at | ATP11A | ATPase, class VI, type 11A |
| 98 | < 1e-07 | 9.012 | 101 | 18.314415 | 57.8091112 | 0.3168085 | 201291 s at | TOP2A | topoisomerase (DNA) II alpha 170kDa |
| 99 | < 1e-07 | 8.957 | 101 | 176.5563142 | 612.8754003 | 0.2880786 | 212998 x at | HLA-DQB1 | major histocompatibility complex, class II, DQ beta 1 |
| 100 | < 1e-07 | -8.66 | 101 | 67.5367103 | 587.7871482 | 0.114999 | 218002 s at | CXCL1 4 | chemokine (C-X-C motif) ligand 14 |
| 101 | < 1e-07 | 8.55 3 | 101 | 42.3211714 | 181.2673119 | 0.2334738 | 219230 at | TMEM1 00 | transmembrane protein 100 |
| 102 | < 1e-07 | 8.27 9 | 101 | 135.4036752 | 237.5129828 | 0.5700896 | 58916 at | KCTD1 4 | potassium channel tetramerisation domain' containing 14 |
| 103 | < 1e-07 | 8.27 5 | 101 | 34.2141944 | 106.5749822 | 0.321034 | 210617 at | PHEX | phosphate regulating ndopeptidase homolog, X-linked |
| 104 | < 1 e-07 | 8.26 1 | 101 | 16.7396797 | 90.2950505 | 0.1853887 | 216191 s at | TRD@ | T cell receptor delta locus |
| 105 | < 1e-07 | 7.89 4 | 101 | 67.84349 | 129.5598578 | 0.5236459 | 204976 s at | AMME CR1 | Alport syndrome, mental retardation, idface hypoplasia and elliptocytosis chromosomal region gene 1 |
| 106 | < 1e-07 | 7.74 8 | 101 | 29.8873184 | 69.0042105 | 0.4331231 | 218039 at | NUSAP 1 | nucleolar and spindle associated protein 1 |
| 107 | < 1e-07 | 7.62 2 | 101 | 151.6514399 | 373.0337836 | 0.4065354 | 220994 s at | STXBP6 | syntaxin binding protein 6 (amisyn) |
| 108 | < 1e-07 | 7.28 3 | 101 | 735.9330604 | 1368.2583881 | 0.5378612 | 213798 s at | CAP1 | CAP, adenylate cyclase-associated protein 1 (yeast) |
| 109 | < 1e-07 | 6.97 7 | 101 | 50.9387725 | 146.8844136 | 0.3467949 | 216945 x at | PASK | PAS domain containing serine/threonine kinase |
| 110 | < 1e-07 | 6.93 8 | 101 | 18.0418695 | 62.5201809 | 0.2885767 | 220595 at | PDZRN 4 | PDZ domain containing ring finger 4 |
| 111 | < 1e-07 | 6.89 3 | 101 | 1057.6158084 | 1743.6261467 | 0.6065611 | 221530 s at | BHLHE 41 | basic helix-loop-helix family, member e41 |
| 112 | < 1e-07 | 6.70 7 | 101 | 27.1048088 | 48.738474 | 0.5561282 | 220777 at | KIF13A | kinesin family member 13A |
| 113 | < 1e-07 | 6.53 6 | 101 | 12.5137837 | 24.7263095 | 0.5060918 | 205869 at | PRSS1 | protease, serine, 1 (trypsin 1) |
| 114 | < 1e-07 | 6.48 6 | 101 | 80.8444542 | 261.7961331 | 0.3088069 | 203963 at | CA12 | carbonic anhydrase XII |
| 115 | < 1e-07 | 6.34 | 101 | 123.812618 | 277.7681701 | 0.4457411 | 203881 s at | DMD | dystrophin |
| 116 | < 1e-07 | 5.78 3 | 101 | 36.1673343 | 68.67259 22 | 0.5266633 | 205794 s at | NOVA1 | neuro-oncological ventral antigen 1 |
| 117 | 1e-07 | - 5.594 | 101 | 55.069615 | 90.1676182 | 0.6107471 | 213298 at | NFIC | nuclear factor I/C (CCAAT-binding transcription factor) |
| 118 | 1e-07 | 5.53 1 | 101 | 77.27051 69 | 147.9201 714 | 0.52237 98 | 208498 s at | AMY1A | amylase, alpha 1A (salivary) |
| 119 | 3e-07 | 5.41 7 | 101 | 184.8978 478 | 264.8005 852 | 0.69825 32 | 218728 s at | CNIH4 | cornichon homolog 4 (Drosophila) |
| 120 | 1.5e-06 | -5.02 8 | 101 | 16.86204 61 | 27.26730 09 | 0.61839 81 | 207165 at | HMMR | hyaluronan-mediated motility receptor (RHAMM) |
| 121 | 1.7e-06 | 4.99 3 | 101 | 33.01245 34 | 45.02369 34 | 1 0.73322 4 | 219518 s at | ELL3 | elongation factor RNA polymerase II-like 3 |
| 122 | 7e-06 | 4.66 4 | 101 | 69.31853 3 | 149.1634 223 | 0.46471 54 | 219759 at | ERAP2 | endoplasmic reticulum aminopeptidase 2 |
| 123 | 1.7e-05 | 4.45 3 | 101 | 77.21862 33 | 120.9265 741 | 0.63855 79 | 213931 at | ID2 | inhibitor of DNA binding 2, dominant negative helix-loop-helix protein |
| 124 | 2.25e-05 | 4.38 3 | 101 | 60.12817 37 | 117.1083 386 | 0.51344 06 | 210096 at | CYP4B1 | cytochrome P450, family 4, subfamily B, polypeptide 1 |
| 125 | 9.92e-05 | 4.00 5 | 101 | 83.22550 6 | 168.0445 571 | 0.49525 86 | 216834 at | RGS1 | regulator of G-protein signaling 1 |
| 126 | 0.0003 04 | 3.70 3 | 101 | 17.05463 89 | 22.36583 94 | 0.76253 07 | 216173 at | NA | NA |
| 127 | 0.0008 065 | 3.42 4 | 101 | 112.9811 642 | 170.0393 695 | 0.66444 12 | 221558 s at | LEF1 | lymphoid enhancer-binding factor 1 |
| 128 | 0.0011 237 | 3.32 5 | 101 | 31.96350 8 | 40.12765 6 | 0.79654 56 | 209708 at | MOXD1 | monooxygenase, DBH-like 1 |
| 129 | 0.0035 278 | 2.96 7 | 101 | 24.67247 87 | 34.29526 49 | 0.71941 36 | 207078 at | MED6 | mediator complex subunit 6 |
| 130 | 0.0041 761 | 2.91 1 | 101 | 31.74136 57 | 43.67997 14 | 0.72668 01 | 211466 at | NFIB | nuclear factor I/B |
| 131 | 0.0068 704 | -2.74 3 | 101 | 1401.901 4512 | 1948.856 9295 | 0.71934 55 | 211959 at | IGFBP5 | insulin-like growth factor binding protein 5 |
| 132 | 0.0231 219 | 2.29 6 | 101 | 11.26507 2 | 17.35374 34 - | 0.64914 36 | 204409 s at | EIF1AY | eukaryotic translation initiation factor 1A, Y-linked |
| 133 | 0.0239 891 | 2.28 2 | 101 | 206.6963 718 | 261.0079 664 | 0.79191 6 | 213350 at | RPS11 | ibosomal protein S11 |
| 134 | 0.0288 723 | 2.20 8 | 101 | 13.07750 47 | 22.31338 51 | 0.58608 34 | 205000 at | DDX3Y | DEAD (Asp-Glu-Ala-Asp) box polypeptide 3, Y-linked |
| 135 | 0.0362877 | 2.114 | 101 | 14.8367962 | 20.7316424 | 0.7156595 | 210163 at | CXCL1 1 | chemokine (C-X-C motif) ligand 11 |
| 136 | 0.0603953 | 1.893 | 101 | 68.7318885 | 81.5450277 | 0.8428704 | 200952 s at | CCND2 | cyclin D2 |
| 137 | 0.0746917 | 1.795 | 101 | 20.3636333 | 25.4058266 | 0.801534 | 214636 at | CALCB | calcitonin-related polypeptide beta |
| 138 | 0.0824881 | 1.74 9 | 101 | 163.1541999 | 183.8311581 | 0.88722 | 213239 at | PIBF1 | progesterone immunomodulatory binding factor 1 |
| 139 | 0.0837148 | 1.74 2 | 101 | 57.2181135 | 87.2434548 | 0.6558442 | 203290 at | HLA-DQA1 | major histocompatibility complex, class II, DQ alpha 1 |
| 140 | 0.2082626 | 1.26 4 | 101 | 125.2148221 | 148.9141984 | 0.8408521 | 203896 s at | PLCB4 | phospholipase C, beta 4 |
| 141 | 0.7426847 | 0.32 9 | 101 | 38.4507136 | 39.5707496 | 0.9716954 | 204861 s at | NAIP | NLR family, apoptosis inhibitory protein |
| 142 | 0.8375717 | 0.205 | 101 | 226.220355 | 232.5041255 | 0.9729733 | 203895 at | PLCB4 | phospholipase C, beta 4 |
| 143 | 0.8648788 | 0.17 | 101 | 2022.2830303 | 1979.9008045 | 1.0214062 | 211600 at | PTPRO | protein tyrosine phosphatase, receptor type, O |
| 144 | 0.8191293 | 0.229 | 101 | 542.429671 | 511 | 1.0361568 | AFFX-r2-Hs18SrRNA-3 s at | NA | NA |
| 145 | 0.6027442 | 0.522 | 101 | 59.110964 | 56.6213802 | 1.043969 | 213813 x at | NA | NA |
| 146 | 0.5104704 | 0.66 | 101 | 356.8412356 | 317.2716965 | 1.1247182 | 202672 s at | ATF3 | activating transcription factor 3 |
| 147 | 0.45538 | 0.749 | 101 | 113.2368 719 | 106.9125 58 | 1.05915 41 | 212044 s at | RPL27A | ribosomal protein L27a |
| 148 | 0.3012495 | 1.038 | 101 | 31.4334948 | 27.4308035 | 1.1459196 | 210728 s at | CALCA | calcitonin-related polypeptide alpha |
| 149 | 0.2775125 | 1.09 | 101 | 106.117816 | 97.5478056 | 1.0878545 | 216246 at | NA | NA |
| 150 | 0.2624131 | 1.125 | 101 | 175.4658129 | 133.0119917 | 1.3191729 | 214218 s at | XIST | X (inactive)-specific transcript (non-protein coding) |
| 151 | 0.1969525 | 1.296 | 101 | 1818.5842489 | 1670.657163 | 1.0885443 | AFFX-r2-Ec-bioD-5 at | NA | NA |
| 152 | 0.0723707 | 1.81 | 101 | 193.7594741 | 154.8483812 | 1.2512851 | AFFX-HUMRGE/M1009 8 3 at | NA | NA |
| 153 | 0.0561596 | 1.92 5 | 101 | 530.044356 | 405.1500548 | 1.3082668 | 209459 s at | ABAT | 4-aminobutyrate aminotransferase |
| 154 | 0.0537204 | 1.945 | 101 | 319.228665 | 278.6021455 | 1.145225 | 221943 x at | RPL38 | ribosomal protein L38 |
| 155 | 0.0356045 | 2.121 | 101 | 270.3802878 | 200.3217176 | 1.3497303 | AFFX-r2-Hs18SrRNA-M x at | NA | NA |
| 156 | 0.0221325 | 2.313 | 101 | 24.5493918 | 17.8731871 | 1.3735318 | 210727 at | CALCA | calcitonin-related polypeptide alpha |
| 157 | 0.0095427 | 2.627 | 101 | 93.5557129 | 69.2743802 | 1.3505096 | AFFX-HUMRGE/M1009 8 M at | NA | NA |
| 158 | 0.0060321 | 2.788 | 101 | 203.0783052 | 136.2169552 | 1.4908445 | AFFX-HUMRGE/M1009 8 5 at | NA | NA |
| 159 | 0.0051686 | 2.84 | 101 | 3334.1956671 | 2336.9724359 | 1.4267159 | AFFX-HSAC07/X00351 5 at | ACTB | actin, beta |
| 160 | 0.0005514 | 3.534 | 101 | 697.0820123 | 533.4789478 | 1.306672 | 212952 at | CALR | calreticulin |
| 161 | 0.0002 955 | 3.71 | 101 | 63.67867 74 | 30.765947 | 2.0697779 | 205048 s at | PSPH | phosphoserine phosphatase |
| 162 | 0.0001815 | 3.844 | 101 | 90.9188584 | 48.297446 | 1.8824776 | AFFX-r2-Hs18SrRNA-5 at | NA | NA |
| 163 | 2.76e-05 | 4.33 3 | 101 | 659.7400101 | 483.5009942 | 1.364506 | 200700 s at | KDELR 2 | KDEL (Lys-Asp-Glu-Leu) endoplasmic reticulum protein retention receptor 2 |
| 164 | 5.7e-06 | 4.71 4 | 101 | 584.0274426 | 417.1941165 | 1.3998938 | 200762 at | DPYSL 2 | dihydropyrimidinase-like 2 |
| 165 | 1.1e-06 | 5.084 | 101 | 90.1734458 | 59.7969499 | 1.5079941 | 204597 x at | STC1 | stanniocalcin 1 |
| 166 | 3e-07 | 5.349 | 101 | 3672.8313231 | 2571.2647693 | 1.4284143 | 213702 x at | ASAH1 | N-acylsphingosine amidohydrolase (acid ceramidase) 1 |
| 167 | 1e-07 | 5.545 | 101 | 282.8859917 | 140.7167228 | 2.0103225 | AFFX-M27830 5 at | NA | NA |
| 168 | < 1e-07 | 6.421 | 101 | 239.3587519 | 144.4173217 | 1.657403 | 203164 at | SLC33A 1 | solute carrier family 33 (acetyl-CoA transporter), member 1 |
| 169 | < 1e-07 | 6.58 4 | 101 | 169.2251 251 | 120.9506 745 | 1.39912 51 | 208730 x at | RAB2A | RAB2A, member RAS oncogene family |
| 170 | < 1e-07 | 6.84 9 | 101 | 309.3095 778 | 171.3274 49 | 1.80537 08 | 204417 at | GALC | galactosylceramidase |
| 171 | < 1e-07 | 6.894 | 101 | 417.5529513 | 169.4613561 | 2.464001 | 201998 at | ST6GA L1 | ST6 beta-galactosamide alpha-2,6-sialyltranferase 1 |
| 172 | < 1e-07 | 7.443 | 101 | 407.8057983 | 237.4327287 | 1.7175635 | 208846 s at | VDAC3 | voltage-dependent anion channel 3 |
| 173 | < 1e-07 | 7.516 | 101 | 92.1133561 | 45.4891319 | 2.024953 | 200879 s at | EPAS1 | endothelial PAS domain protein 1 |
| 174 | < 1e-07 | 7.728 | 101 | 815.1942195 | 399.6068572 | 2.0399906 | 204427 s at | TMED2 | transmembrane emp24 domain trafficking protein 2 |
| 175 | < 1e-07 | 7.788 | 101 | 154.090147 | 79.31036 | 1.9428753 | 203708 at | PDE4B | phosphodiesterase 48, cAMP-specific (phosphodiesterase E4 dunce homolog, Drosophila |
| 176 | < 1e-07 | 7.803 | 101 | 100.5671531 | 49.6316147 | 2.026272 | 205554 s at | DNASE 1L3 | deoxyribonuclease I-like 3 |
| 177 | < 1e-07 | 7.884 | 101 | 56.0453603 | 29.6202625 | 1.8921291 | 211776 s at | EPB41L 3 | rythrocyte membrane protein band 4.1-like 3 |
| 178 | < 1e-07 | 8.017 | 101 | 282.9463265 | 130.1370117 | 2.1742187 | 205321 at | EIF2S3 | eukaryotic translation initiation factor 2, subunit 3 gamma, 52kDa |
| 179 | < 1e-07 | 8.141 | 101 | 373.3021562 | 132.3257497 | 2.8210848 | 205382 s at | CFD | complement factor D (adipsin) |
| 180 | < 1e-07 | 8.201 | 101 | 56.77502 83 | 21.70792 62 | 2.61540 54 | 214971 s at | ST6GA L1 | ST6 beta-galactosamide alpha-2,6-sialyltranferase 1 |
| 181 | < 1e-07 | 8.25 2 | 101 | 250.0427 144 | 147.5208 441 | 1.69496 53 | 200890 s at | SSR1 | signal sequence receptor, alpha |
| 182 | < 1e-07 | 8.64 1 | 101 | 108.5673 242 | 45.34628 63 | 2.39418 34 | 219410 at | TMEM4 5A | transmembrane protein 45A |
| 183 | < 1e-07 | 8.82 4 | 101 | 239.7956 306 | 114.5076 367 | 2.09414 53 | 205392 s at | CCL14 | chemokine (C-C motif) ligand 14 |
| 184 | < 1e-07 | 9.08 1 | 101 | 31.52244 81 | 11.12599 52 | 2.83322 5 | 213920 at | CUX2 | cut-like homeobox 2 |
| 185 | < 1e-07 | 9.31 9 | 101 | 6198.650 6043 | 2859.951 6098 | 2.16739 7 | 214091 s at | GPX3 | glutathione peroxidase 3 (plasma) |
| 186 | < 1e-07 | 9.53 6 | 101 | 84.91175 . 13 | 43.95521 7 | 1.93177 87 | 211302 s at | PDE4B | phosphodiesterase 4B, cAMP-specific (phosphodiesterase E4 dunce homolog, Drosophila) |
| 187 | < 1e-07 | 10.0 54 | 101 | 391.1767 807 | 95.56099 73 | 4.09347 74 | 201843 s at | EFEMP 1 | EGF-containing fibulin-like extracellular matrix protein 1 |
| 188 | < 1e-07 | 10.1 44 | 101 | 339.4659 545 | 83.64747 14 | 4.05829 31 | 210078 s at | KCNAB 1 | potassium voltage-gated channel, shaker-related subfamily, beta member 1 |
| 189 | < 1e-07 | 10.1 56 | 101 | 39.75619 23 | 11.67873 8 | 3.40415 14 | 215350 at | SYNE1 | spectrin repeat containing, nuclear envelope 1 |
| 190 | < 1e-07 | 10.7 66 | 101 | 404.2338 031 | 95.63305 86 | 4.22692 54 | 210471 s at | KCNAB 1 | potassium voltage-gated channel, shaker-related subfamily, beta member 1 |
| 191 | < 1e-07 | 11.8 61 | 101 | 113.920 6771 | 452.1358 327 | 2.46368 59 | 203962 s at | NEBL | nebulette |
| 192 | < 1e-07 | 12.1 07 | 101 | 1080.933 1064 | 228.3732 71 | 4.73318 57 | 212224 at | ALDH1 A1 | aldehyde dehydrogenase 1 family, member A1 |
| 193 | < 1e-07 | 12.376 | 101 | 3788.7943402 | 938.9144826 | 4.0352923 | 206461 x at | MT1H | metallothionein 1H |
| 194 | < 1e-07 | 13.4 55 | 101 | 116.5595 258 | 31.94321 23 | 3.64896 07 | 203178 at | GATM | glycine amidinotransferase (L-arginine:glycine amidinotransferase) |
| 195 | < 1e-07 | 14.2 43 | 101 | 3745.449 2268 | 255.6254 232 | 14.6520 999 | 210342 s at | TPO | thyroid peroxidase |
| 196 | < 1e-07 | 14.6 02 | 101 | 168.6378 588 | 62.13190 99 | 2.71419 08 | 200671 s at | SPTBN1 | spectrin, beta, non-erythrocytic 1 |
| 197 | < 1e-07 | 15.1 44 | 101 | 4592.834 8815 | 863.0348 604 | 5.32172 58 | 204745 x at | MT1G | metallothionein 1G |
| 198 | < 1e-07 | 16.0 81 | 101 | 1153.375 2117 | 411.9641 466 | 2.79969 8 | 217853 at | TNS3 | tensin 3 |
| 199 | < 1e-07 | 16.1 12 | 101 | 465.0215 545 | 158.4792 311 | 2.93427 44 | 200672 x at | SPTBN1 | spectrin, beta, non-erythrocytic 1 |
| 200 | < 1e-07 | 19.1 62 | 101 | 314.9982 571 | 57.07155 75 | 5.51935 62 | 204513 s at | ELMO1 | engulfment and cell motility 1 |

The prediction rule was defined by the inner sum of the weights (*wᵢ*) and expression (*xᵢ*) of significant genes. A sample is classified to the class normal/benign if the sum is greater than the threshold, that is the Σ*ᵢwᵢ xᵢ* > threshold. The threshold for the Support Vector Machine predictor is -4.016. In Table 8 we summarized the weights for all genes.

**Table 8.**

| Gene Weights | Genes | Support Vector Machines |
|---|---|---|
| 1 | 209396_s_at -0.0168 | -0.0168 |
| 2 | 209395_at | -0.0164 |
| 3 | AFFX-r2-Hs18SrRNA-5_at | 0.0163 |
| 4 | 202286_s_at | -0.0154 |
| 5 | 202436_s_at | -0.0149 |
| 6 | 06884_s_at | -0.0138 |
| 7 | 205728_at | -0.0135 |
| 8 | 202435_s_at | -0.0133 |
| 9 | 207144_s-_at | -0.0131 |
| 10 | 205048_s_at | 0.0122 |
| 11 | 220332_at | -0.0123 |
| 12 | 208025_s_at | -0.0121 |
| 13 | 37004_at | -0.0116 |
| 14 | AFFX-HUMRGE/M10098_5_at | 0.0118 |
| 15 | 212464_s_at | -0.0113 |
| 16 | 211478_s_at | -0.0112 |
| 17 | AFFX-HUMRGE/M10098_3_at ) | 0.0111 |
| 18 | 210495_x_at | -0.0109 |
| 19 | 216442_x_at | -0.0109 |
| 20 | AFFX-M27830_5_at | 0.0107 |
| 21 | 202437_s_at | -0.0106 |
| 22 | AFFX-HUMRGE/M10098_M_at | 0.0106 |
| 23 | 221266_s_at | -0.0105 |
| 24 | 203355_s_at | -0.0103 |
| 25 | 209810_at | -0.0097 |
| 26 | AFFX-r2-Hs18SrRNA-M_x_at | 0.0098 |
| 27 | 203717_at | -0.0095 |
| 28 | 212531_at | -0.0094 |
| 29 | 206595_at | -0.0092 |
| 30 | 213524_s_at | -0.0091 |
| 31 | 211719_x_at | -0.0091 |
| 32 | AFFX-r2-Hs18SrRNA-3_s_at | 0.0091 |
| 33 | 202672_s_at | -0.0089 |
| 34 | 220595_at | -0.0088 |
| 35 | 205000_at | -0.0088 |
| 36 | 205402_x_at | -0.0086 |
| 37 | 203290_at | 0.0088 |
| 38 | 203510_at | -0.0086 |
| 39 | 213920_at | 0.0084 |
| 40 | 203548_s_at | -0.0085 |
| 41 | 208893_s_at | -0.0082 |
| 42 | 205321_at | 0.0083 |
| 43 | 210728_s_at | -0.0084 |
| 44 | 206461_x_at | 0.0081 |
| 45 | 208892_s_at | -0.0082 |
| 46 | 210617_at | -0.0081 |
| 47 | 209270_at | -0.008 |
| 48 | 204669_s_at | -0.0081 |
| 49 | 21393_at | -0.0079 |
| 50 | 209774_x_at | -0.0078 |
| 51 | 204513_s_at | 0.0077 |
| 52 | 203354_s_at | -0.0079 |
| 53 | 216191_s_at | -0.0077 |
| 54 | 218002_s_at | -0.0075 |
| 55 | 202068_s_at | -0.0076 |
| 56 | 211564_s_at | -0.0077 |
| 57 | 210727_at | -0.0076 |
| 58 | 221577_x_at | -0.0077 |
| 59 | 205479_s_at | -0.0076 |
| 60 | 213067_at | -0.0076 |
| 61 | 207808_s_at | -0.0074 |
| 62 | 205174_s_at | -0.0072 |
| 63 | 200700_s_at | 0.0071 |
| 64 | 216945_x_at | -0.0072 |
| 65 | 214702_at | -0.0071 |
| 66 | AFFX-HSAC07/X00351_5_at | 0.0071 |
| 67 | 214175_x_at | -0.0071 |
| 68 | 219410_at | 0.007 |
| 69 | 208730_x_at | 0.007 |
| 70 | 213135_at | -0.007 |
| 71 | 213350_at | 0.007 |
| 72 | 215506_s_at | -0.0069 |
| 73 | 213702_x_at | 0.0069 |
| 74 | 219230_at | -0.0068 |
| 75 | 208891_at | -0.0069 |
| 76 | 212952_at | 0.0069 |
| 77 | 212224_at | 0.0069 |
| 78 | 219932_at | -0.0068 |
| 79 | 219250_s_at | -0.0067 |
| 80 | 211343_s_at | -0.0066 |
| 81 | 219630_at | -0.0066 |
| 82 | 201291_s_at | -0.0067 |
| 83 | 205794_s_at | -0.0066 |
| 84 | 204417_at | 0.0066 |
| 85 | 203716_s_at | -0.0067 |
| 86 | 217853_at | 0.0067 |
| 87 | 211679_x_at | 0.0067 |
| 88 | 209459_s_at | 0.0066 |
| 89 | 203896_s_at | -0.0067 |
| 90 | 204285_s_at | -0.0066 |
| 91 | 214091_s_at | 0.0065 |
| 92 | 207691_x_at | -0.0065 |
| 93 | 204427_s_at | 0_{.}0065 |
| 94 | 209474_s_at | -0.0065 |
| 95 | 220994_s_at | -0.0065 |
| 96 | AFFX-r2-Ec-bioD-5-_at | 0.0065 |
| 97 | 221530_s_at | -0.0065 |
| 98 | 211302_s_at | 0.0065 |
| 99 | 211959_at | 0.0065 |
| 100 | 203549_s_at | -0.0065 |
| 101 | 200672_x_at | 0.0064 |
| 102 | 216173_at | -0.0064 |
| 103 | 213298_at | -0.0064 |
| 104 | 214636_at | -0.0063 |
| 105 | 33322_i_at | -0.0063 |
| 106 | 206114_at | -0.0063 |
| 107 | 205485_at | -0.0064 |
| 108 | 203881_s_at | -0.0063 |
| 109 | 219518_s_at | -0.0063 |
| 110 | 203021_at | -0.0063 |
| 111 | 212992_at | -0.0063 |
| 112 | 214971_s_at | 0.0062 |
| 113 | 218039_at | -0.0063 |
| 114 | 201645_at | -0.0062 |
| 115 | 200762_at | 0.0062 |
| 116 | 33323_r_at | -0.0062 |
| 117 | 200671_s_at | 0.0061 |
| 118 | 216246_at | 0.0061 |
| 119 | 204286_s_at | -0.0061 |
| 120 | 207078_at | -0.0061 |
| 121 | 206714_at | -0.0061 |
| 122 | 205392_s_at | 0.006 |
| 123 | 212372_at | -0.0061 |
| 124 | 211668_s_at | -0.0062 |
| 125 | 203708_at | 0.0061 |
| 126 | 218691_s_at | -0.0061 |
| 127 | 218883_s_at | -0.0061 |
| 128 | 200879_s_at | 0.006 |
| 129 | 216470_x_at | -0.0059 |
| 130 | 204745_x_at | 0.0059 |
| 131 | 212850_s_at | -0.006 |
| 132 | 217077_s_at | -0.0061 |
| 133 | 220777_at | -0.0059 |
| 134 | 201012_at | -0.0058 |
| 135 | 209708_at | -0.0058 |
| 136 | 219759_at | -0.0058 |
| 137 | 211599_x_at | -0.0057 |
| 138 | 211600_at | 0.0057 |
| 139 | 204597_x_at | 0.0057 |
| 140 | 58916_at | -0.0059 |
| 141 | 213582_at | -0.0057 |
| 142 | 218885_s_at | -0.0057 |
| 143 | 204446_s_at | -0.0056 |
| 144 | 210471_s_at | 0.0058 |
| 145 | 221840_at | -0.0057 |
| 146 | 211466_at | -0.0058 |
| 147 | 10342_s_at | 0.0056 |
| 148 | 203413_at | -0.0056 |
| 149 | 203962_s_at | 0.0056 |
| 150 | 205382_s_at | 0.0056 |
| 151 | 2186163_at | -0.0057 |
| 152 | 200890_s_at | 0.0056 |
| 153 | 208498_s_at | -0.0056 |
| 154 | 216834_at | -0.0054 |
| 155 | 204409_s_at | -0.0055 |
| 156 | 200952_s_at | 0.0055 |
| 157 | 203757_s_at | -0.0054 |
| 158 | 215350_at | 0.0054 |
| 159 | 202458_at | -0.0056 |
| 160 | 205869_at | -0.0054 |
| 161 | 209365_s_at | -0.0056 |
| 162 | 203895_at | -0.0057 |
| 163 | 206765_at | -0.0056 |
| 164 | 205328_at | -0.0055 |
| 165 | 212044_s_at | 0.0055 |
| 166 | 203963_at | -0.0056 |
| 167 | 205016_at | 0.0056 |
| 168 | 221088_s_at | -0.0056 |
| 169 | 221558_s_at | -0.0055 |
| 170 | 204014_at | -0.0056 |
| 171 | 205554_s_at | 0.0055 |
| 172 | 201926_s_at | -0.0055 |
| 173 | 208846_s_at | 0.0055 |
| 174 | 210163_at | -0.0053 |
| 175 | 214218_s_at | 0.0054 |
| 176 | 218728_s_at | -0.0055 |
| 177 | 201843_s_at | 0.0055 |
| 178 | 219654_at | -0.0055 |
| 179 | 219476_at | 0.0054 |
| 180 | 204976_s_at | -0.0055 |
| 181 | 212611_at | -0.0054 |
| 182 | 205425_at | -0.0055 |
| 183 | 221943_x_at | 0.0054 |
| 184 | 213798_s_at | -0.0054 |
| 185 | 210096_at | -0.0054 |
| 186 | 211776_s_at | 0.0054 |
| 187 | 204760_s_at | -0.0053 |
| 188 | 203178_at | 0.0053 |
| 189 | 210605_s_at | -0.0054 |
| 190 | 201998_at | 0.0053 |
| 191 | 204861_s_at | -0.0052 |
| 192 | 210078_s_at | 0.0053 |
| 193 | 214701_s_at | -0.0053 |
| 194 | 207165_at | -0.0053 |
| 195 | 203164_at | 0.0053 |
| 196 | 212998_x_at | -0.0051 |
| 197 | 213813_x_at | 0.0051 |
| 198 | 219743_at | -0.0053 |
| 199 | 203518_at | -0.005 |
| 200 | 213239_at | -0.0052 |

We obtained 49 genes, which are common to both signatures produced using older and newer generation of microarrays.

**Table 9.**

| | Parametric p-value | FDR | Geom mean of intensities in class 1 | Geom mean of intensities in class 2 | Fold-change | Probe set | Annotations | Gene symbol | Description | Defined Genelist |
|---|---|---|---|---|---|---|---|---|---|---|
| 1 | < 1e-07 | < 1e-07 | 1207.4997386 | 3932.9914083 | 0.3070181 | 201012 at | Info | ANXA 1 | annexin A1 | Corticosteroids and cardioprotection |
| 2 | < 1e-07 | < 1e-07 | 211.7976656 | 1007.3933142 | 0.2102433 | 203413 at | Info | NELL2 | NEL-like 2 (chicken) | |
| 3 | < 1e-07 | < 1e-07 | 233.3335553 | 1352.1461766 | 0.1725653 | 203510 at | Info | MET | met proto-oncogene (hepatocyte growth factor receptor) | CBL mediated ligand-induced downregulati on of EGF receptors, Signaling of Hepatocyte Growth Factor Receptor, Adherens junction, Axon guidance, Colorectal cancer, Cytokine-cytokine receptor interaction, Epithelial cell signaling in Helicobacter pylori infection, Focal adhesion, angiogenesis , tsonc |
| 4 | < 1e-07 | < 1e-07 | 29.1467739 | 182.9049547 | 0.1593548 | 203716 s at | | DPP4 | dipeptidyl-peptidase 4 | angiogenesis immunology, metastasis |
| 5 | < 1e-07 | < 1e-07 | 30.65453 42 | 246.5514 083 | 0.12433 32 | 203717 at | Info | DPP4 | dipeptidyl-peptidase 4 | angiogenesis' immunology, metastasis |
| 6 | < 1e-07 | < 1e-07 | 16.4967182 | 38.5807293 | 0.4275896 | 203757 s at | Info | CEAC AM6 | carcinoembry onic antigen related cell adhesion molecule 6 (non-specific cross reacting antigen) | |
| 7 | < 1e-07 | < 1e-07 | 53.0974773 | 247.8200253 | 0.21425 82 | 205016 at | Info | TGFA | transforming growth factor, alpha | |
| 8 | < 1e-07 | < 1e-07 | 42.5436362 | 242.854265 | 0.17515 21 | 205174 s at | Info | QPCT | glutaminyl-peptide cyclotransfer ase | |
| 9 | < 1e-07 | < 1e-07 | 23.13199 | 100.6199832 | 0.2299066 | 205485 at | Info | RYR1 | ryanodine receptor 1 (skeletal) | Calcium signaling pathway, Long-term depression, immunology |
| 1 0 | < 1e-07 | < 1e-07 | 144.0317304 | 789.2549196 | 0.18249 08 | 206595 at | Info | CST6 | cystatin E/M | |
| 1 1 | < 1e-07 | < 1e-07 | 44.9053145 | 327.8890345 | 0.13695 28 | 206765 at | Info | KCNJ2 | potassium inwardly-rectifying channel, subfamily J, member 2 | |
| 1 2 | < 1e-07 | < 1e-07 | 84.7811374 | 892.1276994 | 0.0950325 | 207144 s at | Info | CITED 1 | Cbp/p300-interacting transactivator ,with Glu/Asp-rich carboxy-terminal domain, 1 | |
| 1 3 | <1e-07 | <1e-07 | 158.1719537 | 910.2062231 | 0.173776 | 207808 s at | Info | PROS1 | protein S (alpha) | Acute Myocardial Infarction, Extrinsic Prothrombin Activation Pathway, Intrinsic Prothrombin Activation Pathway, Complement and coagulation cascades, immunology |
| 1 4 | < 1e-07 | < 1e-07 | 22.299442 | 268.6350138 | 0.0830102 | 208025 s at | Info | HMGA 2 | high mobility group AT-hook 2 | |
| 1 5 | < 1e-07 | < 1e-07 | 35.56798 | 208.0828 369 | 0.17093 18 | 209270 at | Info | LAMB 3 | laminin, beta 3 | Cell Communicat ion, ECM-receptor interaction, Focal adhesion |
| 1 6 | < 1e-07 | < 1e-07 | 93.8565608 | 409.3782964 | 0.2292661 | 209365 s at | Info | ECM1 | extracellular matrix protein 1 | |
| 1 7 | < 1e-07 | < 1e-07 | 39.7815771 | 790.6956874 | 0.0503121 | 209395 at | Info | CHI3L 1 | chitinase 3-like 1 (cartilage glycoprotein-39) | |
| 1 8 | < 1e-07 | < 1e-07 | 44.3338181 | 718.4503458 | 0.0617076 | 209396 s at | Info | CHI3L 1 | chitinase 3-like 1 (cartilage glycoprotein-39) | |
| 1 9 | < 1e-07 | < 1e-07 | 51.8060982 | 213.740473 | 0.2423785 | 209474 s at | Info | ENTPD 1 | ectonucleosid e triphosphate diphosphohy drolase 1 | |
| 2 0 | < 1e-07 | < 1e-07 | 64.7621322 | 753.1097576 | 0.085993 | 209810 at | Info | SFTPB | surfactant protein B | immunology |
| 2 1 | < 1e-07 | < 1e-07 | 173.9938924 | 62.4202949 | 2.7874571 | 210078 s at | Info | KCNA B1 | potassium voltage-gated channel, shaker-related subfamily, beta member 1 | |
| 22 | < 1e-07 | < 1e-07 | 3432.1403281 | 266.4844956 | 12.8793246 | 210342 s at | Info | TPO | thyroid peroxidase | Cytokine-cytokine receptor interaction, Hematopoiet ic cell lineage, Jak-STAT signaling pathway, Methane metabolism, Phenylalanin e metabolism, Stilbene, coumarine and lignin biosynthesis, Tyrosine metabolism, immunology |
| 2 3 | < 1e-07 | < 1e-07 | 823.9711385 | 7141.7242806 | 0.1153743 | 210495 x at | Info | FN1 | fibronectin 1 | Cell Communicat ion, ECM-receptor interaction, Focal adhesion, Regulation of actin cytoskeleton, angiogenesis cell_signalin g, immunology, metastasis |
| 2 4 | < 1e-07 | < 1e-07 | 18.279169 | 160.6084845 | 0.113812 | 211478 s at | Info | DPP4 | dipeptidyl-peptidase 4 | angiogenesis immunology, metastasis |
| 2 5 | < 1e-07 | < 1e-07 | 38.8267373 | 248.8295796 | 0.1560375 | 211564 s at | Info | PDLIM 4 | PDZ and LIM domain 4 | |
| 2 6 | < 1e-07 | < 1e-07 | 49.0409251 | 266.5172439 | 0.1840066 | 211668 s at | Info | PLAU | plasminogen activator, urokinase | Fibrinolysis Pathway, Platelet Amyloid Precursor Protein Pathway, Complement and coagulation cascades, angiogenesis |
| 27 | < 1e-07 | < 1e-07 | 74.57174 13 | 261.7442 717 | 0.28490 3 | 211679 x at | Info | GABB R2 | gamma-aminobutyric acid (GABA) B receptor, 2 | Neuroactive ligand-receptor interaction |
| 2 8 | < 1e-07 | < 1e-07 | 789.7331 311 | 6933.196 735 | 0.11390 61 | 211719 x at | Info | FN1 | fibronectin 1 | Cell Communicat ion, ECM-receptor interaction, Focal adhesion, Regulation of actin cytoskeleton, angiogenesis cell_signalin g, immunology, metastasis |
| 2 9 | < 1e-07 | < 1e-07 | 553.9947 808 | 5755.972 467 | 0.09624 69 | 212464 s at | Info | FN1 | fibronectin 1 | Cell Communicat ion, ECM-receptor interaction, Focal adhesion, Regulation of actin cytoskeleton, angiogenesis cell_signalin g, immunology, metastasis |
| 3 0 | < 1e 07 | < 1e-07 | 271.4822 924 | 1253.061 1466 | 0.21665 53 | 212611 at | Info | DEX4 Info DTX4 | deltex homolog 4 (Drosophila) | Notch signaling pathway |
| 3 1 | < 1e-07 | < 1e-07 | 49.12111 58 | 509.6069 493 | 0.09639 02 | 212850 s at | Info | LRP4 | low density lipoprotein receptor-related protein 4 | |
| 3 2 | < 1e-07 | < 1e-07 | 793.5035 614 | 7272.992 3868 | 28 | 216442 x at | Info | FN1 | fibronectin 1 | Cell Communicat ion, ECM-receptor interaction, Focal adhesion, Regulation of actin cytoskeleton, angiogenesis cell_signalin g, immunology, metastasis |
| 3 3 | < 1e-07 | < 1e-07 | 46.92619 03 | 174.3270 296 | 0.26918 48 | 217077 s at | Info | GABB R2 | gamma-aminobutyric acid (GABA) B receptor, 2 | Neuroactive ligand-receptor interaction |
| 3 4 | < 1e-07 | < 1e-07 | 66.58482 15 | 402.0784 869 | 0.16560 16 | 218002 s at | Info | CXCL1 4 | chemokine (C-X-C motif) ligand 14 | Cytokine-cytokine receptor interaction, Leukocyte transendothel ial migration |
| 35 | < 1e- 07 | < 1e- 07 | 71.3963571 | 268. 1310208 | 0.2662741 | 219250 s at | Info | FLRT3 | fibronectin leucine rich transmembrane protein 3 | |
| 36 | < 1e- 07 | < 1e- 07 | 37.30304 37 | 242.3706098 | 0.1539091 | 219630 at | Info | PDZK1 IP1 | PDZK1 PDZK interacting protein 1 | |
| 37 | < 1e- 07 | < 1e- 07 | 15.4817858 | 85.7078013 | 0.1806345 | 219932 at | Info | SLC27 A6 | solute carrier family 27 transporter), member 6 | PPAR signaling pathway |
| 38 | < 1e- 07 | < 1e- 07 | 30.23265 | 151.1507119 | 0.20001 66 | 220332 at | Info | CLDN16 | claudin 16 | Cell adhesion molecules (CAMs), Leukocyte transendothel ial migration, Tight junction |
| 3 9 | < 1e- 07 | < 1e- 07 | 29.73122 78 | 190.068532 | 0.1564237 | 221577 x at 221577 x at | Info | GDF15 | growth differentiation factor 15 | |
| 40 | < 1e- 07 | < 1e- 07 | 43.5670133 | 546.52968 | 0.0797157 | 37004 at | Info | SFTPB | surfactant protein B | immunology |
| 41 | < 1e-07 | < 1e 07 | 71.6371341 | 1249.8559384 | 0.0573163 | 202286 s at | Info | TACST D2 | tumour-associated calcium signal transducer 2 | |
| 42 | < 1e- 07 | < 1e- 07 | 212.7603836 | 719.0191974974 | 0.2959036 | 202458 at | Info | Info | protease, serine, 23 | Perou's-Intrinsic- Breast-Cancer-Genes |
| 43 | < 1e- 07 | < 1e- 07 | 17.9730618 | 17.9730618 | 0.3653648 | 220595 at | Info | PDZRN4 | PDZ domain containing ring finger 4 | |
| 4 4 | < 1e- 07 | < 1e- 07 | 83.355183 | 205.6220332 | 0.4053806 | 203963 at | Info | CA12 | carbonic anhydrase XII | Nitrogen metabolism |
| 4 5 | < 1e- 07 | < 1e- 07 | 136.8295229 | 87.512575 | 1.5635413 | 203708 at | Info | PDE4B | phosphodiest erase 4B, cAMP- specific (phosphodies terase E4 dunce homolog, Drosophila) | Purine metabolism, cell_signalin g, immunology, signal_transd uction |
| 4 | 0.0010 | 0.0010 | 73.38977 | 41.93794 | 1.74996 | AFFX-r2- | Info | NA | NA | |
| 6 | 141 | 802 | 38 | | 1 | Hs18SrRNA-5 at | | | | |
| 4 7 | 0.0040 077 | 0.0041 782 | 135.3311373 | 95.9312583 | 1.4107095 | AFFX-HUMRGE/M100 98 5 at | Info | NA | NA | |
| 4 8 | 0.326924 | 0.333749 | 182.8958546 | 147.7942985 | 1.2375028 | 214218 s at | Info | XIST | X (inactive)-specific transcript i (non-protein coding) | |
| 4 9 | 0.7916982 | 0.7916982 | 662.405757 | 603.9671328 | 1.096758 | AFFX-r2-Hs18SrRNA-3 s at | Info | NA | NA | |

### Example 4

### Analysis of microdissected samples

In the next step we extended the bulk PTC and benign/normal thyroid analysis, as described above, to the analysis of the gene expression profile of purified PTC cells. We used the microdissection technique to obtain a pure population of PTC cells and normal thyrocytes. To normalize for technological issues associated with the analysis of small specimen, we performed the analysis of single sections of PTC samples and compared their gene expression to the results obtained in pure populations of PTC cells and normal thyrocytes.

We obtained a list of transcripts significantly differentiating microdissected PTC and normal cells (Table 10).

**Table 10.**

| Gene symbol | | | Description | | | Probe set | | | Bulk sections | | | Microdissected cells | | |
|---|---|---|---|---|---|---|---|---|---|---|---|---|---|---|
| | | | Parametric p-value | FDR | Permutation p-value | Guz_noz | Guz norm | Fold-change | Parametric p-value | FDR | Permutation p-value | mic_NORMA | MIC_GUZ | Fold-change |
| CITED1 | Cbp/p30 0-interacting transactivator, with Glu/Asp-rich carboxy terminal domain, 1 | 207144_ s_at | 9.00E-07 | 0.00059 29 | 0.00216 45 | 3367.99 | 37.16 | 90.64 | 0.00000 38 | 0.00972 2 | 0.0004 | 919.63 | 12.87 | 71.47 |
| KITLG | KIT ligand | 226534_ at | 0.00010 16 | 0.00941 11 | 0.00216 45 | 122.81 | 468.82 | 0.26 | 0.00000 47 | 0.00972 2 | 0.0004 | 13.61 | 196.61 | 0.07 |
| NA | NA | 241898_ at | le-07 | < 1e-07 | 0.00216-45 | 283.47 | 10.49 | 27.02 | 0.00003 52 | 0.04854 08 | 0.0004 | 184.64 | 22.79 | 8.10 |
| LOC653 602 | hypothetical LOC653602 | 229546_ at | 0.00063 62 | 0.02727 76 | 0.00216 45 | 69.23 | 15.53 | 4.46 | 0.00005 08 | 0.05253 99 | 0.001 | 94.59 | 14.12 | 6.70 |
| PLA2R 1 | phospholipase A2 receptor 1, 180kDa | 235746_ s_at | 9.90E-06 | 0.00246 04 | 0.00216 45 | 33.32 | 529.57 | 0.06 | 0.00008 11 | 0.06710 21 | 0.0004 | 25.35 | 345.91 | 0.07 |
| GPM6A | glycoprotein M6A | 209469_ at | 2.26E-05 | 0.00388 57 | 0.00216 45 | 14.93 | 703.90 | 0.02 | 0.00018 55 | 0.10785 16 | 0.0013 | 32.49 | 300.35 | 0.11 |
| PROS1 | protein S (alpha) | 207808_ s_at | 4.00E-07 | 0.00036 45 | 0.00216 45 | 3811.97 | 303.04 | 12.58 | 0.00022 12 | 0.10785 16 | 0.0004 | 755.65 | 56.73 | 13.32 |
| CYP1B 1 | cytochrome P450, family 1, subfamily B, polypeptide 1 | 202435_2 s_at | 2.84E-05 | 0.00443 65 | 0.00216 45 | 1898.17 | 61.27 | 30.98 | 0.00024 69 | 0.10785 16 | 0.0015 | 525.40 | 40.47 | 12.98 |
| IGFBP6 | insulin-like growth factor binding protein 6 | 203851 at | 8.46E-05 | 0.00849 72 | 0.00216 45 | 3887.68 | 437.25 | 8.89 | 0.00025 74 | 0.10785 16 | 0.0004 | 549.17 | 42.73 | 12.85 |
| NA | NA | 222877 at | 06 | 0.00063 98 | 0.00216 45 | 582.35 | 54.80 | 10.63 | 0.00026 07 | 0.10785 16 | 0.0008 | 154.14 | 13.11 | 11.76 |
| CENTA 2 | centaurin, alpha 2 | 222876 at | 0.00191 92 | 0.05109 74 | 0.00432 9 | 23.56 | 9.23 | 2.55 | 0.00046 81 | 0.15905 17 | 0.0004 | 43.68 | 8.88 | 4.92 |
| LOC401 115 | hypothetical LOC401 115 | 229860_ x_at | 2.49E-05 | 0.00410 06 | 0.00216 45 | 321.18 | 49.02 | 6.55 | 0.00048 04 | 0.15905 17 | 0.0008 | 371.99 | 36.65 | 10.15 |
| CYP1B 1 | cytochrome P450, family 1, subfamily B, polypeptide 1 | 202437 s_at | 8.37E-05 | 0.00844 34 | 0.00216 45 | 2921.63 | 105.16 | 27.78 | 0.00049 98 | 0.15905 17 | 0.0004 | 23.33 | 36.94 | 16.88 |
| NA | NA | 231181 at | 7.00E-07 | 0.00051 03 | 0.00216 45 | 9.23 | 1441.72 | 0.01 | 0.0054 9 | 0.000541 0.16160 9 | 0.0027 | 38.83 | 815.24 | 0.05 |
| VASN | vasorin | 225867_ at | 3.20E-06 | 0.00120 66 | 3.00216 45 | 343.64 | 104.37 | 3.29 | 0.00061 61 | 0.16992 04 | 0.0025 | 241.20 | 26.39 | 9.14 |
| TFF3 | trefoil factor 3 at (intestinal) | 204623_ at | 1.00E-07 | 0.00014 78 | 0.00216 45 | 10.25 | 3564.281 | 0.00 | 0.00068 84 | 0.17799 44 | 0.002 | 56.29 | 1753.50 | 0.03 |
| SEMA6 D | sema domain, transmembrane domain (TM), and (cytoptasmic domain, (semaphorin) 6D | 226492_ at | 1.67E-05 | 0.00330 82 | 0.00216 45 | 95.71 | 353.72 | 0.27 | 0.00079 08 | 0.18333 8 | 0.0004 | 21.03 | 124.79 | 0.17 |
| NA | NA | 235801_ at | 5.10E-06 | 0.00160 25 | 0.00216 45 | 66.14 | 9.15 | 7.23 | 0.00079 77 | 0.18333 8 | 0.0011 | 70.40 | 8.46 | 8.32 |
| NA | NA | 229866_ at | 2.70E-06 | 0.00111 84 | 0.00216 45 | 244.24 | 16.14 | 15.13 | 0.00092 06 | 0.195201 73 | 0.0011 | 217.27 | 18.50 | 11.74 |
| EFEMP 1 | EGF-containing fibulin-like xtracellular matrix protein 1 | 201843_ s_at | 31 | 0.04819 0.00216 56 | 0.00216 45 | 239.23 | 1527.64 0.16 | 0.16 | 0.00095 85 | 0. 19520 73 | 0.0016 | 64.08 | 457.50 | 0.14 |
| TMEM7 9 | membrane protein 79 | 223544_at | 0.00051 62 | 0.02437 24 | 0.00216 45 | 43.59 | 10.00 | 4.36 | 0.00099 09 | 0.19520 73 | 0.003 | 108.17 | 20.73 | 5.22 |
| ZCCHC 12 | zinc finger, CCHC domain containing 12 | 228715_ at | 07 | 0.00046 2 | 0.00216 45 | 1108.79 | 14.47 | 76.63 | 0.00104 11 | 0.19554 22 | 0.0005 | 284.60 | 25.69 | 11.08 |
| GALE | UDP-galactose-4-epimerase | 1557651 _x_at | 0.00175 08 | 0.04869 02 | 0.00432 9 | 16.00 | 6.72 | 2.38 | 0.00111. 99 | 0.19554. 22 | 0.002 | 92.57 | 10.43 | 8.87 |
| GRB14 | growth factor receptor-bound protein 14 | 206204_ at | 91 | 0.07820 37 | 0.00865 8 | 13.56 | 45.40 | 0.30 | 0.00113 44 | 0.19554 22 | J.0046 | 19.55 | 111.92 | 0.17 |
| ARNTL | aryl hydrocarbon receptor nuclear translocator-like | 209824_ s_at | 0.00034 44 | 0.01925 92 | 0.00432 9 | 640.20 | 111.95 | 5.72 | 0.00123 09 | 0.19794 95 | 0.0025 | 262.79 | 35.39 | 7.43 |
| FHL1 | four and a half LIM domains | 210299_ s_at | 2 | 0.06412 56 | 4.00865 8 | 70.43 | 757.77 | 0.09 | 0.00126 46 | 0.19794 95 | 0.0026 | 14.60 | 147.12 | 0.10 |
| TRD@ | T cell receptor delta locus | 217143_ s_at | 48 | 6 | 0.00216 45 | 180.07 | 12.92 | 13.94 | 0.00131 6 | 0.19794 95 | 0.0012 | 180.81 | 40.34 | 4.48 |
| LOC730 107 | similar to Glycine cleavage system H protein, mitochondrial | 213129_ s_at | 0.00026 84 | 0.01680 91 | 0.00216 5 | 1179.47 | 3841.34 | 0.31 | 0.00136 74 | 0.19794 95 | 0.0023 | 417.63 | 1502.29 | 0.28 |
| SLC26 A7 | solute carrier family 26, member 7 | 239006_ at | 0.00130 41 | 0.04109 28 | 0.00216 45 | 399.69 | 11935.6 0 | 0.03 | 0.00143 5 | 0.19794 95 | 0.003 | 410.96 | 6175.14 | 1.07 |
| SAMM 50 | sorting and assembly machinery component 50 homolog (S. cerevisiae) | 201569_ s_at | 0.0023103 | 0.05749 46 | 0.00432 9 | 271.67 | 400.24 | 0.68 | 0.00148 04 | 0.19794 95 | 0.0026 | 37.05 | 217.28 | 0.17 |
| NA | NA | 205728_ at | 1.51E-05 | 0.00315 67 | 0.00216 45 | 561.51 | 28.92 | 19.41 | 0.00149 59 | 0.19794 95 | 0.0015 | 509.16 | 46.30 | 11.00 |
| LOC286 002 | hypothetical protein LOC286002 | 1557107 _at | 0.00102 01 | 0.03584 45 | 0.00216 45 | 29.74 | 1436.69 | 0.02 | 51 | 0.19794 95 | 0.0022 | 52.23 | 530.85 | 0.10 |
| PTPRF | protein tyrosine phosphatase, receptor type, F | 200637_ s_at | 0.00822 62 | 0.12234 14 | 0.00432 9 | 123.97 | 36.34 | 3.41 | 0.00161 35 | 0.19794 95 | 0.0054 | 92.86 | 15.36 | 6.05 |
| FAM20 A | family with sequence similarity y 20, member A | 226804_ at | 05 | 0.00834 41 | 0.00216 45 | 216.70 | 8.34 | 25.97 | 0.00163 63 | 0.19794 95 | 0.0049 | 91.91 | 11.42 | 8.05 |
| NMU | neuromedin U | 206023_ at | 0.00052 02 | 0.02443 47 | 0.00216 | 340.53 | 7.05 | 48.28 | 0.00167 47 | 0.19794 95 | 0.0004 | 283.70 | 44.13 | 6.43 |
| GDF15 | growth differentiation factor 15 | x_at | 6.96E-05 | 0.00760 65 | 0.00216. 45 | 446.70 | 10.35 | 43.16 | 0.00177 34 | 0.20379 32 | 0.0021 | 186.30 | 33.77 | 5.52 |
| C16orf4 6 | chromosome 16 open reading frame 46 | 230281_ at | 0.00078 87 | 0.03064, 83 | 0.00216 45 | 152.81 | 554.13 | 0.28 | 0.00194 55 | 0.21752 79 | 0.0014 | 55.12 | 279.68 | 0.20 |
| FN1 | fibronectin 1 | 216442_ x_at | 05 | 0.00760 65 | 0.00216 45 | 17633.2 5 | 1010.05 | 17.46 | 0.00201 44 | 0.21930 45 | 0.006 | 8786.33 | 139.11 | 63.16 |
| MGC53 70 | hypothetical protein MGC53 70 | 225160_ x_at | 0.00386 86 | 0.007782 03 | 0.00649 35 | 111.28 | 62.32 | 1.79 | 0.00220 14 | 0.22100 61 | 0.0027 | 368.87 | 212.30 | 1.74 |
| CCDC6 4B | coiled-coil domain containing 64B | 235095_ at | 0.00627 15 | 0.10437 14 | 0.00865 8 | 18.79 | 7.94 | 2.37 | 0.00220 41 | 0.22100 61 | 0.0065 | 111.37 | 21.47 | 5.19 |
| ID4 | inhibitor of DNA binding 4, dominant negative helix-loop-helix protein | 209291_ at | 8.39E-05 | 0.00844 79 | 0.00216 45 | 4195.54 | 13001.6 9 | 0.32 | 0.00232 13 | 0.22100 61 | 0.0034 | 1025.47 | 5189.18 | 0.20 |
| WDR72 | WD repeat domain 72 | 227174_ at | 0.00092 57 | 0.03386 92 | 0.00216 45 | 137.60 | 2812.50 | 0.05 | 0.00242 22 | 0.22100 61 | 0.0048 | 155.21 | 803.19 | 0.19 |
| IGSF1 | immunoglobulin superfamily, member 1 | 207695_s_at | 2.68E-05 | 0.004314 | 0.0021645 | 1083.80 | 47.58 | 22.78 | 0.0024314 | 0.2210061 | 0.0041 | 285.22 | 42.51 | 6.71 |
| SMOC2 | SPARC related modular calcium binding 2 | 223235_ s_at | 0.00058 62 | 0.02609 97 | 0.00216 45 | 176.95 | 1705.63 | 0.10 | 0.00244 7 | 0.22100 61 | 0.0058 | 29.88 | 348.77 | 0.09 |
| SFTPB | surfactant protein B | 37004_a t | 0.00020 99 | 0.01452 69 | 0.00432 9 | 3938.09 | 33.82 | 116.44 | 0.00256 64 | 0.122100 61 | 0.0056 | 893.10 | 170.82 | 5.23 |
| PIP3-E | phosphoinositide binding protein PIP3-E | 214735_ at | 07 | 0.00014 78 | 3.00216 45 | 13.78 | 652.12 | 0.02 | 0.00260 28 | 0.22100 61 | 0.0008 | 52.73 | 488.53 | 0.11 |
| FLJ215 11 | hypothetical protein FLJ21511 | 220724_ at | - 1.22E-05 | 0.00275 63 | 0.00216 45 | 8.62 | 73.63 | 0.12 | 0.00261 55 | 0.22100 61 | 0.002 | 14.65 | 119.36 | 0.12 |
| IGFBPL 1 | insulin-like growth factor binding protein-like 1 | 227760 at | 3.00E-07 | 0.00032 16 | 0.00216 45 | 7.11 | 337.84 | 0.02 | 0.00266 07 | 0.22100 61 | 0.0042 | 39.37 | 264.46 | 0.15 |
| CHI3L1 | chitinas 3-like 1 (cartilag glycoprotein-39) | 209396_ s_at | 3.00E-07 | 0.00032 16 | 0.00216 45 | 1120.87 | 8.99 | 124.67 | 0.00269 47 | 0.22100 61 | 0.0052 | 406.92 | 38.75 | 10.50 |
| S100A1 1 | S100 calcium binding protein A11 | 200660_ at | 1.45E-05 | 0.00307 28 | 0.00216 45 | 1562.36 | 362.93 | 4.30 | 0.00274 07 | 0.22100 61 | 0.0011 | 398.91 | 62.06 | 6.43 |
| C13orf3 1 | chromosome 13 open reading frame 31 | 228937_ at | 0.00829 2 | 0.12284 25 | 0.01082 25 | 130.38 | 69.24 | 1.88 | 0.00277 39 | 0.22100 61 | 0.0041 | 165.80 | 41.77 | 3.97 |
| ARPC5 L | actin related protein 2/3 complex , subunit 5-like | 223101_ s_at | 0.00197 13 | 0.05193 33 | 0.00216 45 | 148.25 | 67.59 | 2.19 | 0.00278 14 | 0.22100 61 | 0.0038 | 165.00 | 44.42 | 3.71 |
| ESRRG | -related receptor *gamma* | 207981_ s_at | 0.00026 06 | 0.01646 56 | 0.00216 45 | 25.92 | 267.80 | 0.10 | 0.00285 56 | 0.22100 61 | 0.0048 | 26.07 | 178.24 | 0.15 |
| SYT12 | synaptotagmin XII | 228072_ at | 2.10E-06 | 0.000939 35 | 0.00216 45 | 48.32 | 7.31 | 6.61 | 0.00290 48 | 0.22100 61 | 0.0024 | 197.81 | 34.46 | 5.74 |
| LRP2 | low density lipoprotein-related protein 2 | 230863_ at | 0.00067 25 | 0.02811 08 | 0.00216 45 | 100.86 | 1901.72 | 0.05 | 0.00293 82 | 0.22100 61 | 0.0014 | 146.67 | 966.38 | 0.15 |
| CTSH | cathepsin H | 202295_ s_at | 0.00022 89 | 0.01524 37 | 0.00216 45 | 5892.04 | 853.15 | 6.91 | 0.00299 17 | 0.22101 18 | 0.0057 | 1506.72 | 271.53 | 5.55 |
| LOC647 121 | embigin homolog (mouse) pseudogene | 226789_ at | 0.00011 64 | 0.01018 27 | 0.00216 5 | 1414.25 | 504.10 | 2.81 | 0.00312 01 | 0.22273 02 | 0.0023 | 312.21 | 57.65 | 5.42 |
| FN1 | fibronectin 1 | 210495_ x_at | 05 | 0.00654. 66 | 0.00216 45 | 20977.4 0 | 1192.51 | 17.59 | 0.00316 01 | 0.22273 02 | 0.0056 | 8077.99 | 127.17 | 63.52 |
| HLF | hepatic leukemia factor | 204755_ x_at | 0.00639 69 | 0.10566 48 | 0.01515 15 | 17.39 | 116.33 | 0.15 | 0.00319 5 | 0.22273 02 | 0.0035 | 44.07 | 201.33 | 0.22 |
| MPPED 2 | metallophosphoe sterase domain containing 2 | 205413_ at | _1.19E-05 | 0.00273 37 | 0.00216 45 | 23.15 | 1346.00 | 0.02 | 0.00324 12 | 0.22273 02 | 0.005 | 44.21 | 381.07 | 0.12 |
| CD58 | CD58 molecule | 211744_ s_at | 0.00183 27 | 0.04987 7 | 0.00649 35 | 329.67 | 152.59 | 2.16 | 0.00332 87 | 0.22273 02 | 0.006 | 67.68 | 11.22 | 6.03 |
| NA | NA | 215643_ at | 0.00040 17 | 0.02078 37 | 3.00649 35 | 7.60 | 14.84 | 0.51 | 0.00340 14 | 0.22273 02 | 0.0034 | 19.47 | 113.90 | 0.17 |
| CTSC | cathepsin C | 225646_ at | 0.0067 15 | 0.02809 05 | 0.00432 | 630.39 | 70.02 | 9.00 | 0.00345 59 | 0.22273 02 | 0.0038 | 185.22 | 28.62 | 6.47 |
| SCUBE 3 | signal peptide, CUB domain, EGF-like 3 | 228407_ at | 0.00015 62 | 0.01225 28 | 0.00216 45 | 275.42 | 1791.23 | 0.15 | 0.00351 37 | 0.22273 02 | 0.0043 | 77.37 | 509.39 | 0.15 |
| IL1RN | interleukin 1 receptor antagonist | 212657_ s_at | _0.00254 91 | 0.06100 39 | 0.00216 45 | 40.37 | 5.68 | 7.11 | 0.00356 55 | 0.22273 02 | 0.0029 | 142.33 | 31.88 | 4.46 |
| GABBR 2 | gamma-aminobutyric acid (GABA) B receptor, 2 | 209990_ is_at | 0.00019 09 | 0.01374 42 | 0.00216 45 | 958.83 | 86.32 | 11.11 | 0.00362 88 | 0.22273 02 | 0.0004 | 298.76 | 75.67 | 3.95 |
| ELF3 | E74-like factor 3 (ets domain transcription factor, epithelia ]-specific ) | 210827 s_at | 0.00075 45 | 0.02991 46 | 0.00216 | 145.16 | 20.23 | 7.18 | 0.00363 99 | 0.22273 02 | 0.0022 | 229.75 | 31.63 | 7.26 |
| NA | NA | 238726 at | 0.00147 63 | 0.04429 45 | 0.00216 45 | 7.80 | 13.78 | 0.57 | 0.00366 52 | 0.22273 02 | 0.0025 | 19.91 | 76.38 | 0.26 |
| TBC1D 4 | TBC 1 domain family, member 4 | 203386_ at | 3.30E-06 | 3.00121 91 | 3.00216 45 | 196.97 | 885.32 | 0.22 | 0.00373 72 | 0.22273 02 | 0.0037 | 212.06 | 727.97 | 0.29 |
| PLSCR 4 | phospholipid scramblase 4 | 218901_ at | 2.89E-05 | 0.00450 17 | 0.00216 45 | 377.87 | 1167.10 | 0.32 | 0.00376 87 | 0.22273 02 | 0.0038 | 101.20 | 493.76 | 0.20 |
| TPO | thyroid peroxidase | 210342_ s_at | 2.73E-05 | 0.00431 4 | 0.00216 45 | 37.43 | 3982.39 | 0.01 | 0.00393 84 | 0.22577 78 | 0.0078 | 63.79 | 921.36 | 0.07 |
| SLC26 A4 | solute carrier family 26, member 4 | 206529_ x_at | 0.00124 7 | 0.03999 29 | 0.00216 45 | 732.56 | 9651.46 | 0.08 | 0.00404 63 | 0.22577 78 | 0.0051 | 271.37 | 2818.44 | 0.10 |
| MATN2 | matrilin 2 | 202350_ s_at | 2.80E-06 | 0.00114 07 | 0.00216 45 | 263.80 | 5455.84 | 0.05 | 0.00407 04 | 0.22577 78 | 0.0068 | 144.23 | 1785.82 | 0.08 |
| SPINK2 | serine peptidase inhibitor , Kazal type 2 (acrosin-trypsin inhibitor ) | 206310_at | 0.00555 22 | 0.09708 96 | 0.00216 45 | 35.26 | 7.65 | 4.61 | 0.00417 79 | 0.22577 78 | 0.0056 | 85.34 | 12.25 | 6.97 |
| ZFP36L 2 | zinc finger protein 36, C3H type-like 2 | 201368_ at | 0.00018 69 | 0.01364 32 | 0.00216 45 | 3065.19 | 8310.52 | 0.37 | 0.00421 13 | 0.22577 78 | 0.0064 | 586.03 | 2037.05 | 0.29 |
| CD55 | CD55 molecule, decay accelerating factor for complement (Cromer blood group) | 201925_ s_at | 0.00018 27 | 0.01344 09 | 0.00216 45 | 4722.90 | 516.95 | 9.14 | 84 | 0.22577 78 | 0.004 709.79 | 709.79 | 94.81 | 7.49 |
| MXRA8 | matrix-remodelling associated 8 | 213422_ s_at | 1.30E-06 | 0.00069 01 | 0.00216 45 | 476.19 | 73.19 | 6.51 | 0.00422 45 | 0.22577 78 | | 447.78 | 161.58 | 2.77 |
| FHL1 | four and a half LIM domains 1 | 201540_ at | 0.00063 7 | 0.02727 76 | 0.00216 45 | 1277.10 | 11277.9 2 | 0.11 | 0.00438 85 | 0.22577 78 | 0.0068 | 211.12 | 2413.37 | 0.09 |
| SPATA 7 | spermatogenesis associated 7 | 234929 s_at | 0.00281 31 | 0.06457 02 | 0.00432 9 | 43.01 | 91.65 | 0.47 | 0.00448 61 | 0.22577 78 | 0.0057 | 19.79 | 98.23 | J.20 |
| OSR 1 | odd-skipped related 1 (Drosop hila) | 228399_ at | 0.00941 35 | 0.13237 73 | 0.01298 7 | 5.92 | 10.92 | 0.54 | 0.00449 81 | 0.22577 78 | 0.0032 | 16.82 | 53.68 | 0.31 |
| MAML D1 | mastermind-like domain containing 1 | 205088_ at | 2.94E-05 | 0.00455 37 | 0.00216 45 | 43.85 | 9.85 | 4.45 | 0.00452 42 | 0.22577 78 | 0.0025 | 117.20 | 32.06 | 3.66 |
| SFTPB | surfactant protein B | 209810_ at | 0.00032 84 | 0.01885 23 | 0.00432 9 | 3840.09 | 29.25 | 131.30 | 0.00468 47 | 0.22577 78 | 0.0044 | 1093.71 | 96.92 | 11.28 |
| SLC4A 4 | solute carrier family 4, sodium bicarbonate cotransporter, member 4 | 203908_ at | 06 | 0.00078 81 | 0.00216 45 | 16.17 | 1286.84 | 0.01 | 0.00471 06 | 0.22577 78 | 0.0063 | 24.77 | 335.41 | 0.07 |
| TBCB | tubulin folding cofactor B | 211759_ x_at | 0.00220 73 | 0.05597 59 | 0.01082 25 | 704.57 | 546.17 | 1.29 | 0.00473 75 | 0.22577 78 | 0.0049 | 219.14 | 48.94 | 4.48 |
| SERPIN A1 | serpin peptidas inhibitor, clade A (alpha-1 antiproteinase, antitrypsin), member 1 | 202833_ s_at | 07 | 0.00032 16 | 0.00216 45 | 5531.87 | 44.41 | 124.56 | 0.00479 61 | 0.22577 78 | 0.0073 | 251.38 | 17.40 | 14.45 |
| DIO 1 | deiodinase, iodothyronine, type I | 206457_ s_at | 0.00064 79 | 0.02752 44 | 0.00432 9 | 18.40 | 2316.26 | 0.01 | 0.00479 83 | 0.22577 78 | 0.0055 | 46.72 | 945.29 | 0.05 |
| CYTH3 | cytohesin 3 | 225147_ at | 1.60E-05 | 0.00325 2 | 0.00216 45 | 309.38 | 868.71 | 0.36 | 0.00490 61 | 0.22577 78 | 0.0059 | 233.48 | 505.77 | 0.46 |
| NA | NA | 239066 at | 0.00148 | 0.04448 69 | 0.00649 35 | 44.73 | 330.36 | 0.14 | 0.00493 66 | 0.22577 78 | 0.007 | 31.74 | 227.30 | 0.14 |
| ZMYM 1 | zinc finger, MYM-type 1 | 220206_ at | 0.00913 57 | 0.12997 51 | 0.00865 8 | 64.78 | 91.69 | 0.71 | 0.00502 42 | 0.22577 78 | 0.0069 | 17.93 | 128.56 | 0.14 |
| ANXA1 | annexin A1 | 201012_ at | 5.50E-06 | 0.00169 89 | 0.00216 45 | 10021.2 0 | 2213.13 | 4.53 | 0.00509 1 | 0.22577 78 | 0.005 | 2135.93 | 404.22 | 5.28 |
| SH3RF 1 | SH3 domain containing ring finger 1 | 225589_ at | 2.94E-05 | 0.00455 37 | 0.00216 45 | 263.99 | 629.98 | 0.42 | 0.00518 34 | 0.22577 78 | 0.0055 | 167.78 | 415.81 | 0.40 |
| TIMP1 | TIMP metallopeptidase inhibitor 1 | 201666_ at | 6.30E-06 | 0.00182 25 | 0.00216 45 | 8889.04 | 906.84 | 9.80 | 0.00521 71 | 0.22577 78 | 0.0092 | 815.59 | 139.36 | 5.85 |
| MPG | N-methylpurine-DNA glycosylase | 203686_ at | 0.00829 51 | 0.12284 25 | 0.01082 25 | 242.19 | 162.70 | 1.49 | 0.00530 1 | 0.22577 78 | 0.006 | 164.49 | 27.41 | 6.00 |
| CXCL1 7 | chemokine (C-X-C motif) ligand 17 | 226960_ at | 0.00082 77 | 0.03158 02 | 0.00216 45 | 68.00 | 9.37 | 7.26 | 0.00531 57 | 0.22577 78 | 0.0117 | 83.08 | 16.00 | 5.19 |
| FCGBP | Fc fragment of IgG binding protein | 203240_ at | 3.18E-05 | 0.00482 96 | 0.00216 45 | 469.58 | 4852.45 | 0.10 | 0.00535 74 | 0.22577 78 | 0.0067 | 448.04 | 1485.55 | 0.30 |
| SDC4 | syndecan 4 | 202071_ at | 8.00E-07 | 0.00055 37 | 0.00216 45 | 6390.26 | 700.32 | 9.12 | 0.00536 15 | 0.22577 78 | 0.0058 | 853.83 | 116.42 | 7.33 |
| MACF1 | microtubule-actin crosslinking factor 1 | 214894_ x_at | 0.00664 2 | 0.10798 03 | 0.004329 | 657.30 | 390.26 | 1.68 | 0.00538 45 | 0.22577 78 | 0.0011 | 231.17 | 54.73 | 4.22 |
| C26orf17 4 | chromosome 6 open reading frame 174 | 233050_ at | 0.00928 76 | 0.13128 22 | 0.00432 9 | 5.69 | 4.72 | 1.42 | 0.00539 25 | 0.22577 78 | 0.0064 | 12.32 | 87.57 | 0.14 |
| CHI3L1 | chitinase 3-like 1 (cartilagglycopr otein-39) | 209395_ at | 5.00E-07 | 0.00041 42 | 0.00216 45 | 4181.48 | 15.03 | 278.18 | 0.00548 16 | 0.22577 78 | 0.0079 | 401.86 | 32.65 | 12.31 |
| COL13 A1 | collagen type XIII, alpha 1 | 211343_ s_at | 4.39E-05 | 0.00595 47 | 0.00216 45 | 221.88 | 20.48 | 10.83 | 0.00551 18 | 0.22577 78 | 0.0086 | 128.04 | 20.37 | 6.28 |
| CITED2 | Cbp/p30 0-interacting transactivator, with Glu/Asp-rich carboxy terminal domain, 2 | 209357_at | 0.00603 69 | 0.10204 03 | 0.01298 7 | 593.83 | 2364.65 | 0.25 | 0.00551 21 | 0.22577 78 | 0.0048 | 117.26 | 668.92 | 0.18 |
| PLCD3 | phospholipase C, delta 3 | 234971_ x_at | 3.24E-05 | 0.00489 36 | 0.00216 45 | 70.33 | 14.71 | 4.78 | 0.00568 46 | 0.23056 07 | 0.0077 | 166.49 | 41.06 | 4.06 |
| CFD | complement factor D (adipsin) | 205382_ s_at | J5 | 0.00838 89 | 0.00432 9 | 69.02 | 832.56 | 0.08 | 0.00594 44 | 0.23670 8 | 0.006 | 41.77 | 290.54 | 0.14 |
| ACAD M | acyl-Coenzyme A dehydrogenase, C-4 to C-12 straight chain | 202502 at | 0.00267 53 | 0.06272 39 | 0.00865 8 | 642.74 | 1067.25 | 0.60 | 0.00595 06 | 0.23670 8 | 0.0124 | 187.46 | 408.42 | 0.46 |
| IGSF3 | immunoglobulin superfamily, member 3 | 202421_ at | 7.30E-06 | 0.00201 58 | 0.00216 45 | 642.74 | 94.90 | 4.68 | 0.00613 88 | 0.24064 85 | 0.0026 | 267.01 | 54.92 | 4.86 |
| CXCR7 | chemokine (C-X-C motif) receptor 7 | 212977_ at | 0.00749 92 | 0.11587 39 | 0.00432 9 | 522.43 | 1678.35 | 0.31 | 0.00616 6 | 0.24064 85 | 0.0054 | 78.77 | 374.40 | 0.21 |
| THBS1 | thrombospondin 1 | 201110_ s_at | 43 | 0.04871 | 0.00432 9 | 956.72 | 151.44 | 6.32 | 0.00633 92 | 0.24509 6 | J.0093 | 144.81 | 30.65 | 4.72 |
| ANXA2 | annexin A2 | 213503_x_at | 0.0012203 | 0.0394721 | 0.0021645 | 5966.49 | 2782.61 | 2.14 | 0.0065609 | 0.2513189 | 0.0032 | 1066.07 | 414.26 | 2.57 |
| CLCNK B | chloride channel Kb | 1554748 _at | 2.16E-05 | 0.00379 74 | 0.00216 45 | 12.22 | 282.29 | 0.04 | 0.00672 39 | 0.25519 98 | 0.0107 | 30.66 | 230.02 | 0.13 |
| NPC2 | Nieman n-Pick disease, type C2 | 200701_ at | 3.20E-06 | 0.00120 66 | 0.00216 45 | 12426.9 7 | 2749.10 | 4.52 | 0.00726 17 | 0.26951 57 | 0.0038 | 2737.80 | 402.75 | 6.80 |
| CTSC | cathepsin C | 225647_ s_at | 4.75E-05 | 0.00621 31 | 0.00216 45 | 834.13 | 68.84 | 12.12 | 0.00727 9 | 0.26951 57 | 0.0082 | 126.33 | 32.47 | 3.89 |
| MET | met proto-oncogene (hepatocyte growth factor receptor ) | 203510_ at | 1.00E-07 | 0.00014 78 | 0.00216 45 | 9967.58 | 965.86 | 10.32 | 0.00737 68 | 0.26951 57 | 0.0102 | 1441.07 | 273.60 | 5.27 |
| NA | NA | 240135_ x_at | 0.00394 51 | 0.07860 73 | 0.01298 7 | 9.50 | 50.59 | 0.19 | 0.00739 21 | 0.26951 57 | 0.0109 | 18.99 | 122.56 | 0.15 |
| SIGLEC 15 | sialic acid binding Ig-like lectin 15 | 215856_ at | 0.00150 21 | 0.04473 17 | 0.00216 45 | 47.70 | 7.98 | 5.98 | 0.00742 86 | 0.26951 57 | 0.0097 | 121.34 | 21.68 | 5.60 |
| CABLE S1 | Cdk5 and Abl substrate 1 | 225531_ at | 0.00575 63 | 0.09915 74 | 0.00649 35 | 10.91 | 26.15 | 0.42 | 0.00757 07 | 0.26951 57 | 0.0031 | 37.74 | 149.99 | 0.25 |
| GABRB 2 | gamma-aminobutyric acid (GABA) A receptor, beta 2 | 242344_ at | 07 | 0.00036 45 | 0.00216 45 | 2470.05 | 12.09 | 204.31 | 0.00765 95 | 0.26951 57 | 0.005 | 509.68 | 64.99 | 7.84 |
| MY06 | myosin VI | 203215_ s_at | 0.00800 32 | 0.12051 09 | 0.01298 7 | 651.84 | 108.27 | 6.02 | 0.00770 62 | 0.26951 57 | 0.0029 | 310.63 | 64.00 | 4.85 |
| IRS1 | insulin receptor substrat e 1 | 204686_ at | 0.00101 73 | 0.03581 51 | 0.00432 9 | 335.72 | 1771.15 | 0.19 | 0.00775 12 | 0.26951 57 | 0.007 | 159.65 | 787.06 | 0.20 |
| CDH16 | cadherin 16, KSP-cadherin | 206517_ at | 3.60E-06 | 0.00127 81 | 0.00216 45 | 6.04 | 100.55 | 0.06 | 0.00795 6 | 0.26951 57 | 0.0104 | 14.66 | 112.73 | 0.13 |
| FOXE1 | for khead box E1 (thyroid transcription factor 2) | 206912_ at | 0.00414 7 | 0.08099 57 | 0.00865 8 | 1383.29 | 2535.58 | 0.55 | 0.00796 89 | 0.26951 57 | 0.0121 | 387.57 | 762.64 | 0.51 |
| C12orf3 9 | chromosome 12 open reading frame 39 | 229778_ at | 2.60E-06 | 0.00109 35 | 0.00216 45 | 11.11 | 424.21 | 0.03 | 0.00797 03 | 0.26951 57 | 0.0104 | 22.55 | 241.66 | 0.09 |
| LGALS 3 | lectin, galactoside-binding, soluble, 3 | 208949_ s_at | 1.00E-07 | 0.00014 78 | 0.00216 45 | 13531.0 2 | 2491.44 | 5.43 | 0.00804 37 | 0.26951 57 | 0.0086 | 1839.52 | 353.04 | 5.21 |
| SLC2A 13 | solute carrier family 2 (facilitated glucose transporter), member 13 | 227176_ at | 3.94E-05 | 0.00555 2 | 0.00216 45 | 292.86 | 114.74 | 2.55 | 0.00804 67 | 0.26951 57 | 0.0012 | 142.34 | 52.50 | 2.71 |
| PAPSS23 | 3@#$% &-phospho adenosine 5@#$% &-phosphosulfate synthase 2 | 203060 s_at | 0.00015 66 | 0.01226 66 | 0.00216 45 | 110.66 | 592.69 | 0.16 | 0.00811 32 | 0.26951 57 | 0.007 | 70.47 | 436.61 | 0.16 |
| TMED4 | membrane emp24 protein transport domain containing 4 | 1558487 _a_at | 0.00076 21 | 0.03002 | 0.004329 | 433.76 | 1619.53 | 0.27 | 0.00820 86 | 0.26951 57 | 0.0098 | 264.02 | 863.06 | 0.31 |
| ALDH6 A1 | aldehyde dehydrogenase 6 family, member A1 | 221589_ s_at | 7.95E-05 | 0.00825 83 | 0.00216 45 | 611.73 | 1179.56 | 0.52 | 0.00820 86 | 0.26951 57 | 0.0124 | 178.28 | 488.44 | 0.36 |
| LRP4 | low density lipoprotein receptor-related protein 4 | 212850_ s_at | 6.00E-07 | 0.00046 2 | 0.00216 45 | 1066.97 | 23.08 | 46.23 | 0.00830 13 | 0.26958 62 | 0.0061 | 330.42 | 443.40 | 7.61 |
| DPP6 | dipeptidyl-peptidase 6 | 228546_ at | 1.00E07 | 0.00014. 78 | 0.00216 45 | 5.60 | 485.68 | 0.01 | 0.00848 47 | 0.26958 62 | 0.012 | 15.54 | 97.72 | 0.16 |
| SFTPB | surfactant protein B | 213936_ x_at | 1.53E-05 | 0.00315 67 | 0.00216 45 | 1300.91 | 19.25 | 67.59 96 | 0.00850 96 | 0.26958 62 | 0.0036 | 506.83 | 132.13 | 3.84 |
| PSPH | phosphoserine phosphatase | 205194_ at | 0.00227 25 | 0.05702 11 | 0.00216 45 | 61.94 | 119.73 | 0.52 | 0.00854 71 | 0.26958 62 | 0.0102 | 72.72 | 167.15 | 0.44 |
| SORBS 2 | sorbin and SH3: domain containing 2 | 225728_ at | 6.28E-05 | 0.00712 36 | 0.00216 45 | 939.00 | 7510.50 | 0.13 | 0.00859 35 | 0.26958 62 | 0.0107 | 261.90 | 1497.00 | 0.17 |
| IDII | isopentenyl-liphosphate delta isomerase 1 | 204615_ x_at | 0.00141 47 | 0.04335 169 | 0.00216 45 | 277.60 | 546.29 | 0.51 | 0.00860 84 | 0.26958 62 | 0.0083 | 34.51 | 253.79 | 0.14 |
| PAX8 | paired box 8 | 209552_ at | 0.00136 57 | 0.04228 18 | 0.00216 45 | 385.03 | 1063.33 | 0.36 | 0.00866 69 | 0.26958 62 | 0.0016 | 242.68 | 763.49 | 0.32 |
| SELEN BP1 | selenium binding protein 1 | 214433_ s_at | 0.00225 79 | 0.05686 35 | 0.00649 35 | 236.10 | 963.75 | 0.24 | 0.00873 32 | 0.26962 13 | 0.0076 | 158.70 | 586.48 | 0.27 |
| RUNX2 | runt-related transcription factor 2 | 232231_ at | 0.00129 19 | 0.04083 41 | 0.00649 35 | 865.03 | 92.02 | 9.40 | 0.0088738 | 0.27193 27 | 0.01 | 235.07 | 30.89 | 7.61 |
| VDR | vitamin D (1,25-dihydroxyvitamin D3) receptor | s_at | 0.00045 52 | 0.02250 28 | 0.00216 45 | 163.34 | 46.02 | 3.55 | 0.00903 45 | 0.27378 31 | 0.0101 | 138.14 | 25.08 | 5.51 |
| UNC5CL | unc-5 homolo at g C (C. elegans)-like | 231008_at | 0.0001441 | 0.0116376 | 0.0021645 | 72.28 | 11.53 | 6.27 | 0.0090967 | 0.2737831 | 0.0026 | 225.75 | 51.85 | 4.35 |
| RAB11 FIP4 | RAB11 family at interacting protein 4 (class II) | 225739_ at | 0.00439 52 | 0.08407 33 | 0.00865 8 | 50.61 | 22.08 | 2.29 | 0.00916 74 | 0.27378 31 | 0.0092 | 81.57 | 18.01 | 4.53 |
| TMCC3 | transmembrane and coiled-coil domain family 3 | 235146_ at | 05 | 0.00871 26 | 0.00216 45 | 1935.16 | 397.71 | 4.87 | 0.00919 89 | 0.27378 31 | 0.0102 | 463.24 | 167.09 | 2.77 |
| SORD | sorbitol dehydro at genase | 201563_ at | 0.00199 12 | 0.05224 79 | 0.00216 45 | 1974.25 | 5785.17 | 0.34 | 0.00956 7 | 0.2805 | 0.0097 | 593.65 | 1587.81 | J.37 |
| BEX1 | brain expressed, X-linked 1 | 218332_ at | 3.28E-05 | 0.00491 33 | 0.00216 45 | 79.17 | 1456.43 | 0.05 | 0.00963 23 | 0.2805 | 0.0111 | 101.64 | 479.54 | 0.21 |
| MSN | moesin | 200600_ at | 0.00195 76 | 0.05178 12 | 0.00432 9 | 3932.03 | 1599.99 | 2.46 | 0.00970 03 | 0.2805 | 0.0077 | 589.24 | 202.92 | 2.90 |
| CCND3 | cyclin D3 | 201700_ at | 0.00032 51 | 0.01873 58 | 0.00216 45 | 646.94 | 371.74 | 1.74 | 0.00972 46 | 0.2805 | 0.0058 | 225.46 | 60.20 | 3.75 |
| SCUBE 3 | signal peptide, CUB domains, EGF-like 3 | 230290_ at | 0.001950 7 | 0.05178 12 | 0.01082 25 | 9.00 | 69.94 | 0.13 | 0.00983 14 | 0.2805 | 0.0109 | 35.40 | 173.12 | 0.20 |

Next, we tested the genes from the microdissection on the full dataset of 281 samples, 140 samples of normal/benign thyroid and 141 PTC samples analyzed by both generations of microarrays. 89 probe sets from microddisected samples analysis matched the array data. The Recursive Feature Elimination method was used to select the best 40 genes. The 0.632+ bootstrap cross-validation method was used to compute misclassification rate. 91% of all samples were correctly predicted,. with sensitivity 0.882, specificity 0.871, PPV 0.873 and NPV 0.880.

**Table 11.**

| | Parametr ic p-value | t-value | % CV support | Geom mean of intensities in class "normal" | Geom mean of intensities in class "PTC" | Fold-change | Probe set | Annotations | Gene symbol | Description |
|---|---|---|---|---|---|---|---|---|---|---|
| 1 | < 1e-07 | 26.76 | 66 | 793.503561 4 | 7272.9923868 | 0.1091028 | 216442 x at | Info | FN1 | fibronectin 1 |
| | < 1e-07 | 22.288 | 66 | 9.1211158 | 509.6069493 | 0.0963902 | 212850 s at | Info | LRP4 | low density lipoprotein receptor-related protein 4 |
| 3 | < 1e-07 | 19.447 | 66 | 33.333555 | 1352.1461766 66 | 0.1725653 | 203510 at | Info | MET | met proto-oncogene (hepatocyte growth factor receptor) |
| 4 | < 1e-07 | 18.654 | 66 | 44.3338181 | 718.4503458 | 0.0617076 | 209396 s at | Info | CHI3L1 | chitinase 3-like 1 (cartilage glycoprotein-39) |
| 5 | < 1e-07 | 18.145 | 66 | 461.703041 8 | 963.4248862 | 0.479231 | 200660 at | Info | S100A11 | S 100 calcium binding protein A11 |
| 6 | < 1e-07 | 17.152 | 66 | 64.7621322 | 753.1097576 | 0.085993 | 209810 at | Info | SFTPB | surfactant protein B |
| 7 | < 1e-07 | 17.017 | 66 | 43.5670133 | 546.52968 | 0.079715 7 | 37004 at | Info | SFTPB | surfactant proteins |
| 8 | < 1e-07 | 16.968 | 66 | 29.5735542 | 235.1422655 | 0.1257688 | 202437 s at | Info | CYP1B1 | cytochrome P450, family 1, subfamily B, polypeptide 1 |
| 9 | < 1e-07 | 15.403 | 66 | 34.0598459 | 164.4190941 | 0.2071526 | 202435 s at | Info | CYP1B1 | cytochrome P450, family 1, subfamily B, polypeptide 1 |
| 10 | < 1e-07 | 15.263 | 66 | 1207.4997386 | 3932.9914083 | 0.307018 | 201012 at Info | Info | ANXA1 | annexin A1 |
| 11 | < 1e-07 | 14.472 | 66 | 67.7872884 | 244.9338249 | 0.2767576 | 213936 x at | Info | SFTPB | surfactant protein B |
| 12 | < 1e-07 | 14.017 | 66 | 886.233399 | 3400.8528771 | 0.2605915 | 201666 at | Info | TIMP1 | 'TIMP metallopeptid ase inhibitor |
| 13 | < 1e-07 | 13.16 | 66 | 16.4470523 | 44.5614234 | 0.369087 2 | 211343 s at | Info | COL13A1 | collagen, type XIII, alpha 1 |
| 14 | < 1e-07 | 9.043 | 66 | 258.6153879 | 676.5019908 | 0.3822833 | 203851 at | Info | IGFBP6 | insulin-like growth factor binding protein 6 |
| 15 | < 1e-07 | 8.942 | 66 | 75.1000965 | 135.6912968 | 0.5534629 | 211744 s at | Info | CD58 | CD58 molecule |
| 16 | < 1e-07 | 6.513 | 66 | 27.1115504 | 51.9731657 | 0.5216452 | 201110s at | Info | THBS1 | thrombospond |
| 17 | < 1e-07 | 6.107 | 66 | 337.860929 | 430.2448653 | 0.7852759 | 214894 x at | Info | MACF 1 | microtubule-actin crosslinking factor 1 |
| 18 | 4e-07 | -5.197 | 66 | 498.5889649 | 576.9623518 | 0.864162 | 211759 x at | Info | TBCB | tubulin folding cofactor B |
| 19 | 0.4227642 | 0.803 | 66 | 42.3737825 | 40.6492273 | 1.0424253 | 220206 at | Info | ZMYM1 | zinc finger, MYM-type 1 |
| 20 | 1e-05 | 4.5 | 66 | 30.3610545 | 21.3178921 | 1.4242053 | 205194 at | Info | PSPH | phosphoserine phosphatase |
| 21 | 8e-07 | 5.046 | 66 | 22.1797533 | 16.189094 | 1.3700429 | 206204 at | Info | GRB14 | growth factor receptor-bound protein 14 |
| 22 | < 1e-07 | 6.894 | 66 | 172.9871971 | 96.523038 | 1.7921856 | 221589 s at | Info | ALDH6A 1 | aldehyde dehydrogenas e 6 family, member A1 |
| 23 | < 1e-07 | 7.604 | 66 | 1161.1935938 | 583.0268364 | 1.9916641 | 213129 s at | Info | LOC7301 07 | similar to Glycine cleavage system H protein, mitochondrial |
| 24 | < 1e-07 | 8.253 | 66 | 361.431533 2 | 276.7636458 | 1.3059213 | 204615 x at | Info | IDI 1 | isopentenyl-diphosphate delta isomerase 1 |
| 25 | < 1e-07 | 8.952 | 66 | 108.747295 | 53.3707994 | 2.0375804 | 204755 x at | Info | HLF | hepatic leukemia factor |
| 26 | < 1e-07 | 9.005 | 66 | 1702.984976 | 826.1757774 | 2.0612865 | 209552 at | Info | PAX8 | paired box 8 |
| 27 | < 1e-07 | 9.236 | 66 | 1944.7243195 | 1 1085.9947577 | 1.7907309 | 201368 at | Info | ZFP36L2 | zinc finger protein 36, C3H type-like |
| 28 | < 1e-07 | 9.342 | 66 | 85.2551026 | 36.8596795 | 2.3129638 | 207981 s at | Info | ESRRG | estrogen-related receptor gamma |
| 29 | < 1e-07 | 10.308 | 66 | 1983.0524634 | 908.556636 | 2.1826404 | 201563 at | Info | SORD | sorbitol dehydrogenas e |
| 30 | < 1e-07 | 10.485 | 66 | 343.3413845 | 136.4927831 | 2.5154545 | 204686 at | Info | IRS 1 | insulin receptor substrate 1 |
| 31 | < 1e-07 | 11.093 | 66 | 852.4120367 | 420.6123745 | 2.0265976 | 214433 s at | Info | SELENBP 1 | selenium binding protein 1 |
| 32 | < 1e-07 | 11.524 | 66 | 839.1006393 | 181.1786953 | 4.6313428 | 206529 x at | Info | SLC26A4 | solute carrier family 26, member 4 |
| 33 | < 1e-07 | 1 12.971 | 66 | 143.0049641 | 65.5097755 | 2.1829561 | 203386 at | Info | TBC1D4 | TBC 1 domain family, member 4 |
| 34 | < 1e-07 | 15.447 | 66 | 442.5685623 | 92.6038329 | 4.7791603 | 218332 at | Info | BEX1 | brain expressed, X-linked 1 |
| 35 | < 1e-07 | 16.162 | 66 | 1292.2581334 | 103.1253572 | 12.5309446 | 206457 s at | Info | DIO1 | deiodinase, iodothyronine, type I |
| 36 | < 1e-07 | 16.283 | 66 | 3432.1403281 | 266.4844956 | 12.8793246 | 210342 at | Info | TPO | thyroid peroxidase |
| 37 | < 1e-07 | 17.693 | 66 | 203.9923738 | 43.6205541 | 4.6765195 | 206517 at | Info | CDH16 | cadherin 16, KSP-cadherin |
| 38 | < 1e-07 | 18.227 | 66 | 1546.7928322 | 248.4189175 | 6.2265501 | 202350 s at | Info | MATN2 | matrilin 2 |
| 39 | < 1e-07 | 19.844 | 66 | 404.9159288 | 66.1209457 | 6.1238678 | 214735 at | Info | IPCEF 1 | interaction protein for cytohesin exchange factors 1 |
| 40 | < 1e-07 | 21.45 | 66 | 1888.2708521 | 115.0757254 | 16.4089415 | 204623 at | Info | TFF3 | trefoil factor 3 (intestinal) |

On the basis of the results obtained, we designed a classification scheme based on 40 genes. The prediction is based on a weighted sum of the expression of these genes, and a result exceeding -22.512 is evidence to classify a sample as normal.

The prediction rule is defined by the inner sum of the weights and expression of significant genes. A sample is classified to the class normal if the sum is greater than the threshold. The threshold for the Support Vector Machine predictor is -22.512

**Table 12.**

| Table Gene Weights | Genes | Support Vector Machines |
|---|---|---|
| 1 | 204755_x_at | -1.2008 |
| 2 | 220206_at | -1.0342 |
| 3 | 204686_at | 0.9135 |
| 4 | 204615_x_at | 0.9176 |
| 5 | 202435_s_at | -0.9174 |
| 6 | 211759_x_at | -0.8181 |
| 7 | 212850_s_at | -0.7167 |
| 8 | 201563_at | 0.7312 |
| 9 | 209810_at | -0.6582 |
| 10 | 206529_x_at | 0.633 |
| 11 | 201666_at | -0.6368 |
| 12 | 211343_s_at | 0.6352 |
| 13 | 216442_x_at | -0.6334 |
| 14 | 221589_s_at | 0.5951 |
| 15 | 209552_at | 0.5736 |
| 16 | 37004_at | 0.5084 |
| 17 | 201110_s_at | 0.5668 |
| 18 | 214735_at | -0.5482 |
| 19 | 203386_at | 0.5326 |
| 20 | 209396_s_at | 0.5191 |
| 21 | 203851_at | 0.4914 |
| 22 | 210342_s_at | -0.4755 |
| 23 | 201012_at | -0.4837 |
| 24 | 202437_s_at | 0.4525 |
| 25 | 203510_at | -0.4131 |
| 26 | 207981_s_at | -0.418 |
| 27 | 214433_s_at | -0.4249 |
| 28 | 204623_at | 0.4028 |
| 29 | 202350_s_at | -0.3642 |
| 30 | 201368_at | -0.413 |
| 31 | 206517_at | -0.3702 |
| 32 | 200660_at | -0.3594 |
| 33 | 206204_at | -0.2993 |
| 34 | 213936_x_at | -0.3067 |
| 35 | 35 211744_s_at | -0.2296 |
| 36 | 218332_at | 0.299 |
| 37 | 206457_s_at | 0.2255 |
| 38 | 213129_s_at | 0.2021 |
| 39 | 205194_at | -0.1509 |
| 40 | 214894_x_at | -0.0906 |

Finally, we obtained 13 probe sets (10 genes), which differ both in two microarray datasets (one treated as the training group, and the second one as the validation group) and in microdissection-obtained data.

**Table 13.**

| | Parametric p-value | FDR | Geom mean of intensities in class "normal" | Geom mean of intensities in class "PTC" | Fold-change | Probe set | Gene symbol | Description |
|---|---|---|---|---|---|---|---|---|
| 1 | < 1e-07 | < 1e-07 | 1207.4997386 | 3932.9914083 | 0.3070181 | 201012 at | ANXA1 | annexin A1 |
| 2 | < 1e-07 | < 1e-07 | 158.1719537 | 910.2062231 | 0.173776 | 207808 s at | PROS1 | protein S (alpha) |
| 3 | < 1e-07 | < 1e-07 | 39.7815771 | 790.6956874 | 0.0503121 | 209395 at | CHI3L1 | chitinase 3-like 1 (cartilage glycoprotein-39) |
| 4 | < 1e-07 | < 1e-07 | 44.3338181 | 718.4503458 | 0.0617076 | 209396 s at | CHI3L1 | chitinase 3-like 1 (cartilage glycoprotein-39) |
| 5 | < 1e-07 | < 1e-07 | 64.7621322 | 753.1097576 | 0.085993 | 209810 at | SFTPB | surfactant protein B |
| 6 | < 1e-07 | < 1e-07 | 3432.1403281 | 266.4844956 | 12.8793246 | 210342 s at | TPO | thyroid peroxidase |
| 7 | < 1e-07 | < 1e-07 | 823.9711385 | 7141.7242806 | 0.1153743 | 210495 x at | FN1 | fibronectin 1 |
| 8 | < 1e-07 | < 1e-07 | 49.1211158 | 509.6069493 | 0.0963902 | 212850 s at | LRP4 | low density lipoprotein receptor-related protein 4 |
| 9 | < 1e-07 | < 1e-07 | 793.5035614 | 7272.9923868 | 0.1091028 | 216442 x at | FN1 | fibronectin 1 |
| 10 | < 1e-07 | < 1e-07 | 29.7312278 | 190.068532 | 0.1564237 | 221577 x at | GDF15 | growth differentiation factor 15 |
| 11 | < 1e-07 | < 1e-07 | 233.3335553 | 1352.1461766 | 0.1725653 | 203510 at | MET | met proto-oncogene (hepatocyte growth factor receptor) |
| 12 | < 1e-07 | < 1e-07 | 84.7811374 | 892.1276994 | 0.0950325 | 207144s at | CITED1 | Cbp/p300-interacting transactivator, with Glu/Asp-rich carboxy-terminal domains, 1 |
| 13 | < 1e-07 | < 1e-07 | 143.5670133 | 546.52968 | 0.0797157 | 37004 at | SFTPB | surfactant protein B |

These genes, ANXA1, CHI3L1, CITED1, FN1, GDF15, LRP4, MET, PROS1, SFTPB, TPO shown by the performed analysis to be the top genes, together with three knowledge-derived transcripts (MPPED2, TFF3, DPP4) constitute the optimal signature to differentiate between PTC and other thyroid tumours, which has been validated in two clinical microarray datasets and one microdissection-obtained dataset.

Conclusion: The following set of genes is proposed for molecular diagnosis of papillary thyroid cancer: ANXA1, CHI3L1, CITED1, FN1, GDF15, LRP4, MET, PROS1, SFTPB, TPO, MPPED2, TFF3, DPP4.

### Example 5

### The use of a set based on genetic signature in the diagnosis of papillary thyroid cancer

The previously selected 13 diagnostic genes were verified in 52 samples collected from mildly changed or unchanged thyroids, as well as from 44 samples of papillary thyroid cancer. The Support Vector Machine technique was used, and the quality of the classification was assessed using the cross-validation method. In the present example, we managed to correctly identify 96% of the tested samples. The method was characterised by a sensitivity of 95.5%, a specificity of 96.2%, a positive result predictive value of 95.5% and a negative result predictive value of 96.2%. These results are sufficient to transfer the technique into clinical use.

### Example 6

### The use of a set based on genetic signature in the diagnosis of papillary thyroid cancer

The previously selected signatures of 13 diagnostic genes were verified in a particular population, in papillary thyroid cancer patients of a young age. The analysis was performed on 14 samples collected from mildly changed or unchanged thyroid as well as 38 papillary thyroid cancer samples. The Support Vector Machine technique was used, and the quality of the classification was assessed using the cross-validation method. In the present example, we managed to correctly identify 100% of the tested samples. The method was characterised by a sensitivity of 100%, a specificity of 100%, a positive result predictive value of 100% and a negative result predictive value of 100%. These results are sufficient to transfer the technique into clinical use.

To summarise, due to the embodiment of the present invention, we identified RNA markers for papillary thyroid cancer focused on the detection of the most common cancer of this organ, papillary thyroid cancer, through performing a microarray analysis on sections and isolated papillary cancer cells obtained via laser microdissection, with validation using microarray analysis of larger tumour fragments collected during surgery, as well as real time PCR analysis of this material. We indicated groups of genes which exhibit changes in their expression during papillary thyroid cancer, due to which the analysis of their expression can be used in the diagnosis of this type of tumour.

### Literature:

**1.** M. A. Aldred, M. E. Ginn-Pease and C. Eng. Caveolin-1 and caveolin-2,together with three bone morphogenetic protein-related genes, may encode novel tumour suppressors downregulated in sporadic follicular thyroid carcinogenesis. Cancer Res. 63 (11):2864-2871, 2003.
**2.** M. A. Aldred, Y. Huang, S. Liyanarachchi, N. S. Pellegata, O. Gimm, S. Jhiang, R. V. Davuluri, A. de la Chapelle, and C. Eng. Papillary and follicular thyroid carcinomas show distinctly different microarray expression profiles and can be distinguished by a minimum offive genes. J.Clin Oncol. 22 (17):3531-3539, 2004.
**3.** S. L. Asa. The role of immunohistochemical markers in the diagnosis of follicularpatterned lesions of the thyroid. Endocr.Pathol. 16 (4):295-309, 2005.
**4.** C. B. Barden, K. W. Shister and T. J. Fahey, III. Classification of follicular thyroid tumours by molecular signature: results of gene profiling. Clin Cancer Res. 9 (5):1792-1800,2003.
**5.** O. Baris, F. Savagner and R. Houlgatte. Transcriptional profiling reveals coordinated up-regulation of oxidative metabolism genes in thyroid oncocytic tumours. J.Clin Endocrinol Metab 89 (2):994-1005, 2004.
**6.** O. Baris, D. Mirebeau-Prunier and Y. Malthiery. Gene profiling reveals specific oncogenic mechanisms and signaling pathways in oncocytic and papillary thyroid carcinoma. Oncogene 24 (25):4155-4161, 2005.
**7.** V. J. Bernet, J. Anderson and M. D. Ringel. Determination of galectin-3 messenger ribonucleic Acid overexpression in papillary thyroid cancer by quantitative reverse transcription-polymerase chain reaction. J.Clin.Endocrinol.Metab 87 (10):4792-4796, 2002.
**8.** J. M. Cerutti, R. Delcelo, M. J. Amadei, C. Nakabashi, R. M. Maciel, B. Peterson, J. Shoemaker, and G. J. Riggins. A preoperative diagnostic test that distinguishes benign from malignant thyroid carcinoma based on gene expression. J.Clin Invest 113 (8):1234-1242, 2004.
**9.** K. T. Chen, J. D. Lin and E. C. Chan. Identifying differentially expressed genes associated with metastasis of follicular thyroid cancer by cDNA expression array. Thyroid 11 (1):41-46, 2001.
**10**. S. Chevillard, N. Ugolin and A. K. El-Naggar. Gene expression profiling of differentiated thyroid neoplasms: diagnostic and clinical implications. Clin Cancer Res. 10 (19):6586-6597, 2004.
**11.** P. S. de Matos, A. P. Ferreira and L. S. Ward. Usefulness of HBME-1, cytokeratin 19 and galectin-3 immunostaining in the diagnosis of thyroid malignancy. Histopathology 47(4):391-401,2005.
**12.** V. Detours, S. Wattel and C. Maenhaut. Absence of a specific radiation signature in post-Chernobyl thyroid cancers. Br.J.Cancer 92 (8):1545-1552, 2005.
**13.** M. Eszlinger, K. Krohn, and R. Paschke. Complementary DNA expression array analysis suggests a lower expression of signal transduction proteins and receptors in cold and hot thyroid nodules. J.Clin Endocrinol Metab 86 (10):4834-4842, 2001.
**14.** M. Eszlinger, K. Krohn, R. Frenzel, S. Kropf, A. Tonjes, and R. Paschke. Gene expression analysis reveals evidence for inactivation of the TGF-beta signaling cascade in autonomously functioning thyroid nodules. Oncogene 23 (3):795-804, 2004.
**15.** M. Eszlinger, M. Wiench and R. Paschke. Meta- and reanalysis of gene expression profiles of hot and cold thyroid nodules and papillary thyroid carcinoma for gene groups. Clin Endocrinol Metab, 2006.
**16.** D. J. Finley, N. Arora and T. J. Fahey, III. Molecular profiling distinguishes papillary carcinoma from benign thyroid nodules. J.Clin Endocrinol Metab 89 (7):3214-3223, 2004.
**17.** D. J. Finley, B. Zhu and T. J. Fahey, III. Discrimination of benign and malignant thyroid nodules by molecular profiling. Ann.Surg. 240 (3):425-436, 2004.
**18.** K. Fujarewicz, M Jarz.b and A .wierniak. A multigene approach to differentiate papillary thyroid carcinoma from benign lesions: gene selection using Support Vector Machines with bootstrapping. Endocr.Relat Cancer submited, 2007.
**19.** R. Giannini, P. Faviana, T. Cavinato, R. Elisei, F. Pacini, P. Berti, G. Fontanini, C. Ugolini, T. Camacci, Ieso K. De, P. Miccoli, A. Pinchera, and F. Basolo. Galectin-3 and oncofetal-fibronectin expression in thyroid neoplasia as assessed by reverse transcriptionpolymerase chain reaction and immunochemistry in cytologic and pathologic specimens. Thyroid 13 (8):765-770, 2003.
**20.** T. J. Giordano, R. Kuick and Y. E. Nikiforov. Molecular classification of papillary thyroid carcinoma: distinct BRAF, RAS, and RET/PTC mutation-specific gene expression profiles discovered by DNA microarray analysis. Oncogene 24 (44):6646-6656, 2005.
**21.** O. L. Griffith, A. Melck, S. J. Jones, and S. M. Wiseman. Meta-analysis and metareview of thyroid cancer gene expression profiling studies identifies important diagnostic biomarkers. J.Clin.Oncol. 24 (31):5043-5051, 2006.
**22.** A. Hamada, S. Mankovskaya and S. Yamashita. Diagnostic usefulness of PCR profiling of the differentially expressed marker genes in thyroid papillary carcinomas. Cancer Lett. 224 (2):289-301, 2005.
**23.** Y. Hasegawa, T. Takano, A. Miyauchi, F. Matsuzuka, H. Yoshida, K. Kuma, and N. Amino. Decreased expression of catalase mRNA in thyroid anaplastic carcinoma. Jpn.J.Clin.Oncol. 33 (1):6-9, 2003.
**24.** L. Hawthorn, L. Stein, R. Varma, S. Wiseman, T. Loree, and D. Tan. TIMP1 and SERPIN-A overexpression and TFF3 and CRABP1 underexpression as biomarkers for papillary thyroid carcinoma. Head Neck 26 (12):1069-1083, 2004.
**25**. A. Hoos, A. Stojadinovic, B. Singh, M. E. Dudas, D. H. Leung, A. R. Shaha, J. P. Shah, M. F. Brennan, C. Cordon-Cardo, and R. Ghossein. Clinical significance of molecular expression profiles of Hurthle cell tumours of the thyroid gland analyzed via tissue microarrays. Am.J.Pathol. 160 (1):175-183, 2002.
**26**. Y. Huang, M. Prasad, W. J. Lemon, H. Hampel, F. A. Wright, K. Kornacker, V. LiVolsi, W. Frankel, R. T. Kloos, C. Eng, N. S. Pellegata, and A. de la Chapelle. Gene expression in papillary thyroid carcinoma reveals highly consistent profiles. Proc.Natl.Acad.Sci.U.S.A 98 (26):15044-15049, 2001.
**27.** H. Inohara, Y. Honjo, T. Yoshii, S. Akahani, J. Yoshida, K. Hattori, S. Okamoto, T. Sawada, A. Raz, and T. Kubo. Expression of galectin-3 in fine-needle aspirates as a diagnostic marker differentiating benign from malignant thyroid neoplasms. Cancer 85 (11):2475-2484, 1999.
**28.** C. Jacques, O. Baris, D. Prunier-Mirebeau, F. Savagner, P. Rodien, V. Rohmer, B. Franc, S. Guyetant, Y. Malthiery, and P. Reynier. Two-step differential expression analysis reveals a new set of genes involved in thyroid oncocytic tumours. J.Clin Endocrinol Metab 90 (4):2314-2320, 2005.
**29.** S. Jain, M. A. Watson, M. K. DeBenedetti, Y. Hiraki, J. F. Moley, and J. Milbrandt. Expression profiles provide insights into early malignant potential and skeletal abnormalities in multiple endocrine neoplasia type 2B syndrome tumours. Cancer Res. 64 (11):3907-3913, 2004.
**30.** B. Jarzab, M. Wiench, K. Fujarewicz, K. Simek, M. Jarzab, M. Oczko- Wojciechowska, J. Wloch, A. Czarniecka, E. Chmielik, D. Lange, A. Pawlaczek, S. Szpak, E. Gubala, and A. Swierniak. Gene expression profile of papillary thyroid cancer: sources of variability and diagnostic implications. Cancer Res. 65 (4):1587-1597, 2005.
**31.** E. Kebebew, M. Peng, E. Reiff, and A. McMillan. Diagnostic and extent of disease multigene assay for malignant thyroid neoplasms. Cancer, 2006.
**32.** K. P. Letsas, M. Frangou-Lazaridis, A. Skyrlas, A. Tsatsoulis, and V. Malamou-Mitsi. Transcription factor-mediated proliferation and apoptosis in benign and malignant thyroid lesions. Pathol.Int. 55 (11):694-702, 2005.
**33.** C. C. Lubitz and T. J. Fahey, III. The differentiation of benign and malignant thyroid nodules. Adv.Surg. 39:355-377, 2005.
**34.** C. C. Lubitz and T. J. Fahey, III. Gene expression profiling of thyroid tumours—clinical applicability. Nat.Clin.Pract.Endocrinol.Metab 2 (9):472-473, 2006.
**35.** C. Mazzanti, M. A. Zeiger, N. G. Costouros, C. Umbricht, W. H. Westra, D. Smith, H. Somervell, G. Bevilacqua, H. R. Alexander, and S. K. Libutti. Using gene expression profiling to differentiate benign versus malignant thyroid tumours. Cancer Res. 64 (8):2898-2903, 2004.
**36.** A. Mitselou, D. Peschos, P. Dallas, K. Charalabopoulos, N. J. Agnantis, and T. Vougiouklakis. Immunohistochemical analysis of expression of bcl-2 protein in papillary carcinomas and papillary microcarcinomas of the thyroid gland. Exp.Oncol. 26 (4):282-286, 2004.
**37.** M. Onda, M. Emi, A. Yoshida, S. Miyamoto, J. Akaishi, S. Asaka, K. Mizutani, K. Shimizu, M. Nagahama, K. Ito, T. Tanaka, and T. Tsunoda. Comprehensive gene expression profiling of anaplastic thyroid cancers with cDNA microarray of 25 344 genes. Endocr.Relat Cancer 11 (4):843-854, 2004.
**38.** M. Onda, J. Akaishi, S. Asaka, J. Okamoto, S. Miyamoto, K. Mizutani, A. Yoshida, K. Ito, and M. Emi. Decreased expression of haemoglobin beta (HBB) gene in anaplastic thyroid cancer and recovery of its expression inhibits cell growth. Br.J.Cancer 92 (12):2216-2224, 2005.
**39.** M. Papotti, J. Rodriguez, Pompa R. De, A. Bartolazzi, and J. Rosai. Galectin-3 and HBME-1 expression in well-differentiated thyroid tumours with follicular architecture of uncertain malignant potential. Mod.Pathol. 18 (4):541-546, 2005.
**40.** M. L. Prasad, N. S. Pellegata, R. T. Kloos, C. Barbacioru, Y. Huang, and A. de la Chapelle. CITED1 protein expression suggests Papillary Thyroid Carcinoma in high throughput tissue microarray-based study. Thyroid 14 (3):169-175, 2004.
**41.** E. Saggiorato, Pompa R. De, M. Volante, S. Cappia, F. Arecco, A. P. Dei Tos, F. Orlandi, and M. Papotti. Characterization of thyroid follicular neoplasms' in fine-needle aspiration cytological specimens using a panel of immunohistochemical markers: a proposal for clinical use. Endocr.Relat Cancer 12 (2):305-317, 2005.
**42.** M. S. Sarquis, F. Weber, L. Shen, C. E. Broelsch, S. M. Jhiang, J. Zedenius, A. Frilling, and C. Eng. High frequency of loss of heterozygosity in imprinted, compared with nonimprinted, genomic regions in follicular thyroid carcinomas and atypical adenomas. J.Clin Endocrinol Metab 91 (1):262-269, 2006.
**43.** T. Takano, F. Matsuzuka, A. Miyauchi, T. Yokozawa, G. Liu, S. Morita, K. Kuma, and N. Amino. Restricted expression of oncofetal fibronectin mRNA in thyroid papillary and anaplastic carcinoma: an in situ hybridization study. Br.J.Cancer 78 (2):221-224, 1998.
**44.** T. Takano, Y. Hasegawa, F. Matsuzuka, A. Miyauchi, H. Yoshida, T. Higashiyama, K. Kuma, and N. Amino. Gene expression profiles in thyroid carcinomas. Br.J Cancer 83 (11):1495-1502, 2000.
**45.** T. Takano, Y. Hasegawa, A. Miyauchi, F. Matsuzuka, H. Yoshida, K. Kuma, N. Hayashi, S. Nakamori, and N. Amino. Quantitative analysis of osteonectin mRNA in thyroid carcinomas. Endocr.J. 49 (4):511-516, 2002.
**46.** T. Takano, A. Miyauchi, H. Yoshida, K. Kuma, and N. Amino. High-throughput differential screening of mRNAs by serial analysis of gene expression: decreased expression of trefoil factor 3 mRNA in thyroid follicular carcinomas. Br.J.Cancer 90 (8):1600-1605, 2004.
**47.** V. M. Wasenius, S. Hemmer, E. Kettunen, S. Knuutila, K. Franssila, and H. Joensuu. Hepatocyte growth factor receptor, matrix metalloproteinase-11, tissue inhibitor of metalloproteinase-1, and fibronectin are up-regulated in papillary thyroid carcinoma: a cDNAand tissue microarray study. Clin Cancer Res. 9 (1):68-75, 2003.
**48.** F. Weber and C. Eng. Gene-expression profiling in differentiated thyroid cancer-aviable strategy for the practice of genomic medicine? Future.Oncol. 1 (4):497-510, 2005.
**49.** F. Weber, L. Shen, M. A. Aldred, C. D. Morrison, A. Frilling, M. Saji, F. Schuppert, C. E. Broelsch, M. D. Ringel, and C. Eng. Genetic classification of benign and malignant thyroid follicular neoplasia based on a three-gene combination. J.Clin.Endocrinol.Metab 90(5):2512-2521,2005.
**50.** J. Xu, F. Moatamed, J. S. Caldwell, J. R. Walker, Z. Kraiem, K. Taki, G. A. Brent, and J. M. Hershman. Enhanced expression of nicotinamide N-methyltransferase in human papillary thyroid carcinoma cells. J.Clin Endocrinol Metab 88 (10):4990-4996, 2003.
51. Y. Yano, N. Uematsu, T. Yashiro, H. Hara, E. Ueno, M. Miwa, G. Tsujimoto, Y. Aiyoshi, and K. Uchida. Gene expression profiling identifies platelet-derived growth factor as a diagnostic molecular marker for papillary thyroid carcinoma. Clin Cancer Res. 10 (6):2035-2043,2004.
**52.** M. Zou, K. S. Famulski, R. S. Parhar, E. Baitei, F. A. Al-Mohanna, N. R. Farid, and Y. Shi. Microarray analysis of metastasis-associated gene expression profiling in a murine model of thyroid carcinoma pulmonary metastasis: identification of S100A4 (Mts1) gene overexpression as a poor prognostic marker for thyroid carcinoma. J.Clin Endocrinol Metab 89 (12):6146-6154, 2004.
**53.** Reduced expression of N-Myc downstream-regulated gene 2 in human thyroid cancer.Zhao H, Zhang J, Lu J, He X, Chen C, Li X, Gong L, Bao G, Fu Q, Chen S, Lin W, Shi H, Ma J, Liu X, Ma Q, Yao L. BMC Cancer. 2008 Oct 22;8:303.
**54.** Immunohistochemical distinction of follicular thyroid adenomas and follicular carcinomas. Bryson PC, Shores CG, Hart C, Thorne L, Patel MR, Richey L, Farag A, Zanation AM. Arch Otolaryngol Head Neck Surg. 2008 Jun;134(6):581-6.
**55.** Genome-wide gene expression profiles of thyroid carcinoma: Identification of molecular targets for treatment of thyroid carcinoma. Nikolova DN, Zembutsu H, Sechanov T, Vidinov K, Kee LS, Ivanova R, Becheva E, Kocova
   M, Toncheva D, Nakamura Y.Oncol Rep. 2008 Ju1;20(1):105-21.
**56.** Gene expression in RET/PTC3 and E7 transgenic mouse thyroids: RET/PTC3 but not E7 tumours are partial and transient models of human papillary thyroid cancers. Burniat A, Jin L, Detours V, Driessens N, Goffard JC, Santoro M, Rothstein J, Dumont JE, Miot F, Corvilain B.Endocrinology. 2008 Oct;149(10):5107-17. Epub 2008 Jun 26.
**57.** Identification of immunohistochemical biomarkers for papillary thyroid carcinoma using gene expression profiling.Murphy KM, Chen F, Clark DP.Hum Pathol. 2008 Mar; 39(3):420-6.
**58.** TFF3-based candidate gene discrimination of benign and malignant thyroid tumours in a region with borderline iodine deficiency. Krause K, Eszlinger M, Gimm O, Karger S, Engelhardt C, Dralle H, Fuhrer D. J Clin Endocrinol Metab. 2008 Apr;93(4):1390-3. Epub 2008 Jan 15.
**59.** Microarray analysis refines classification of non-medullary thyroid tumours of uncertain malignancy. Fontaine JF, Mirebeau-Prunier D, Franc B, Triau S, Rodien P, Houlgatte R, Malthiery Y, Savagner F. Oncogene. 2008 Apr 3;27(15):2228-36. Epub 2007 Oct 29.
**60.** Diagnostic value of differential expression of CK19, Galectin-3, HBME-1, ERK, RET, and p16 in benign and malignant follicular-derived lesions of the thyroid: an immunohistochemical tissue microarray analysis. Barroeta JE, Baloch ZW, Lal P, Pasha TL, Zhang PJ, LiVolsi VA. Endocr Pathol. 2006 Fall;17(3):225-34.
**61.** Characterization of microarray gene expression profiles of early stage thyroid tumours. Gombos K, Zele E, Kiss I, Varjas T, Puskas L, Kozma L, Juhasz F, Kovacs E, Szanyi I, Ember I. Cancer Genomics Proteomics. 2007 Nov-Dec;4(6):403-9.
**62.** Identification of molecular markers altered during transformation of differentiated into anaplastic thyroid carcinoma. Wiseman SM, Griffith OL, Deen S, Rajput A, Masoudi H, Gilks B, Goldstein L, Gown A, Jones SJ. Arch Surg. 2007 Aug;142(8):717-27; discussion 727-9.
**63.** Genome-wide expression analysis of Middle Eastern papillary thyroid cancer reveals c-MET as a novel target for cancer therapy. Siraj AK, Bavi P, Abubaker J, Jehan Z, Sultana M, A-Dayel F, Al-Nuaim A, Alzahrani A, Ahmed M, Al-Sanea O, Uddin S, Al-Kuraya KS. J Pathol. 2007 Oct;213(2):190-9.
**64.** Gene expression and the biological phenotype of papillary thyroid carcinomas. Delys L, Detours V, Franc B, Thomas G, Bogdanova T, Tronko M, Libert F, Dumont JE,
   Maenhaut C. Oncogene. 2007 Dec 13;26(57):7894-903. Epub 2007 Jul 9.
**65.** Expression microarray analysis of papillary thyroid carcinoma and benign thyroid tissue: emphasis on the follicular variant and potential markers of malignancy. Finn SP, Smyth P, Cahill S, Streck C, O'Regan EM, Flavin R, Sherlock J, Howells D, Henfrey R, Cullen M, Toner M, Timon C, O'Leary JJ, Sheils OM. Virchows Arch. 2007
   Mar;450(3):249-60. Epub 2007 Jan 25.
**66.** Anaplastic thyroid carcinoma: expression profile of targets for therapy offers new insights for disease treatment. Wiseman SM, Masoudi H, Niblock P, Turbin D, Rajput A, Hay J, Bugis S, Filipenko D, Huntsman D, Gilks B.Ann Surg Oncol. 2007 Feb;14(2):719-29. Epub 2006 Nov 10.
**67.** Microarray analysis of thyroid nodule fine-needle aspirates accurately classifies benign and malignant lesions. Lubitz CC, Ugras SK, Kazam JJ, Zhu B, Scognamiglio T, Chen YT, Fahey TJ 3rd. J Mol Diagn. 2006 Sep;8(4):490-8; quiz 528.
**68.** Molecular analysis of minimally invasive follicular carcinomas by gene profiling. Lubitz CC, Gallagher LA, Finley DJ, Zhu B, Fahey TJ 3rd. Surgery. 2005 Dec;138(6):1042-8; discussion 1048-9.
**69.** S100A4 (Mts1) gene overexpression is associated with invasion and metastasis of papillary thyroid carcinoma. Zou M, Al-Baradie RS, AI-Hindi H, Farid NR, Shi Y. Br J Cancer. 2005 Nov 28;93(11):1277-84.
**70.** Overexpressed in anaplastic thyroid carcinoma-1 (OEATC-1) as a novel gene responsible for anaplastic thyroid carcinoma. Mizutani K, Onda M, Asaka S, Akaishi J, Miyamoto S, Yoshida A, Nagahama M, Ito K, Emi M. Cancer. 2005 May 1;103(9):1785-90.
**71.** Microarray comparative genomic hybridization reveals genome-wide patterns of DNA gains and losses in post-Chernobyl thyroid cancer. Kimmel RR, Zhao LP, Nguyen D, Lee S, Aronszajn M, Cheng C, Troshin VP, Abrosimov A, Delrow J, Tuttle RM, Tsyb AF, Kopecky KJ, Davis S, Neiman PE. Radiat Res. 2006 Sep;166(3):519-31.

## Claims

1. A kit for detecting the expression of genes characteristic of papillary thyroid cancer, **characterised in that** it contains elements used for the detection of the following genes:
ANXA1, CHI3L1, CITED1, FN1, GDF15, LRP4, MET, PROS1, SFTPB, TPO, MPPED2, TFF3, and DPP4.

2. The kit according to claim 1, **characterised in that** it is used to evaluate expression at the RNA level.

3. The kit according to claim 1, **characterised in that** it contains nucleotide probes included in the following sets: 201012_at (SEQ ID NO 1-11), 207808_s_at (SEQ ID NO 12-22), 209395_at (SEQ ID NO 23-33), 209396_s_at (SEQ ID NO 34-44), 209810_at (SEQ ID NO 45-55), 210342_s_at (SEQ ID NO 56-66), 210495_x_at (SEQ ID NO 67-77), 212850_s_at (SEQ ID NO 78-88), 216442_x_at (SEQ ID NO 89-99), 221577_x_at (SEQ ID NO 100-110), 203510_at (SEQ ID NO 111-121), 207144_s_at (SEQ ID NO 122-132), 37004_at (SEQ ID NO 132-148).

4. A method of detecting papillary thyroid cancer, **characterised in that** a sample of tumour tissue is collected from a patient and the gene expression profile is examined in the collected sample, wherein the expression profile for papillary thyroid cancer is the profile encompassing the increased expression of the following genes: ANXA1, CHI3L1, CITED1, FN1, GDF15, LRP4, MET, PROS1, SFTPB, MPPED2, TFF3 and DPP4 as well as the decreased expression of TPO.

5. The method according to claim 4, **characterised in that** the expression is evaluated at the level of RNA.

6. The method according to claim 5, **characterised in that** the analysis of the expression profile makes use of nucleotide probes included in the following sets: 201012_at (SEQ ID NO 1-11), 207808_s_at (SEQ ID NO 12-22), 209395_at (SEQ ID NO 23-33), 209396_s_at (SEQ ID NO 34-44), 209810_at (SEQ ID NO 45-55), 210342_s_at (SEQ ID NO 56-66), 210495_x_at (SEQ ID NO 67-77), 212850_s_at (SEQ ID NO 78-88), 216442_x_at (SEQ ID NO 89-99), 221577_x_at (SEQ ID NO 100-110), 203510_at (SEQ ID NO 111-121), 207144_s_at (SEQ ID NO 122-132), 37004_at (SEQ ID NO 132-148).

7. Use of a gene set consisting of: ANXA1, CHI3L1, CITED1, FN1, GDF15, LRP4, MET, PROS1, SFTPB, TPO, MPPED2, TFF3, and DPP4 in the detection of papillary thyroid cancer.

8. Use of probes belonging to a group encompassing nucleotide probes included in the following sets: 201012_at (SEQ ID NO 1-11), 207808_s_at (SEQ ID NO 12-22), 209395_at (SEQ ID NO 23-33), 209396_s_at (SEQ ID NO 34-44), 209810_at (SEQ ID NO 45-55), 210342_s_at (SEQ ID NO 56-66), 210495_x_at (SEQ ID NO 67-77), 212850_s_at (SEQ ID NO 78-88), 216442_x_at (SEQ ID NO 89-99), 221577_x_at (SEQ ID NO 100-110), 203510_at (SEQ ID NO 111-121), 207144_s_at (SEQ ID NO 122-132), 37004_at (SEQ ID NO 132-148) in the detection of papillary thyroid cancer.
